# EUROPEAN PATENT APPLICATION

(11) **EP 2 233 479 A1**
(43) Date of publication of application: **29.09.2010**
(21) Application number: 09013187.1
(22) Date of filing: 01.10.2007
(51) Int. Cl.: C07D 323/04, C07D 491/10, A61K 31/357, A61K 31/438

(54) **DISPIRO TETRAOXANE COMPOUNDS AND THEIR USE IN THE TREATMENT OF MALARIA AND/OR CANCER**

(30) Priority: 30.09.2006 GB 0619333
(62) Divisional of application: 07823981.1
(71) Applicant: Liverpool School of Tropical Medicine, Liverpool L3 5QA (GB)
(72) Inventor: AMEWU Richard, Liverpool L69 7ZD (GB); O'NEILL Paul Michael, Liverpool L69 7ZD (GB); STACHULSKI Andrew, Liverpool L69 7ZD (GB); ELLIS Gemma, Liverpool L69 7ZD (GB); WARD Stephen Andrew, Liverpool L3 5QA (GB)
(74) Representative: Dauncey, Mark Peter

(57) **Abstract**

A compound having the formula (I) 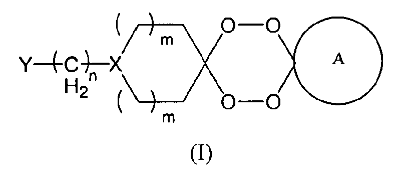 wherein
ring A represents a substituted or unsubstituted monocyclic or multicyclic ring; m=any positive integer; n=0-5;
X=CH and Y=-C(O)NR¹R², -NR¹R² or -S(O)₂R⁴, where R¹, R² and R⁴ are each individually selected from the group consisting of H, substituted or unsubstituted alkyl, substituted or unsubstituted aryl, substituted or unsubstituted amine, substituted or unsubstituted carbocyclic ring, substituted or unsubstituted heterocyclic ring, or any combination thereof, or R¹ and R² are linked so as to form part of a substituted or unsubstituted heterocyclic ring;
or
X=N and Y=-S(O)₂R³ or -C(O)R³, where R³ is selected from the group consisting of H, substituted or unsubstituted alkyl, substituted or unsubstituted aryl, substituted or unsubstituted amine, substituted or unsubstituted carbocyclic ring, substituted or unsubstituted heterocyclic ring or any combination thereof.

## Description

The present invention relates to dispiro tetraoxane compounds, particularly but not exclusively, for use in the treatment of malaria and/or cancer, and methods for producing such compounds.

The discovery of artemisinin and the establishment that the peroxide pharmacophore is important for antimalarial activity has seen many attempts by chemists to synthesise simple but effective synthetic or semi-synthetic endoperoxides. Artemisinin **(2)** is a naturally occurring endoperoxide sesquiterpene lactone compound of *Artemisia annua,* an herbal remedy used in Chinese medicine. Although artemisinin derivatives are extensively used against malaria, cost, supply and high recrudescent rates remain issues with this class of drug. Other known peroxides with antimalarial potency include *Yingzhaousu* (**3**), WR148999 (**4**) and steroid amide **(5).**

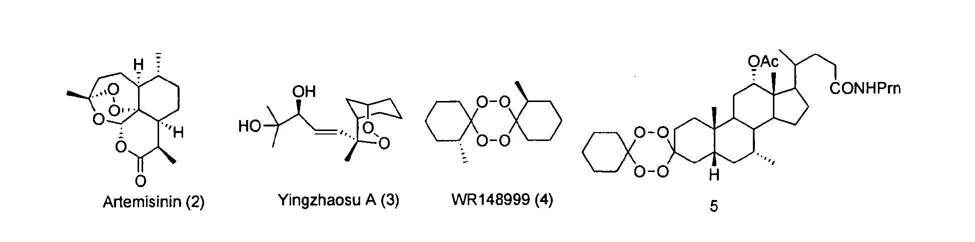

The endoperoxides group is an important functional group in medicinal chemistry. It is found in the artemisinin class of antimalarials such as artemether and artesunate, in which its reaction with heme (or free Fe(II)) generates cytotoxic radicals which cause parasite death. More recently, artemisinin derived 1,2,4-trioxane monomers and dimers have been shown to be potent inhibitors of cancer cell proliferation. A disadvantage with the semi-synthetic compounds described is that their production requires artemisinin as starting material. Artemisinin is extracted from the plant *Artemisinia annua* in low yield, a fact that necessitates significant crop-production. Therefore, there is much need for the development of new and improved approaches to synthetic endoperoxides.

Tetraoxanes were initially used industrially for the production of macrocyclic hydrocarbons and lactones, however, pioneering work by the Vennerstrom group demonstrated that symmetrical tetraoxanes possess impressive antimalarial activity *in vitro.* Tetraoxanes are believed to have a similar mode of activity as the naturally occurring peroxides such are artemisinin.

The major drawbacks with tetraoxanes that have been synthesized to date include poor stability and low oral antimalarial activity. Apart from some recent success with steroidal-based 1,2,4,5-tetraoxanes such as **(5)**, previously synthesised tetraoxanes all have poor oral bioavailability. Although many of the first generation tetraoxane derivatives are highly lipophilic, suggesting that poor absorption was the key factor affecting bioavailability, it is also apparent that first pass metabolism plays a role in reducing effective drug absorption.

The object of the present invention is to obviate or mitigate one or more of the above problems.

According to a first aspect of the present invention there is provided a compound having the formula (I) 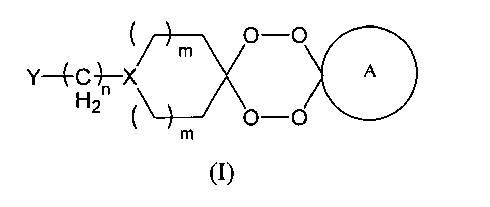 wherein
ring A represents a substituted or unsubstituted monocyclic or multicyclic ring; m=any positive integer; n=0-5;
X=CH and Y=C(O)NR¹R², -NR¹R² or -S(O)₂R⁴, where R¹, R² and R⁴ are each individually selected from the group consisting of H, substituted or unsubstituted alkyl, substituted or unsubstituted aryl, substituted or unsubstituted amine, substituted or unsubstituted carbocyclic ring, substituted or unsubstituted heterocyclic ring, or any combination thereof, or R¹ and R² are linked so as to form part of a substituted or unsubstituted heterocyclic ring;
or
X=N and Y=-S(O)₂R³ or -C(O)R³, where R³ is selected from the group consisting of H, substituted or unsubstituted alkyl, substituted or unsubstituted aryl, substituted or unsubstituted amine, substituted or unsubstituted carbocyclic ring, substituted or unsubstituted heterocyclic ring or any combination thereof.

Preferred compounds in accordance with the first aspect of the present invention have unprecedented *in vivo* levels of antimalarial activity for the tetraoxane class of drug.

A second aspect of the present invention provides a method for the production of a compound according to the first aspect of the present invention, wherein the method comprises reacting a bishydoperoxide compound having formula (Ia) with a ketone having formula (Ib)

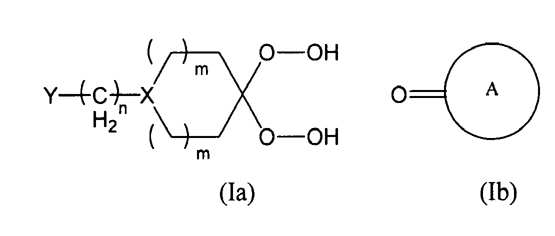

In the compound forming the first aspect of the present invention preferably ring A contains 3 to 30 carbon atoms, more preferably 5 to 15 carbon atoms, and most preferably 6, 8, 10 or 12 carbon atoms. Ring A is preferably a substituted or unsubstituted mono- or polycyclic alkyl ring.

Polycyclic alkyl rings, which contain more than one ring system may be "fused",
where adjacent rings share two adjacent carbon atoms, "bridged", where the rings are defined by at least two common carbon atoms (bridgeheads) and at least three acyclic chains (bridges) connecting the common carbon atoms, or "spiro" compounds where adjacent rings are linked by a single common carbon atom.

Preferably ring A is selected from the group consisting of a substituted or unsubstituted cyclopentyl ring, a substituted or unsubstituted cyclohexyl ring, a substituted or unsubstituted cyclododecanyl ring, and a substituted or unsubstituted adamantyl group. In a particularly preferred embodiment of the compound forming the first aspect of the present invention, ring A is an adamantyl group.

In a preferred embodiment of the compound according to the first aspect of the present invention X=CH, Y=C(O)NR¹R² or -NR¹R², R¹=H and R²=alkyl group substituted with an ester group, amine group or amido group.

Said alkyl group may be an ethyl group.

Said amino group may be a diethylaminoethyl group.

Said ester group may be a methylester group.

In a further preferred embodiment of the compound according to the first aspect of the present invention X=CH, Y=C(O)NR¹R² or -NR¹R², R¹=H and R² contains a substituted or unsubstituted carbocyclic ring or a substituted or unsubstituted heterocyclic ring, zero, one or more methylene radicals being provided in between said carbocyclic or heterocyclic ring and the nitrogen atom of group Y.

In this embodiment R² preferably contains a substituted or unsubstituted cycloalkyl group containing 3 to 6 carbon atoms. The cycloalkyl group is most preferably bonded directly to the nitrogen atom of group Y.

Alternatively, R² preferably contains a substituted or unsubstituted heterocyclic group containing 3 to 6 carbon atoms and at least one heteroatom, the or each heteroatom being separately selected from the group consisting of nitrogen, oxygen and sulfur.

The heterocyclic group may be linked to the nitrogen atom of group Y by any appropriate number of methylene radicals, such as one, two, three or four methylene group. It is most preferred that the heterocyclic group is linked to the nitrogen atom of group Y via two methylene radicals.

Said heterocyclic group is preferably selected from the group consisting of a pyrrolidyl group, a piperidyl group, a morpholinyl group, a thiomorpholinyl group and a thiomorpholinyl sulfone group.

In a further preferred embodiment of the compound according to the first aspect of the present invention X=CH, Y=C(O)NR¹R² or NR¹R², and R¹ and R² are linked so as to form part of a substituted or unsubstituted heterocyclic ring selected from the group consisting of a pyrrolidyl group, a piperidyl group, a morpholinyl group, a thiomorpholinyl group and a thiomorpholinyl sulfone group.

In another preferred embodiment of the compound according to the first aspect of the present invention X=N, Y=-S(O)₂R³ or -C(O)R³, and R³ is a substituted or unsubstituted phenyl group or a substituted or unsubstituted heterocyclic group selected from the group consisting of a pyrrolidyl group, a piperidyl group, a morpholinyl group, a thiomorpholinyl group and a thiomorpholinyl sulfone group.

In a yet further preferred embodiment of the compound forming the first aspect of the present invention m=1, n=0, X=CH and Y=NHR², where R² is selected from the group consisting of H, substituted or unsubstituted alkyl, substituted or unsubstituted aryl, substituted or unsubstituted amine, substituted or unsubstituted carbocyclic ring, substituted or unsubstituted heterocyclic ring, or any combination thereof.

Preferably ring A is an adamantyl group.

In a further preferred embodiment m=1, n=1, X=CH, Y=S(O)₂R⁴, wherein R⁴ is selected from the group consisting of H, substituted or unsubstituted alkyl, substituted or unsubstituted aryl, substituted or unsubstituted amine, substituted or unsubstituted carbocyclic ring, substituted or unsubstituted heterocyclic ring, or any combination thereof.

It is preferred that ring A is a C₄-C₁₅ carbocyclic group. More preferably ring A is selected from a cyclohexanyl carbocylic group and an adamantyl group.

In a still further preferred embodiment of the first aspect of the present invention m=1, n=0, X=N and Y=C(O)R³, where R³ is a substituted or unsubstituted amine group or a substituted or unsubstituted heterocyclic ring containing a nitrogen atom
where said nitrogen atom connects the heterocyclic ring to the carbonyl carbon atom or group Y.

Where R³ is an amine group that is substituted, i.e. the nitrogen atom of the amine group is substituted with atoms and/or groups other than hydrogen atoms, the pattern of substitution may be symmetric or unsymetric. One or both amine substituents may be the same or different alkyl or aryl groups, which may themselves be substituted or unsubstituted. Preferably the amine group is substituted with one or two methyl, ethyl or propyl groups. The amine group may be substituted with an aromatic group, such as a phenyl group.

Ring A may be a C₄-C₁₅ carbocyclic group, preferably a cyclohexanyl carbocylic group or an adamantyl group.

Where R³ is a substituted or unsubstituted heterocyclic ring containing a nitrogen atom in which said nitrogen atom connects the heterocyclic ring to the carbonyl carbon atom, R³ preferably forms part of a pyrrolidyl group, a piperidyl group, a morpholinyl group, a thiomorpholinyl group and a thiomorpholinyl sulfone group.

As explained below in the Examples, reductive amination of appropriate ketones with various amino compounds afforded compounds **14-19**, which represent preferred compounds according to the first aspect of the present invention.

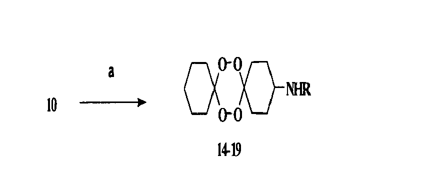

| | |
|---|---|
| **14** R = CH(CH₂)₂ | **15** R = CH₂CH₂N(CH₂)₄ |
| **16** R = CH₂CH₂N(CH₂)₅ | **18** R = CH₂CH₂N(C₂H₅)₂ |
| **17** R = CH₂CH₂N(CH₂)₄O | **19** R = (CH₂)₄O |

In a still further preferred embodiment of the compound according to the first aspect of the present invention m=1, n=1, X=CH and Y=-C(O)NR¹R², where R¹ and R² are each individually selected from the group consisting of H, substituted or unsubstituted alkyl, substituted or unsubstituted aryl, substituted or unsubstituted carbocyclic ring, substituted or unsubstituted heterocyclic ring, or any combination thereof, or R¹ and R² are linked so as to form part of a substituted or unsubstituted heterocyclic ring.

Preferably ring A is an adamantyl group.

Preferred compounds according to the first aspect of the present invention are represented below 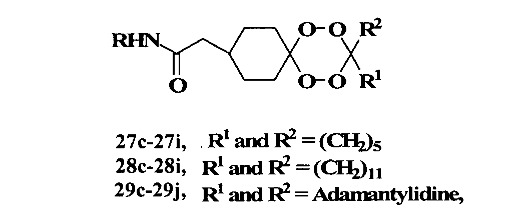 where

| | |
|---|---|
| **27c,** R= CH(CH₂)₂ | **28h,** R = (CH₂)₄O |
| **27d,** R =CH2CH₂(CH₂)₄ | **28i,** R = (CH₂)₄S |
| **27e,** R =CH₂CH₂N(CH₂)₅ | **29c,** R= CH(CH₂)₂ |
| **27f,** R=CH₂CH₂N(CH₂)₄O | **29d,** R =CH₂CH₂N(CH₂)₄ |
| **27g,** R= CH₂CH₂N(C₂H₅)₂ | **29e,** R =CH₂CH₂N(CH₂)₅ |
| **27h,** R = (CH₂)₄O | **29f,** R =CH₂CH₂N(CH₂)₄O |
| **27i,** R=CH₂CO₂CH₃ | **29g,** R= CH₂CH₂N(C₂H₅)₂ |
| **28c,** R= CH(CH₂)₂ | **29h,** R = (CH₂)₄O |
| **28d,** R=CH₂CH₂N(CH₂)₄ | **29i,** R = (CH₂)₄S |
| **28e,** R =CH₂CH₂N(CH₂)₅ | **29j,** R = CHCH(CH₃)₂CO₂CH₃ |
| **28f,** R =CH₂CH₂N(CH₂)₄O | **31,** R = (CH₂)₄SO₂, R¹&R²=(CH₂)₁₁ |
| **28g,** R= CH₂CH₂N(C₂H₅)₂ | **32,** R = (CH₂)₄SO₂, R¹&R²=adamantyl |

Further aspects of the present invention provide compounds having formulae (II) and (III)

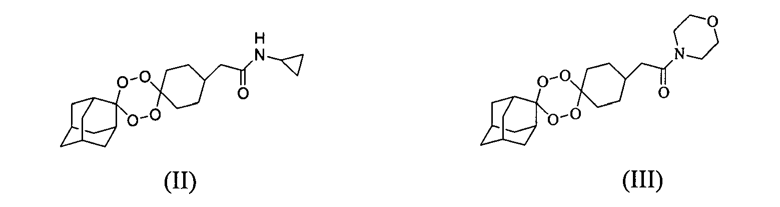

In a further preferred embodiment of the compound according to the first aspect of the present invention m=1, n=0, X=N and Y=S(O)₂R³ or -C(O)R³, where R³ is selected from the group consisting of H, substituted or unsubstituted alkyl, substituted or unsubstituted aryl, substituted or unsubstituted carbocyclic ring, substituted or unsubstituted heterocyclic ring or any combination thereof.

Preferably ring A is an adamantyl group.

Preferred compounds according to the first aspect of the present invention are compounds **39a, 39f, 34p, 40a** and **44** which are prepared from the corresponding ketone compound **38a, 38f** and **34f**, as shown below, some of which are described in more detail in the Examples.

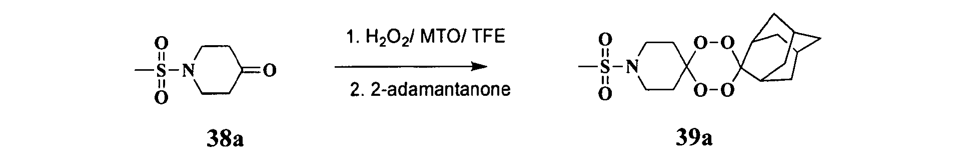

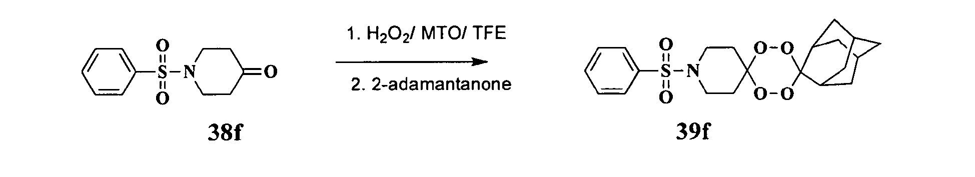

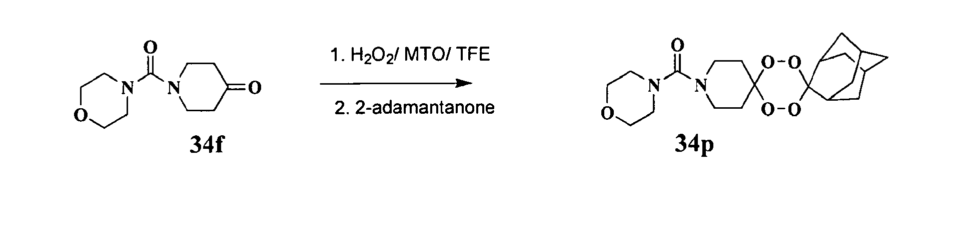

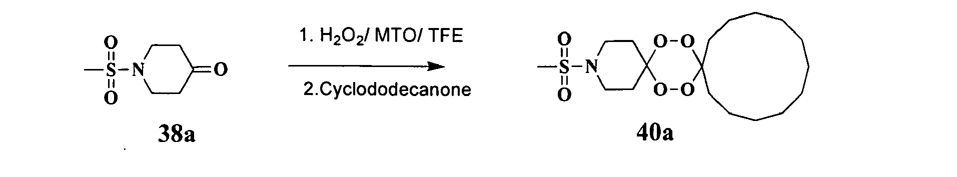

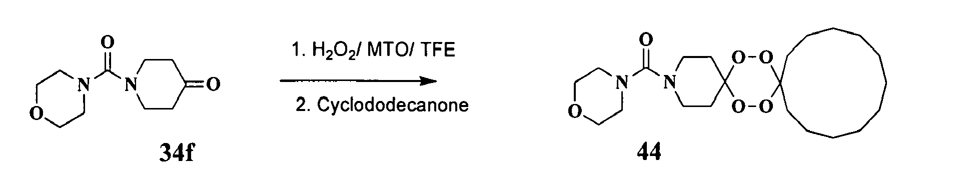

A preferred group of compounds according to the first aspect of the present invention are represented below 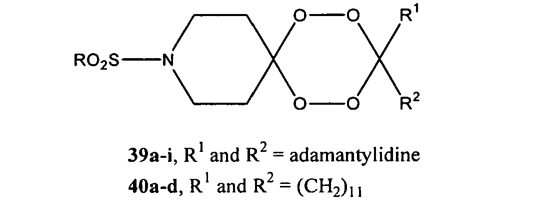 where

| | |
|---|---|
| **39a,** R = Me | **40a,** R = Me |
| **39b,** R = Et | **40b,** R = Et |
| **39c,** R = *i*-Pr | **40c,** R = *i*-Pr |
| **39d,** R = cyclopropyl | **40d,** R = C₆H₅ |
| **39e,** R = CH₂CF₃ | |
| **39f,** R = C₆H₅ | |
| **39g,** R =*p*-ClC₆H₄ | |
| **39h,** R =*p*-FC₆H₄ | |
| **39i,** R =*p*-CF3C₆H₄ | |

In a preferred embodiment of the first aspect of the present invention there is provided a compound having the formula (IX) (corresponding to compound **39b)**

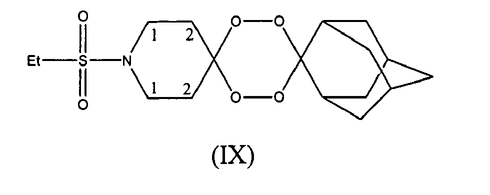

In a further preferred embodiment of the first aspect of the present invention there is provided a compound having the formula (X) (corresponding to compound **39d)**

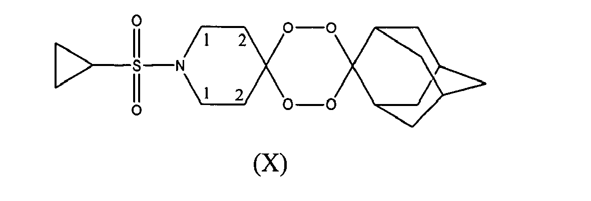

A still further preferred embodiment provides a compound having formula (XI) (corresponding to compound **34p**)

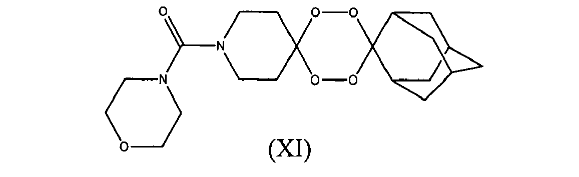

A further aspect of the present invention provides a compound having the general formula (XII) 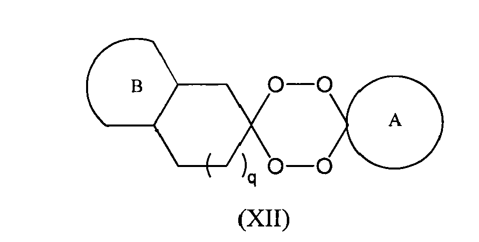 wherein ring A represents a substituted or unsubstituted monocyclic or multicyclic ring; q=any integer; and ring B represents a fused substituted or unsubstituted monocyclic or multicyclic ring.
q may take any appropriate integer value, such as 0 (in which case the ring fused to ring B will contain 5 carbon atoms), 1, 2, 3 or more.

Ring B may be a carbocyclic or heterocyclic ring aromatic or non-aromatic ring. Preferably, ring B is a non-substituted aromatic ring, such as a phenyl group.

Ring A may take any of the optional forms set out above in respect of the first aspect of the present invention. For example, ring A may be an adamantyl group.

Preferred embodiments of the class of compounds of general formula (XII) are compounds (XIII), (XIV) and (XV) corresponding to compounds **35d, 36c** and **36d** described below respectively.

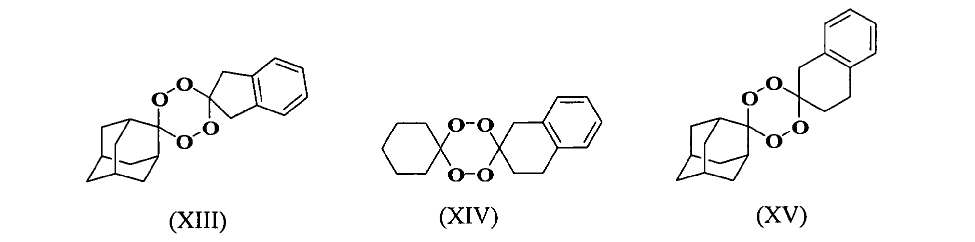

A still further aspect of the present invention provides a compound having the general formula (XVI) 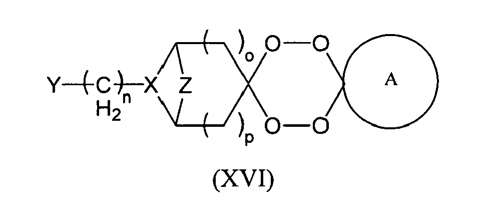 wherein ring A represents a substituted or unsubstituted monocyclic or multicyclic ring; o=any integer; p=any integer; n=0-5; Z represents a bridging group; and
X=CH and Y=C(O)NR¹R², -NR¹R² or -S(O)₂R⁴, where R¹, R² and R⁴ are each individually selected from the group consisting of H, substituted or unsubstituted alkyl, substituted or unsubstituted aryl, substituted or unsubstituted amine, substituted or unsubstituted carbocyclic ring, substituted or unsubstituted heterocyclic ring, or any combination thereof, or R¹ and R² are linked so as to form part of a substituted or unsubstituted heterocyclic ring,
or
X=N and Y=S(O)₂R³ or -C(O)R³, where R³ is selected from the group consisting of H, substituted or unsubstituted alkyl, substituted or unsubstituted aryl, substituted or unsubstituted amine, substituted or unsubstituted carbocyclic ring, substituted or unsubstituted heterocyclic ring or any combination thereof.

Z is any desirable bridging group. Preferably Z is an alkylidene or arylidene group which may be substituted or substituted and may incorporate one or more heteroatoms, such as oxygen, sulfur and/or nitrogen atoms. More preferably Z is a C₁-C₃ alkylidene group, most preferably an ethylidene group.

The various substituents set out above in the general definition of compound (XVI) may take any of the optional or preferred substituents specified above in respect of the first aspect of the present invention.

A preferred embodiment of the class of compounds of general formula (XVI) is compound corresponding to compound (XVII) below.

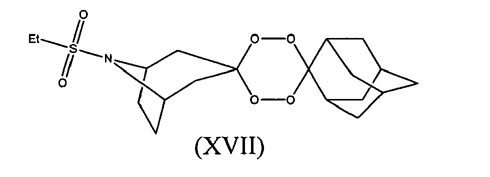

A further aspect of the present invention provides a salt of the compound according to the first aspect of the present invention. Said salt may be an acid addition salt produced by reacting a suitable compound according to the first aspect of the present invention with an appropriate acid, such as an organic acid or mineral acid.

The present invention further provides a pharmaceutical composition comprising a compound according to the first aspect of the present invention and a pharmaceutically acceptable excipient.

There is still further provided a pharmaceutical composition comprising a compound according to the first aspect of the present invention and a pharmaceutically acceptable excipient for the treatment of malaria.

A further aspect of the present invention provides use of a compound according to the first aspect of the present invention in the preparation of a medicament for the treatment of malaria.

Another aspect of the present invention provides a method of treating malaria in a human or animal patient comprising administering to said patient a therapeutically effective amount of a compound according to the first aspect of the present invention.

A yet further aspect of the present invention provides a pharmaceutical composition for the treatment of a cancer comprising a compound according to the first aspect of the present invention and a pharmaceutically acceptable excipient.

There is further provides use of a compound according to the first aspect of the present invention in the preparation of a medicament for the treatment of cancer.

A still further aspect of the present invention provides a method of treating a cancer in a human or animal patient comprising administering to said patient a therapeutically effective amount of a compound according to the first aspect of the present invention.

The aforementioned second aspect of the present invention provides a method for the production of a compound according to the first aspect of the present invention, wherein the method comprises reacting a bishydoperoxide compound having formula (Ia) with a ketone having formula (Ib)

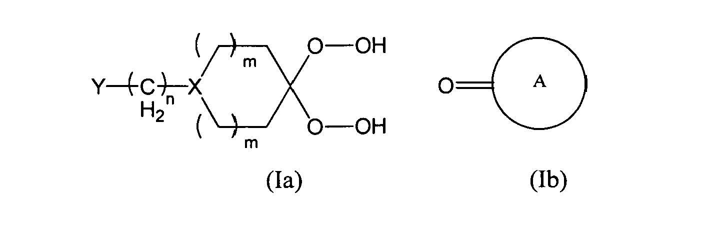

It is preferred that compound (Ia) is prepared by oxidising an appropriate starting material using an oxidising agent and isolating compound (Ia) from any excess unreacted oxidising agent prior to reacting compound (Ia) with compound (Ib).

Any appropriate oxidising agent may used but a preferred oxidising agent is hydrogen peroxide.

It is preferred that oxidation of said appropriate starting material is carried out in the presence of acetonitrile.

Said appropriate starting material is preferably selected from the group consisting of compounds (Ic) and (Id)

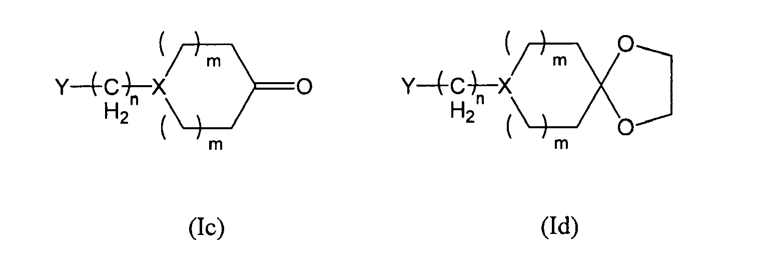

In a preferred embodiment of the method forming the second aspect of the present invention, the compound to be prepared in accordance with the first aspect of the present invention comprises X=CH and Y=C(O)NR¹R², and the method for its preparation comprises an amide coupling reaction between NHR¹R² and a compound having formula (IV) 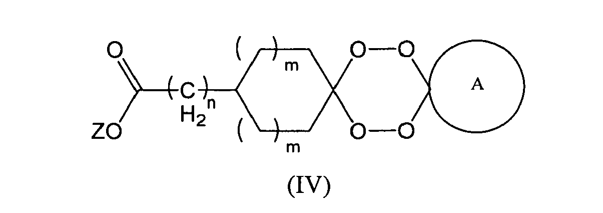 wherein Z=H or alkyl.

It is preferred that compound (IV) is prepared by reacting compound (V) with compound (Ib)

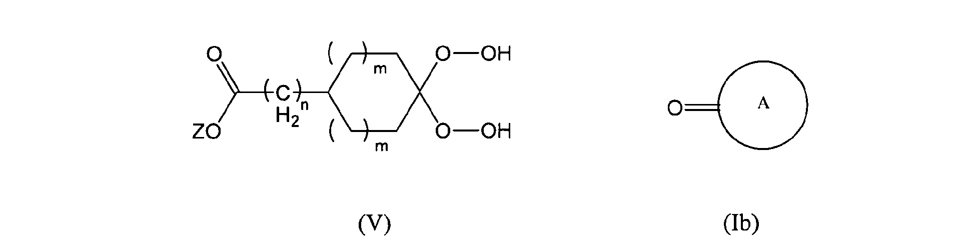

Preferably compound (V) is prepared by oxidising compound (VI)

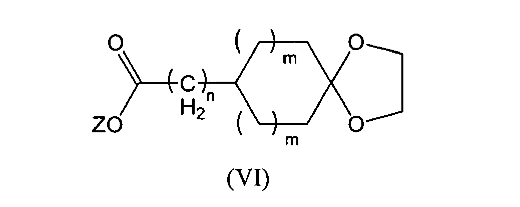

Oxidation of compound (VI) may be effected using any suitable oxidising agent but it is preferably effected by the addition of hydrogen peroxide.

Where the compound according to the first aspect of the present invention is to be prepared where n=1 to 4, it is preferred that compound (VI) is prepared by reacting compound (VII) with compound (VIII) under conditions to facilitate a Wittig reaction between said compounds and subsequently hydrogenating the resulting C=C bond formed as a result of said Wittig reaction.

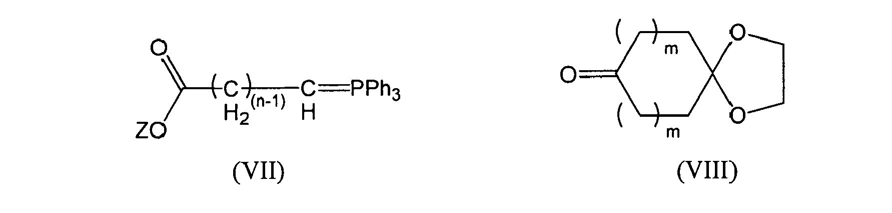

In a preferred embodiment of the method for the production of a compound according to the first aspect of the present invention, the method comprises reacting a ketone compound (Ic) with an oxidising agent in a reaction mixture so as to oxidise said ketone (Ic) to provide a bishydoperoxide compound (Ia) and adding a ketone compound (Ib) to said reaction mixture so as to react compound (Ia) with said ketone (Ib), said oxidising reaction and said reaction of compound (Ia) with compound (Ib) being effected in the presence of a fluorinated alcoholic solvent.

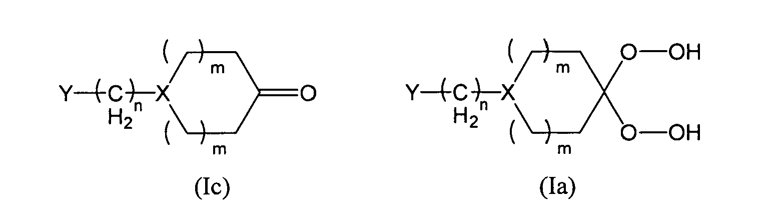

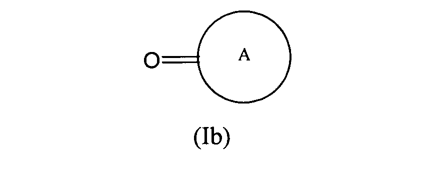

The fluorinate solvent is preferably 1,1,1,3,3,3-hexafluoro-2-propanol.

When any of the foregoing substituents are designated as being optionally substituted, the substituent groups which are optionally present may be any one or more of those customarily employed in the development of pharmaceutical compounds and/or the modification of such compounds to influence their structure/activity, stability, bioavailability or other property. Specific examples of such substituents include, for example, halogen atoms, nitro, cyano, hydroxyl, cycloalkyl, alkyl, alkenyl, haloalkyl, cycloalkyloxy, alkoxy, haloalkoxy, amino, alkylamino, dialkylamino, formyl, alkoxycarbonyl, carboxyl, alkanoyl, alkylthio, alkylsulphinyl, alkylsulphonyl, alkylsulphonato, arylsulphinyl, arylsulphonyl, arylsulphonato, carbamoyl, alkylamido, aryl, aralkyl, optionally substituted aryl, heterocyclic and alkyl- or aryl-substituted heterocyclic groups. A halogen atom may be fluorine, chlorine, bromine or iodine atom and any group which contains a halo moiety, such as a haloalkyl group, may thus contain any one or more of these halogen atoms.

The compound of the first aspect of the present invention may take a number of different forms depending, in particular on the manner in which the compound is to be used. Thus, for example, the compound may be provided in the form of a powder, tablet, capsule, liquid, ointment, cream, gel, hydrogel, aerosol, spray, micelle, transdermal patch, liposome or any other suitable form that may be administered to a person or animal. It will be appreciated that the vehicle of the compound of the invention should be one which is well tolerated by the subject to whom it is given and enables delivery of the compound to the required location.

The compound may be administered orally either in liquid or solid composition form. Compositions suitable for oral administration include solid forms, such as pills, capsules, granules, tablets, and powders, and liquid forms, such as solutions, syrups, elixirs, and suspensions. Forms useful for parenteral administration include sterile solutions, emulsions, and suspensions.

The compound of the invention may be used in a number of ways. For instance, systemic administration may be required in which case the compound may, for example, be ingested orally in the form of a tablet, capsule or liquid. Alternatively the compound may be administered by injection into the blood stream. Injections may be intravenous (bolus or infusion) or subcutaneous (bolus or infusion). The compounds may be administered by inhalation (e.g. intranasally).

The compound may also be administered centrally by means of intrathecal delivery.

The compound may also be incorporated within a slow or delayed release device. Such devices may, for example, be inserted on or under the skin and the compound may be released over weeks or even months. The devices may be particularly advantageous when a compound is used which would normally require frequent administration (e.g. at least daily ingestion of a tablet or daily injection).

It will be appreciated that the amount of a compound required is determined by biological activity and bioavailability which in turn depends on the mode of administration, the physicochemical properties of the compound employed and whether the compound is being used as a monotherapy or in a combined therapy. The frequency of administration will also be influenced by the above mentioned factors and particularly the half-life of the compound within the subject being treated.

Optimal dosages of the compound to be administered may be determined by those skilled in the art, and will vary with the particular compound in use, the strength of the preparation, the mode of administration, and the advancement of the disease condition. Additional factors depending on the particular subject being treated will result in a need to adjust dosages, including subject age, weight, gender, diet, and time of administration.

Known procedures, such as those conventionally employed by the pharmaceutical industry (e.g. *in vivo* experimentation, clinical trials, etc.), may be used to establish specific formulations of compounds and compositions and precise therapeutic regimes (such as daily doses of the compounds and the frequency of administration).

Generally, a daily dose of between 0.01 µg/kg of body weight and 1.0 g/kg of body weight of the inventive compound may be used depending upon which specific compound is used. More preferably, the daily dose is between 0.01 mg/kg of body weight and 100 mg/kg of body weight.

Daily doses may be given as a single administration (e.g. a daily tablet for oral consumption or as a single daily injection). Alternatively, the compound used may require administration twice or more times during a day. As an example, patients may be administered as two or more daily doses of between 25 mgs and 5000 mgs in tablet form. A patient receiving treatment may take a first dose upon waking and then a second dose in the evening (if on a two dose regime) or at 3 or 4 hourly intervals thereafter. Alternatively, a slow release device may be used to provide optimal doses to a patient without the need to administer repeated doses.

This invention provides a pharmaceutical composition comprising a therapeutically effective amount of the compound of the invention and, preferably, a pharmaceutically acceptable vehicle. In the subject invention a "therapeutically effective amount" is any amount of a compound or composition which, when administered to a subject suffering from a disease against which the compounds are effective, causes reduction, remission, or regression of the disease. A "subject" is a vertebrate, mammal, domestic animal or human being. In the practice of this invention the "pharmaceutically acceptable vehicle" is any physiological vehicle known to those of ordinary skill in the art useful in formulating pharmaceutical compositions.

In one embodiment, the amount of the compound in the composition according to the the present invention is an amount from about 0.01 mg to about 800 mg. In another embodiment, the amount of the compound is an amount from about 0.01 mg to about 500 mg. In another embodiment, the amount of the compound is an amount from about 0.01 mg to about 250 mg. In another embodiment, the amount of the compound is an amount from about 0.1 mg to about 60 mg. In another embodiment, the amount of the compound is an amount from about 1 mg to about 20 mg.

In one embodiment, the pharmaceutical vehicle employed in the composition of the present invention may be a liquid and the pharmaceutical composition would be in the form of a solution. In another embodiment, the pharmaceutically acceptable vehicle is a solid and the composition is in the form of a powder or tablet. In a further embodiment, the pharmaceutical vehicle is a gel and the composition is in the form of a suppository or cream. In a further embodiment the compound or composition may be formulated as a part of a pharmaceutically acceptable transdermal patch.

A solid vehicle employed in the composition according to the present invention can include one or more substances which may also act as flavoring agents, lubricants, solubilizers, suspending agents, fillers, glidants, compression aids, binders or tablet-disintegrating agents; it can also be an encapsulating material. In powders, the vehicle is a finely divided solid which is in admixture with the finely divided active ingredient. In tablets, the active ingredient is mixed with a vehicle having the necessary compression properties in suitable proportions and compacted in the shape and size desired. The powders and tablets preferably contain up to 99% of the active ingredient. Suitable solid vehicles include, for example, calcium phosphate, magnesium stearate, talc, sugars, lactose, dextrin, starch, gelatin, cellulose, polyvinylpyrrolidine, low melting waxes and ion exchange resins.

Liquid vehicles may be used in preparing solutions, suspensions, emulsions, syrups, elixirs and pressurized compositions according to the present invention. The compound of the first aspect of the present invention can be dissolved or suspended in a pharmaceutically acceptable liquid vehicle such as water, an organic solvent, a mixture of both or pharmaceutically acceptable oils or fats.

The liquid vehicle can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid vehicles for oral and parenteral administration of the compound forming the first aspect of the present invention include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the vehicle can also be an oily ester such as ethyl oleate and isopropyl myristate. Sterile liquid vehicles are useful in sterile liquid form compositions for parenteral administration. The liquid vehicle for pressurized compositions can be halogenated hydrocarbon or other pharmaceutically acceptable propellent.

The compound forming the first aspect of the present invention can be administered orally in the form of a sterile solution or suspension containing other solutes or suspending agents (for example, enough saline or glucose to make the solution isotonic), bile salts, acacia, gelatin, sorbitan monoleate, polysorbate 80 (oleate esters of sorbitol and its anhydrides copolymerized with ethylene oxide) and the like.

Liquid pharmaceutical compositions which are sterile solutions or suspensions can be utilized by for example, intramuscular, intrathecal, epidural, intraperitoneal or subcutaneous injection. Sterile solutions can also be administered intravenously. The inventive compounds may be prepared as a sterile solid composition according to the present invention which may be dissolved or suspended at the time of administration using sterile water, saline, or other appropriate sterile injectable medium. Vehicles are intended to include necessary and inert binders, suspending agents, lubricants, flavorants, sweeteners, preservatives, dyes, and coatings.

The compound forming part of the present invention is eminently suitable for use in prophylactic treatment. By the term "prophylactic treatment" we include any treatment applied to prevent, or mitigate the effect of a disorder. The prophylactic treatment may be given, for example, periodically to a person who is of a predetermined minimum age or who is genetically predisposed to a disorder. Alternatively the prophylactic treatment may be given on an *ad hoc* basis to a person who is to be subjected to conditions which might make the onset of a disorder more likely.

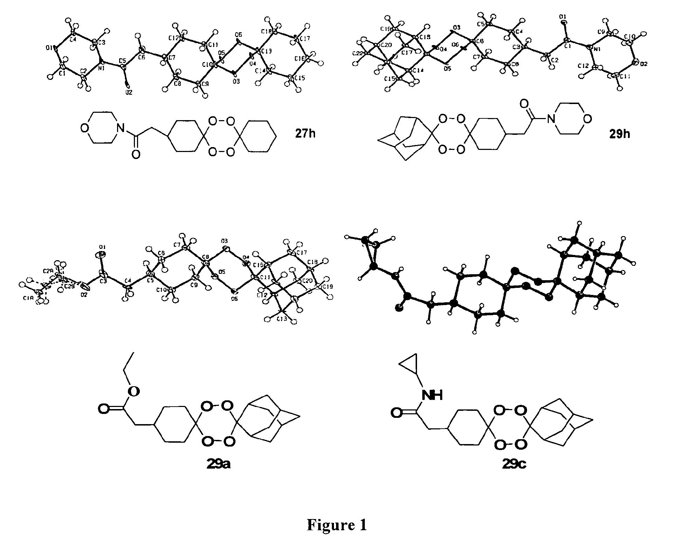

The invention will be further described by way of example only with reference to the following non-limiting Examples and Figure 1 which shows single crystal X-ray structures of compounds **27h, 29a, 29c** and **29h**.

### EXAMPLES

### Structure A Derivatives

Compounds of formula (I) wherein m=1, n=0, X=CH and Y=NHR², where R² is selected from the group consisting of H, substituted or unsubstituted alkyl, substituted or unsubstituted aryl, substituted or unsubstituted amine, substituted or unsubstituted carbocyclic ring, substituted or unsubstituted heterocyclic ring, or any combination thereof.

The initial target molecule was prepared by the method reported by Iskra et al. (Scheme 1) in which cyclohexanone **5** and 1,4-cyclohexanedione **6** are allowed to react in a two step sequence.

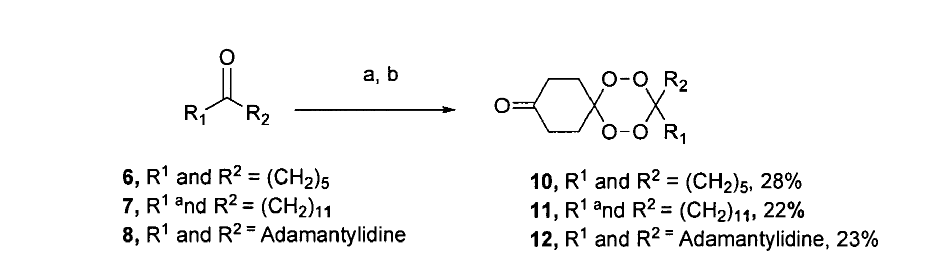

**Scheme 1.** Reagents and conditions: a) 30% H₂O₂ (2 equiv.), MTO (0.1equiv.) /TFE-0.5M, b) [1,4-cyclohexanedione-9 (2 equiv.), EtOAc/HBF₄(1 equiv.)]

The required 1,2,4,5-tetraoxane **10**, formed by cross-condensation of the bishydroperoxide and the 1,4-cyclohexanedione, was obtained in rather low yield. A significant amount of the symmetrical 1,2,4,5-tetraoxane, resulting from competitive homo-cyclocondensation of bishydroperoxide, was also recovered with a small amount of the trimeric cyclic peroxide.

To avoid the formation of any trimeric product any excess hydrogen peroxide was removed by carrying out a two step synthesis of the tetraoxanes; first by preparing the bishydroperoxide and removing any unreacted hydrogen peroxide followed by the tetraoxane formation reaction (Scheme 2). The yield of the required tetraoxane was improved slightly.

Various methodologies available for the formation of the bishydroperoxide were investigated and the method reported by Ledaal and co workers¹ was identified. Performing the reaction in acetonitrile led to the elimination of the formation of a solid mass in the flask leading to quantitative conversion of the ketone to the bishydroperoxide.

While some methodologies led to an exclusive formation of the symmetrical tetraoxanes, others led to the formation of compound **13**.

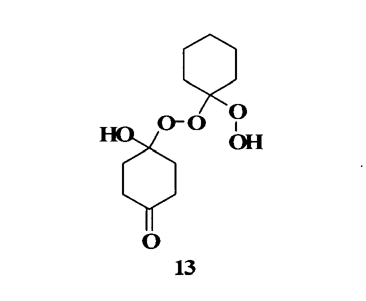

Several attempts to close the ring according to existing literature¹ procedures failed.

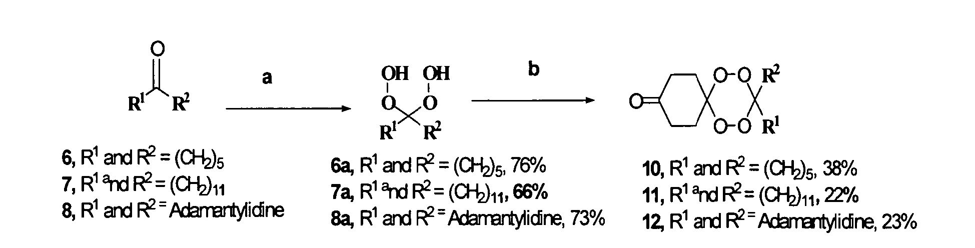

**Scheme 2**. Reagents and conditions: a) H₂O₂ 30% (2 equiv.), CH₃CN, 0°C, 4min., b) 1,4-cyclohexanedione-9 (2 equiv.), EtOAc, HBF₄ (1 equiv.)

Reductive amination² of the ketone with various amino compounds afforded compounds 14-19 in moderate to good yields.(20-85%) (Scheme 3)

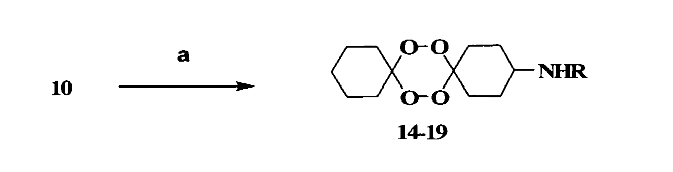

| | |
|---|---|
| **14** R = CH(CH₂)₂, 55% | **17** R = CH₂CH₂N(CH₂)₄O, 47% |
| **15** R = CH₂CH₂N(CH₂)₄, 85% | **18** R = CH₂CH₂N(C₂H₅)₂, 20% |
| **16** R = CH₂CH₂N(CH₂)₅, 60% | **19** R = (CH₂)₄O, 56% |

**Scheme 3**. Reagents and conditions: a) RNH₂ (1.3 equiv.), NaBH(OAc)₃ (1.3 equiv.), CH₂Cl₂.

### Structure B Derivatives

Compounds of formula (I) wherein m=1, n=1, X=CH and Y=C(O)NR¹R², where R¹ and R² are each individually selected from the group consisting of H, substituted or unsubstituted alkyl, substituted or unsubstituted aryl, substituted or unsubstituted amine, substituted or unsubstituted carbocyclic ring, substituted or unsubstituted heterocyclic ring, or any combination thereof, or R¹ and R² are linked so as to form part of a substituted or unsubstituted heterocyclic ring.

Structure B derivatives were prepared via an alternative route by first carrying out a Wittig reaction between 1,4-cyclohexanedionemo-noethylketal with the appropriate ylide (Scheme 4). Hydrogenation in the presence of Palladium on charcoal afforded the required starting material **17.** The bishydroperoxide formed was condensed with various ketones to afford the corresponding tetraoxanes **27a, 28a, 29a, 30a**. Hydrolysis, followed by amide coupling reactions led to various water-soluble analogues listed in Table 1.

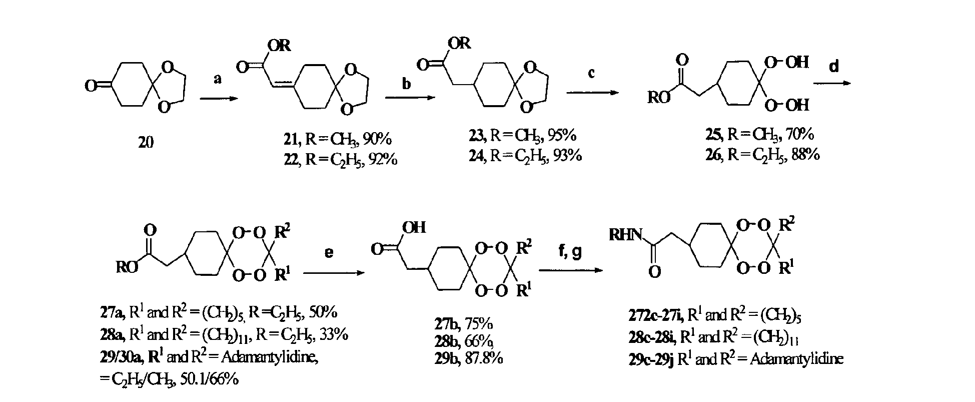

**Scheme 4**. Reagents and conditions: a) Ph₃P=CHCO₂R, R = CH₃/C₂H₅ (1.1 equiv), benzene/toluene, reflux, 24hrs, b) Pd/C, H₂, EtOAc, c) H₂WO₄(2 equiv.), THF, 30% H₂O₂(2 equiv.), 0°C, 48hrs d) cyclohexanone/cyclododecanone/adamantanone (2 equiv.), EtOAc, HBF₄ (1 equiv.), e) KOH (5.5 equiv.), CH₃OH, 70°C, 1Hr, f) Et₃N (1 equiv.), ClCO₂C₂H₅ (1.3 equiv.), c, 0°C, 1hr. g) RNH₂ (2 equiv.), 0°C- r.t.

**Table 1. Yields for Amide Synthesis.**

| | | |
|---|---|---|
| 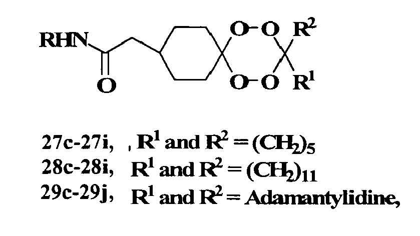 | | |

| **Acid** | **Amide Product** | **Yield (%)** |
|---|---|---|
| 27b | 27c, R= CH(CH₂)₂ | 85 |
| 27b | 27d, R = CH₂CH₂N(CH₂)₄ | 78 |
| 27b | 27e, R = CH₂CH₂N(CH₂)ₛ | 81 |
| 27b | 27f, R = CH₂CH₂N(CH₂)₄O | 76 |
| 27b | 27g, R= CH₂CH₂N(C₂H₅)₂ | 58 |
| 27b | 27h, R = (CH₂)₄O | 84 |
| 27b | 27i, R =CH₂CO₂CH₃ | 45 |
| 28b | 28c, R= CH(CH₂)₂ | 88 |
| 28b | 28d, R = CH₂CH₂N(CH₂)₄ | 81 |
| 28b | 28e, R = CH₂CH₂N(CH₂)₅ | 82 |
| 28b | 28f, R = CH₂CH₂N(CH₂)₄O | 78 |
| 28b | 28g, R= CH₂CH₂N(C₂H₅)₂ | 74 |
| 28b | 28h, R =(CH₂)₄O | 90 |
| 28b | 28i, R =(CH₂)₄S | 78 |
| 29b | 29c, R= CH(CH₂)₂ | 83 |
| 29b | 29d, R = CH₂CH₂N(CH₂)₄ | 80 |
| 29b | 29e, R = CH₂CH₂N(CH₂)₅ | 78 |
| 29b | 29f, R = CH₂CH₂N(CH₂)₄O | 77 |
| 29b | 29g, R= CH₂CH₂N(C₂H₅)₂ | 66 |
| 29b | 29h, R = (CH₂)₄O | 81 |
| 29b | 29i, R = (CH₂)₄S | 77 |
| 29b | 29j, R =CHCH(CH₃)₂CO₂CH₃ | 69 |
| 28b | 31; R = (CH₂)₄SO₂ | 92 |
| 29b | 32; R = (CH₂)₄SO₂ | 88 |

For analogues **27h, 29a, 29c** and **29h** crystals were grown by slowly evaporating a dichloromethane/hexane mixture and the single crystal X-ray structures were solved for these two tetraoxanes (Figure 1).

Compounds **28i** and **29i** were converted into the corresponding sulfones **31** and **32** using excess amount of m-Chloroperbenzoic acid in dichloromethane in excellent yields.

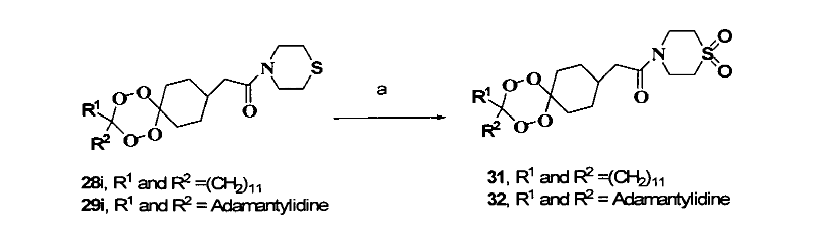

**Scheme 5.** Reagents and conditions: (a) *m*-CPBA (2.2 equiv.), CH₂Cl₂, room temperature, 4-6hrs.

Preliminary *in vitro* antimalarial data indicated that the amides containing the adamantylidine group were the most active so the synthesis of a wider range of adamantylidine amides **29k-29w** was undertaken (Table 2).

**Table 2. Yields for extended amide synthesis**

| | | | | | |
|---|---|---|---|---|---|
| 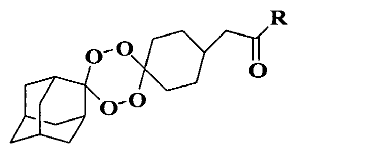 | | | | | |

| **Compound** | R | % Yield | **Compound** | R | % Yield |
|---|---|---|---|---|---|
| **29k** | 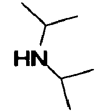 | 83 | **29r** | **HN-NH₂** | 72 |
| **29l** | 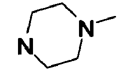 | 87 | **29s** | 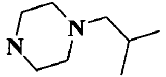 | 87 |
| **29m** | 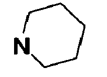 | 89 | **29t** | 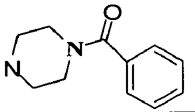 | 68 |
| **29n** | **NH₂** | 43 | **29u** | 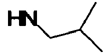 | 73 |
| **29o** | 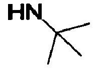 | 83 | **29v** | 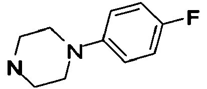 | 77 |
| **29p** | 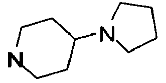 | 76 | **29w** | 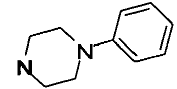 | 64 |
| **29q** | 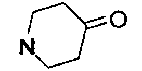 | 80 | **29x** | 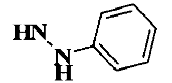 | 70 |

### Structure C Derivatives

Compounds of formula (I) where in m=1, n=0, X=N and Y=-S(O)₂R³ or -C(O)R³, where R³ is selected from the group consisting of H, substituted or unsubstituted alkyl, substituted or unsubstituted aryl, substituted or unsubstituted amine, substituted or unsubstituted carbocyclic ring, substituted or unsubstituted heterocyclic ring or any combination thereof.

Structure C derivatives **39a-i** and **40a-d** were prepared as shown below. The process involved a one step procedure in which the sulfonyl piperidinones **38a-i** were oxidised to the gem-dihydroperoxide *in situ* using 2 equivalents of hydrogen peroxide and approximately 0.1 mol% of methyltrioxorhenium (MTO). The second ketone was then added along with 2 equivalents of HBF₄ to give selectively the non-symmetric tetraoxanes **39a-i** and **40a-d** in 25-65% yields. The use of fluorous alcohols as the solvent for these reactions is important to this selectivity with 1,1,1,3,3,3-hexafluoro-2-propanol (HFIP) being used in this case. This one-pot methodology allows the rapid synthesis of non-symmetrical tetraoxanes without the use of an excess of hydrogen peroxide or the need to isolate the potentially explosive dihydroperoxide intermediate.

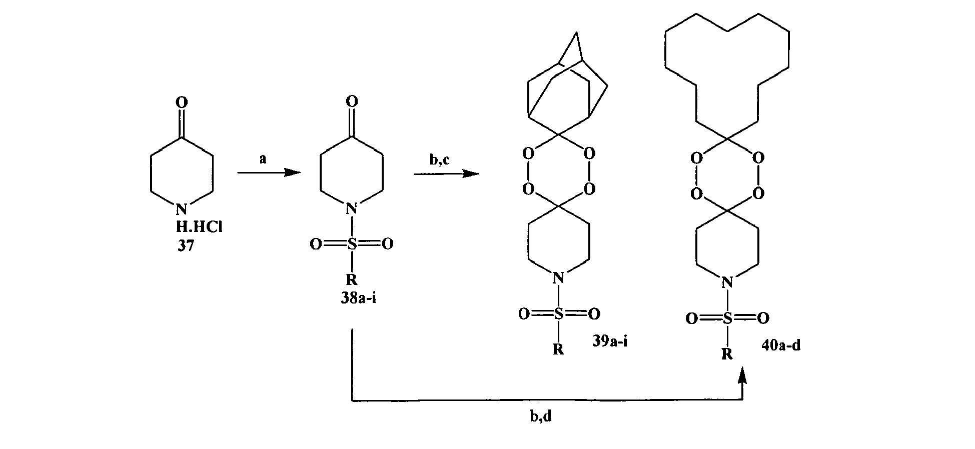

**Scheme 6**. Reagents and conditions: (a) RSO₂Cl, K₂CO₃, DCM, H₂O, rt, overnight (b) H₂O₂, MTO, HFIP, rt, 1h30mins (c) HBF₄, adamantanone, rt, 1h (d) HBF₄, cyclododecanone, rt, 1h.

Adamantanone and cyclododecanone have both been successfully incorporated in good yields (Table 3 and Table 4). The range of compounds with cyclododecanone incorporated was limited as it rapidly became apparent from preliminary *in vitro* test results that these compounds were less active than their adamantane counterparts.

**Table 3: Yields of adamantane dispiro compounds**

| COMPOUND | R | % YIELD **38** | % YIELD **39** |
|---|---|---|---|
| **a** | Me | 62 | 61 |
| **b** | Et | 59 | 60 |
| **c** | *i*-Pr | 52 | 56 |
| **d** | Cp | 59 | 53 |
| **e** | CH₂CF₃ | 62 | 51 |
| **f** | Ph | 98 | 36 |
| **g** | *p*-FPh | 98 | 41 |
| **h** | *p*-ClPh | 99 | 38 |
| **i** | *p*-CF₃Ph | 95 | 25 |

**Table 4: Yields of cyclododecanone dispiro compounds**

| COMPOUND | R | % YIELD |
|---|---|---|
| **40a** | Me | 36 |
| **40b** | Et | 32 |
| **40c** | *i*-Pr | 38 |
| **40d** | Ph | 20 |

Preparation of a further example of a structure C derivative, tetraoxane **34n**, was investigated. First, a reflux reaction between 4-piperidinone monohydrate hydrochloride **34a** and an appropriate carbonyl chloride afforded the corresponding piperidinone **34h** (Scheme 7).

**Scheme 7.** Reagents and conditions: (a) Toluene, reflux, triethylamine, 12 hours, carbonyl chloride.

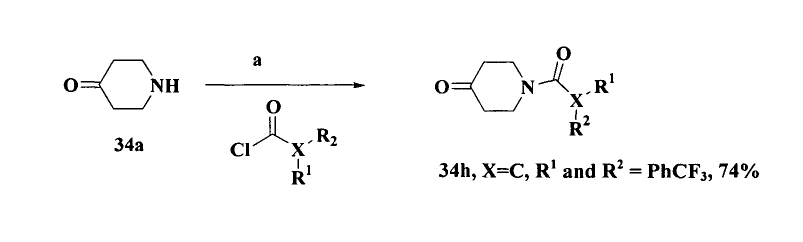

1,2-dihydroperoxycyclohexane **6a** was prepared and condensed with piperidinone **34h** to afford the target tetraoxane **34n** (Scheme 8).

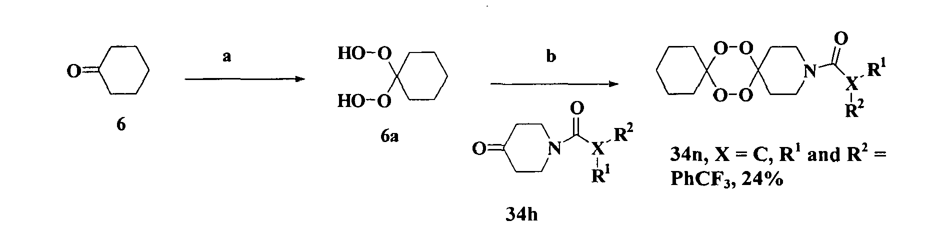

**Scheme 8**. Reagents and conditions: (a) HCOOH, CH₃CN, 30% H₂O₂ ,0°C, 15min (b) EtOAc, HBF₄, rtp.

### Structure D Derivatives

Compounds of formula (I) wherein m=1, n=1, X=CH and Y=S(O)₂R⁴, where R⁴ is selected from the group consisting of H, substituted or unsubstituted alkyl, substituted or unsubstituted aryl, substituted or unsubstituted amine, substituted or unsubstituted carbocyclic ring, substituted or unsubstituted heterocyclic ring or any combination thereof.

The presence of a sulfonyl group in place of the amide was also investigated. Diethylmethyl thiomethyl phosphorane **33a** was oxidized using *m*CPBA to the corresponding sulfone **33b.** The sulfone was then reacted with 1,4-cyclohexane dione monoethylene ketal via a Wittig reaction to the corresponding vinyl compound **33c.** Hydrogenation afforded the required starting material **33d** which was oxidized with hydrogen peroxide to the gem-dihydroperoxide **33e** and condensed with cyclohexanone/2-adamantanone to the corresponding tetraoxanes **33f and 33g** (Scheme 9).

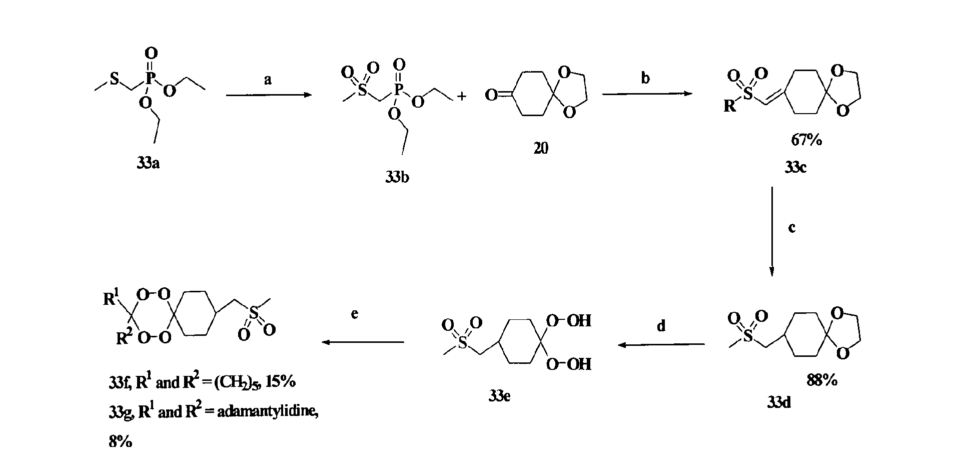

**Scheme 9**. Reagents and conditions: (a) *m*CPBA, CH₂Cl₂ (b) THF, -78°C or THF, NaH, -10°C (c) ethylacetate, H₂, 10% Pd/C (d) THF, H₃WO₄, 0°C, 48 hours (e) Ketone, ethylacetate, HBF₄

### Structure E Derivatives

Compounds of formula (I) wherein m=1, n=0, X=N and Y=C(O)R³, where R³ is a substituted or unsubstituted amine group or a substituted or unsubstituted heterocyclic ring containing a nitrogen atom where said nitrogen atom connects the heterocyclic ring to the carbonyl carbon atom or group Y.

Further, preparations of Urea type tetraoxanes **34j-o** were investigated. First, a reflux reaction between 4-piperidinone monohydrate hydrochloride **34a** and an appropriate carbonyl chloride afforded the corresponding piperidinones **34c-g** (Scheme 10).

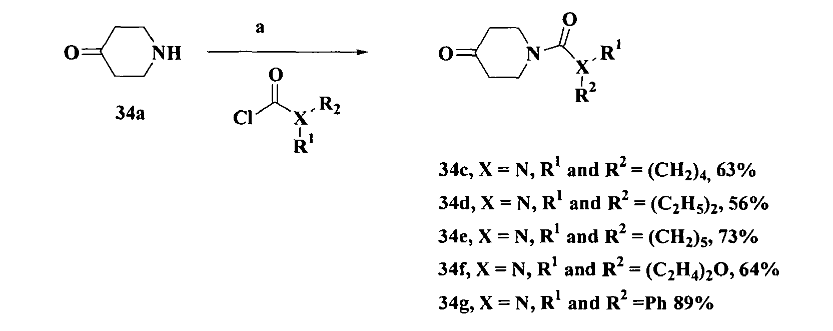

**Scheme 10**. Reagents and conditions: (a) Toluene, reflux, triethylamine, 12 hours, carbonyl chloride.

Three pathways were then explored to prepare the urea type tetraoxanes. First, the gem-dihydroperoxide **34i** was prepared using the formic acid procedure of the piperidinone **34e** and condensed with cyclohexanone or 2-adamantanone to the target tetraoxanes **34j** and **34k** (Scheme 11).

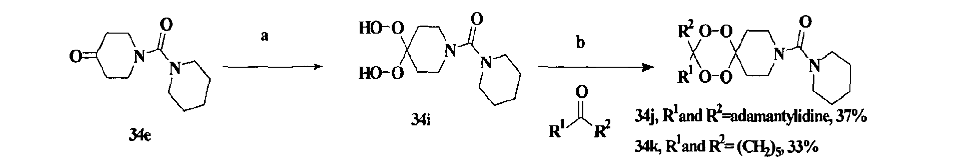

**Scheme 11.** Reagents and conditions: (a) HCOOH, CH₃CN, 30% H₂O₂, 0°C, 15min (b) diethylether, BF3.OEt₂

Alternatively, 1,2-dihydroperoxycyclohexane **6a** was prepared and condensed with the appropriate piperidinone to afford the target tetraoxanes **341-o** (Scheme 12).

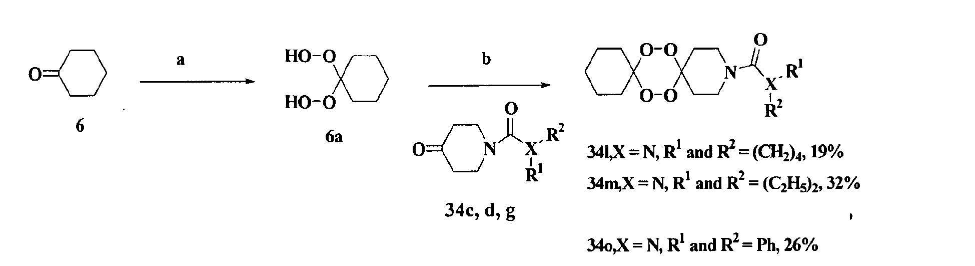

**Scheme 12.** Reagents and conditions: (a) HCOOH, CH₃CN, 30% H₂O₂,0°C, 15min (b) EtOAc, HBF₄, rtp.

An alternative strategy using a one pot methodology for conversion to the *gem-*dihydroperoxide followed by tetraoxane formation was also investigated to give the morpholine urea **34p** (Scheme 13).

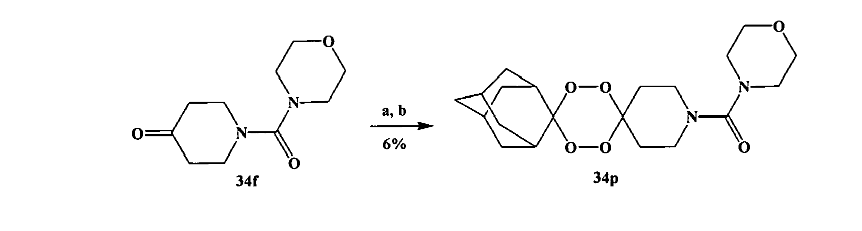

**Scheme 13.** Reagents and conditions: (a) H₂O₂, MTO, HFIP, 1h30min (b) HBF₄, Adamantanone, 1h.

### Structure F Derivatives

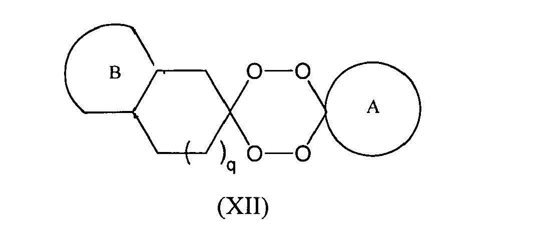

wherein ring A represents a substituted or unsubstituted monocyclic or multicyclic ring; q=any integer; and ring B represents a fused substituted or unsubstituted monocyclic or multicyclic ring.

In addition, we investigated the preparation of the diaspiro1,2,4,5-tetraoxanes using 2-indanone **35a** and β-tetralones **36a**. The gem-dihydroperoxides **35b** and **36b** prepared by treating 2-indanone and β-tetralone with 30% H₂O₂ were condensed with cyclohexanone and 2-adamantanone to give tetraoxanes **35c, 35d, 36c** and **36d**. The reactions are low yielding. Nevertheless, the required compounds were obtained (Scheme 14).

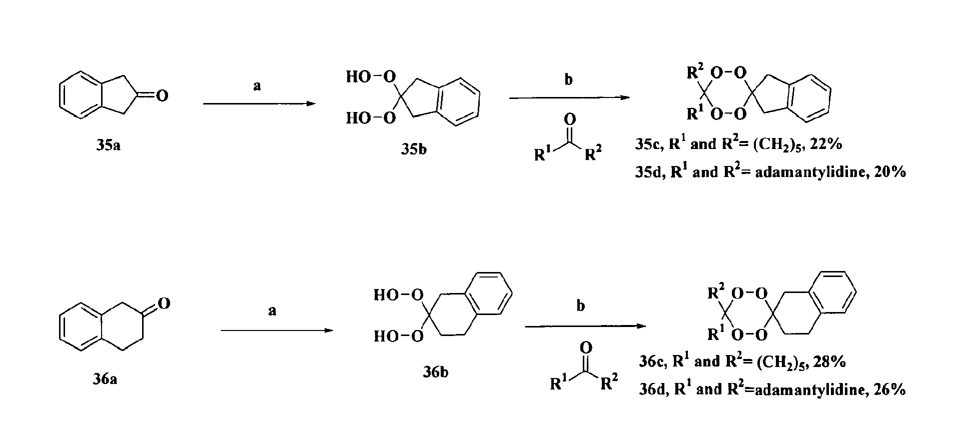

**Scheme 14.** Reagents and conditions: (a) HCOOH, CH₃CN, 30% H₂O₂, 0°C, 15min (b) EtOAc, HBF₄, rtp.

### Structure G Derivatives

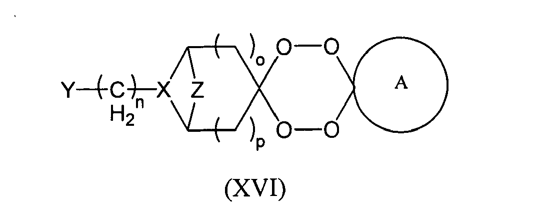

wherein ring A represents a substituted or unsubstituted monocyclic or multicyclic ring; o=any integer; p=any integer; n=0-5; Z represents a bridging group; and X=CH and Y=C(O)NR¹R², -NR¹R² or -S(O)₂R⁴, where R¹, R² and R⁴ are each individually selected from the group consisting of H, substituted or unsubstituted alkyl, substituted or unsubstituted aryl, substituted or unsubstituted amine, substituted or unsubstituted carbocyclic ring, substituted or unsubstituted heterocyclic ring, or any combination thereof, or R¹ and R² are linked so as to form part of a substituted or unsubstituted heterocyclic ring, or X=N and Y=-S(O)₂R³ or - C(O)R³, where R³ is selected from the group consisting of H, substituted or unsubstituted alkyl, substituted or unsubstituted aryl, substituted or unsubstituted amine, substituted or unsubstituted carbocyclic ring, substituted or unsubstituted heterocyclic ring or any combination thereof.

The increased activity of the adamantane systems compared to the cyclododecane systems lead us to postulate that the fused, more rigid adamantane ring has a stabilising effect. The effect of having a more rigid ring system at the other end of the molecule was also investigated using tropinone **41a** as the starting material. Commercially available tropinone **41a** was demethylated, sulfonated and subjected to the one pot reaction described above to give the anticipated tetraoxane **41d** in a reasonable yield (Scheme 15).

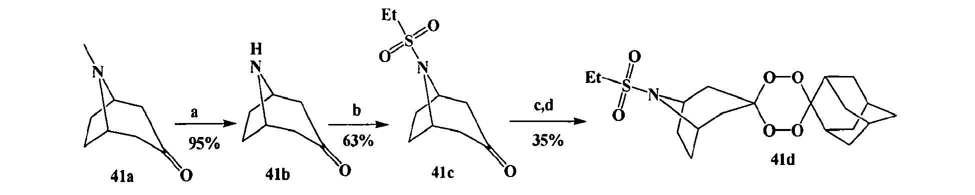

**Scheme 15**. Reagents and conditions: (a) 1-chloroethyl chloroformate, DCE, 12h, reflux (b) triethylamine, Et SO₂Cl, DCM, overnight, rt (c) H₂O₂, MTO, HFIP, 1h30min, rt (d) HBF₄, adamantanone, 1h, rt. **Biological Activity**

A selection of the 1,2,4,5-tetraoxanes were tested against the 3D7 strain of the *Plasmodium falciparum* and the results are summarized in Table 5 below. Most of the analogues have comparable antimalarial IC₅₀ values to the naturally occurring peroxide artermisinin. The adamantane analogues of the tetraoxanes and their corresponding amide have a better activity than their cyclohexanone and cyclododecanone counterparts.

**Table 5. In Vitro Antimalarial Activity of 1,2,4,5-tetraoxanes (10-34k) versus 3D7* strain of Plasmodium falciparum**

| **Compound** | **^{a}Mean IC₅₀ (nM)** |
|---|---|
| Artemether | 1.7 |
| Chloroquine | 8.5 |
| Artemisinin | 9.5 |
| 10 | 6.0 |
| 14 | 20.0 |
| 16 | 28.1 |
| 19 | 29.4 |
| 27a | 24.2 |
| 27d | 19.1 |
| 27e | 19.2 |
| 27f | 19.1 |
| 27g | 5.15 |
| 27h | 22.2 |
| 28c | 18.7 |
| 28h | 23.7 |
| 28i | 26.9 |
| 29c | 2.3 |
| 29h | 5.2 |
| 29i | 5.9 |
| 291 | 0.5 |
| 29m | 0.5 |
| 29s | 2.7 |
| 31 | 92.6 |
| 32 | 24.2 |
| 34f | 4.7 |
| 34i | 7.2 |
| 34k | 7.0 |

^{a}The mean IC₅₀ was calculated from triplicate results. Antimalarial activities were assessed by a previously published protocol.³

A 4-day Peter's suppressive test was performed on a selection of the compounds and the results are summarized in Table 6. The adamantylidine analogues **29c, 29h** and **291** showed 100% inhibition by oral administration at a dose of 30mg/kg; based on this exciting result, several adamantane analogues are currently undergoing full assessment in the 4-day Peter's test to determine ED₅₀ and ED₉₀ values.

**Table 6. Peter's suppressive test results verses Plasmodium yoelli in mice**

| | | | |
|---|---|---|---|
| 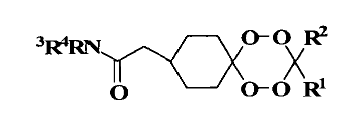 | | | |

| **Compound** | **R¹ and R²** | **R³ and R⁴** | **Percentage of inhibition at 30mg/kg (p.o)** |
|---|---|---|---|
| **27c** | (CH₂)₅- | H and CH(CH₂)₂ | 24.8 |
| **27h** | (CH₂)₅- | (CH₂)₄O- | 33.0 |
| **29c** | Adamantylidene- | H and CH(CH₂)₂ | 100 |
| **29h** | Adamantylidene- | (CH₂)₄O- | 100 |
| **291** | Adamantylidene- | (C₂H₄)₂NCH₃ | 100 |
| **Artesunate** | - | - | 100 |
| **Artemether** | - | - | 100 |

It is clear that the adamantyl based systems **29c**, **29h** and **29l** have unprecedented *in vivo* levels of antimalarial activity for the tetroxane class of drug.

Compound **29c, 29h** and **29l** were then subjected to dose response experiment against the *P. berghei* ANKA and the results are summarized in **Table 7** below.

**Table 7: In vivo Screening Against the Chloroquine Resistant Strains of the P. Berghei ANKA.**

| | | | | |
|---|---|---|---|---|
| 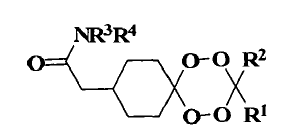 | | | | |

| **Compound** | **R¹ and R²** | **R³ and R⁴** | **ED50(mg/kg)** | **ED90(mg/kg)** |
|---|---|---|---|---|
| **29c** | Adamantylidene | H and CH(CH₂)₂ | 10.27 | 20.33 |
| **29h** | Adamantylidene | (CH₂)₄O | 3.18 | 3.88 |
| **29l** | Adamantylidine | (C₂H₄)₂NCH₃ | 2.70 | - |
| Artemether | - | - | 5.88 | 10.57 |

### Sulfonamide dispiro 1,2,4,5-tetraoxanes

**Table 8: IC₅₀ values for sulfonamide dispiro 1,2,4,5-tetraoxanes**

| | | | |
|---|---|---|---|
| 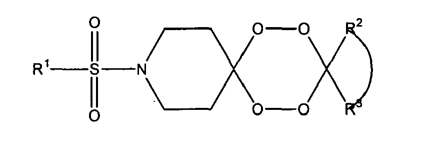 | | | |

| Compound | R¹ | R² and R³ | Mean IC₅₀ (nM) |
|---|---|---|---|
| Artemether | | | 3.20 |
| Artemisinin | | | 9.20 |
| 39a | Me | Adamantyl | 10.18 |
| 40a | Me | Cyclododecyl | 27.75 |
| 39b | Et | Adamantyl | 5.55 |
| 40b | Et | Cyclododecyl | 29.13 |
| 39c | iPr | Adamantyl | 5.87 |
| 40c | iPr | Cyclododecyl | 86.37 |
| 39d | Cp | Adamantyl | 3.52 |
| 39e | CH₃CF₃ | Adamantyl | 14.35 |
| 39f | Ph | Adamantyl | 8.10 |
| 40d | Ph | Cyclododecyl | 131.07 |
| 39g | p-FPh | Adamantyl | 16.73 |
| 39h | p-ClPh | Adamantyl | 22.73 |
| 39i | p-CF₃ | Adamantyl | 20.73 |
| | | | |
| 41d | Et* | Adamantyl | 60.57 |

| | | | |
|---|---|---|---|
| *Note: ring carbon atoms bonded directly to ring nitrogen atom further linked via briding ethylene moiety. | | | |

*In Vitro* IC₅₀ test results show that the majority of these compounds have activity in the 3-30 nM region. There are clear trends in the SAR required for maximum activity. The presence of an adamantyl group **39a-i** greatly increases activity. Smaller alkyl groups at R¹ **39a-e** as apposed to larger aromatic groups **39f-i** also increase activity (Table 8). The presence of the tropinone group resulted in a loss in activity **41d.** Compounds **39b** and **39d** were selected for *in vivo* screening.

**Table 9: In vivo screening of 39b and 39d against P. berghei ANKA infected mice**

| **Compound** | **ED50 (mg/kg)** | **ED90 (mg/kg)** |
|---|---|---|
| 39b | 6.61 | 35.48 |
| Artesunate | 3.20 | 17.78 |
| | | |

| **Compound** | **ED50 (mg/kg)** | **ED90 (mg/kg)** |
|---|---|---|
| 39d | 7.93 | 49.77 |
| Artesunate | 2.90 | 16.67 |

Note: 94% inhibition was found at 30mg/kg with **39b**, so the ED90 of **39b** is below 30mg/kg but because of the sigmoidal curve calculations is giving 35.48mg/kg

Due to the high *in vitro* and *in vivo* activity of compounds **29h** and **39b** these compounds were selected for *further in vitro* studies.

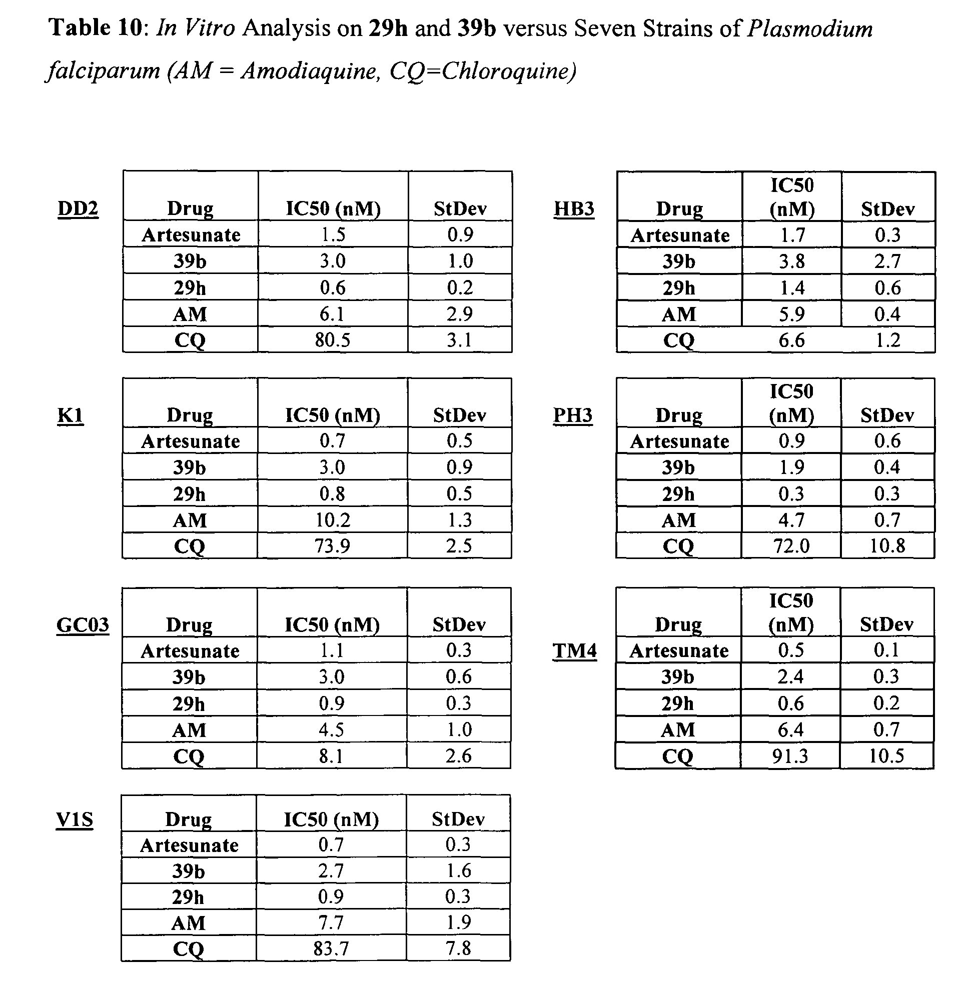

### Antimalarial Activity

The 3D7 strain of Plasmodium falciparum was used in this study. This strain is known to be CQ resistant Parasites were maintained in continuous culture using the method of Jensen and Trager⁴. Cultures were grown in flasks containing human erythrocytes (2-5%) with parasitemia in the range of 1% to 10% suspended in RPMI 1640 medium supplemented with 25 mM HEPES and 32 mM NaHCO₃ and 10% human serum (complete medium). Cultures were gassed with a mixture of 3% O₂, 4% CO₂ and 93% N₂. Antimalarial activity was assessed with an adaption of the 48-h sensitivity assay of Desjardins *et al.*⁵ using [³H]-hypoxanthine incorporation as an assessment of parasite growth. Stock drug solutions were prepared in 100% dimethylsulphoxide (DMSO) and diluted to the appropriate concentration using complete medium. Assays were performed in sterile 96-well microtitre plates, each plate contained 200 µl of parasite culture (2% parasitemia, 0.5% haematocrit) with or without 10 µl drug dilutions. Each drug was tested in triplicate and parasite growth compared to control wells (which consituted 100 % parasite growth). After 24-h incubation at 37°C, 0.5 µCi hypoxanthine was added to each well. Cultures were incubated for a further 24 h before they were harvested onto filter-mats, dried for 1 h at 55°C and counted using a Wallac 1450 Microbeta Trilux Liquid scintillation and luminescence counter. IC₅₀ values were calculated by interpolation of the probit transformation of the log dose - response curve.

### In vivo Antimalarial Screening

Selections of the compounds were screened for *in vivo* activity. In vivo data (Table 6) was determined using 30 mg/kg oral (po) and subcutaneous (sc) doses in a 4 -days Peter's test. For subcutaneous administration, compounds were dissolved in 10%dimethylsulfoxide (DMSO) 0.05% Tween 80 (Sigma, Dorset, UK) in distilled water. For oral admistration, compounds were dissolved in standard suspending formula (SSV) [0.5% sodium carboxymethylcellulose, 0.5% benzyl alcohol, 0.4% Tween 80, 0.9%NaCl (all Sigma)]. Subcutaneous (s.c) or oral (p.o) treatment was done with 0.2ml of a solution of the test compound two hours (day 0) and on days 1, 2, and 3 post infections. Parasitaemia was determined by microscopic examination of Giemsa stained blood films taken on day 4. Microscopic counts of blood films from each mouse were processed using MICROSOFT@EXCELL spreadsheet (Microsoft Corp.) and expressed as percentage of inhibition from the arithmetic mean parasitaemias of each group in relation to the untreated group.

### Cytotoxicity Studies

**Table 11: Cellular Cytotoxicity Screens and Theraputic Index (TI) for Selected Lead Tetraoxanes, 29h, 39b and 39d.**

| **Drug** | **Hep2G** | **L6** | **MRC-5** | **VERO** | **H9c(2-1)** |
|---|---|---|---|---|---|
| **29h** | >50 | 23 | >50 | >50 | >50 |
| **TI** | >16666 | 7666 | >16666 | >16666 | >16666 |
| **39b** | >50 | 31 | >50 | >50 | >50 |
| **TI** | >9090 | 5636 | >9090 | >9090 | >9090 |
| **39d** | >50 | >50 | >50 | >50 | >50 |
| **TI** | >14285 | >14285 | >14285 | >14285 | >14285 |
| **Doxorubicin** | 0.3 | >5 | 2 | >5 | 3 |

HepG2 Human Caucasian hepatocyte carcinoma
H9c2(2-1) Myocardium, heart, rat
L6 Sketal muscle myoblast, rat
Vero Kidney, African green monkey, Cercopithecus aethiops
MRC-5 Embryonal lung, diploid, male, Human

### Values represent Tox 50 in µM. Cytotoxicity measured by Resazurin reduction.

Single full dose response curves generated using 10 independent drug concentrations. The *100* therapeutic index (TI) is the ration of the TOX 50 to the IC50 for the specific compound against the 3D7 *P. falciparum* isolate. The primary hepatocytes have demonstrable drug metabolising activity. The tetraoxane derivatives are remarkably non-toxic in these screens with *in vitro* TIs of between 5000 to 17000!**.**

### Genotoxicity Studies

The potential genotoxicity of selected lead compounds (RKA 216 **(29h)**, GE75 **(39b)** and GE114 **(39d)**) has been determined by the *Salmonella typhimurium* SOS/*umu* assay in two strains (Table 12 and Table 13): TA1535/pSK1002 and NM2009. This assay is based on the ability of DNA damaging agents to induce the expression of the *umu* operon. The *Salmonella* strains have a plasmid pSK1002 which carries an *umu*C-lacZ fused gene that produces a hybrid protein with β-galactosidase activity and whose expression is controlled by the *umu* regulatory region. Since many compounds do not exert their mutagenicity effect until they have been metabolized, the assay was also performed in the presence of rat liver S9-mix. Positive control agents (4-Nitroquinoline-1-oxide (4NNQO) and 2-Aminoanthracene (2Aan)) were used to test the response of the tester strains. Negative results were obtained for GE75 **(39b)**, GE114 **(39d)** and RKA216 **(29h)** at the highest concentration tested (50µM), both in the absence and in the presence of an *in vitro* metabolic activation system (S-9 mix).

**Table 12. Mutagenic potential on Salmonella typhimurium TA 1535/pSK1002 strain in the absence and presence of S9-mix. (D535 = 4'Chloro N-tertbutyl amodiaquine as an additional comparitor)**

| **Cmpd** | **Max.solubility in the assay (µM)** | **MCE¹ TA1535 (µM)** | **Range of Co tested (µM)** | **-S9** | | **+ Rat S9** | |
|---|---|---|---|---|---|---|---|
| | | | | **Max. Fold Increase** | **Potential genotox** | **Max. Fold Increase** | **Potential genotox** |
| **D535** | 140 | 140 | 140-0.27 | 1.10±0.07 | NEG | 0.93±0.02 | NEG |
| **GE75** | 50 | 50 | 50-0.10 | 1.03±0.07 | NEG | 0.98±0.06 | NEG |
| **GE114** | 50 | 50 | 50-0.10 | 1.05±0.09 | NEG | 0.99±0.09 | NEG |
| **RKA216** | 50 | 50 | 50-0.10 | 1.04±0.04 | NEG | 0.98±0.02 | NEG |
| | **(µg/mL)** | **(µg/mL)** | **(µg/mL)** | | | | |
| **4NNQO** | 2 | 0.25 | 2-0.004 | 7.22±0.54 | Positive² | 8.68±1.32 | Positive |
| **2Aan** | 5 | 5 | 5-0.01 | 1.10±0.11 | NEG | 7.34±0.32 | Positive |

| | | | | | | | |
|---|---|---|---|---|---|---|---|
| 1. MCE: Maximum Co w/o effects on bacteria growth or β-galactosidase production. 2. Positive response: > 2-fold dose-related increase in β-galactosidase activity over the mean control values. | | | | | | | |

**Table 13. Mutagenic potential on Salmonella typhimurium NM2009 strain in the absence and presence of S9-mix.**

| **Cmpd** | **Max.solubility in the assay (µM)** | **MCE¹ NM2009 (µM)** | **Range of Co tested (µM)** | **-S9** | | **+ Rat S9** | |
|---|---|---|---|---|---|---|---|
| | | | | **Max. Fold Increase** | **Potential genotox** | **Max. Fold Increase** | **Potential genotox** |
| D535 | 140 | 140 | 140-0.27 | 1.04±0.10 | NEG | 1.05±0.02 | NEG |
| GE75 | 50 | 50 | 50-0.10 | 1.07±0.03 | NEG | 0.95±0.03 | NEG |
| GE114 | 50 | 50 | 50-0.10 | 1.09±0.03 | NEG | 1.07±0.10 | NEG |
| RKA216 | 50 | 50 | 50-0.10 | 1.22±0.03 | NEG | 1.02±0.06 | NEG |
| | **(µg/mL)** | **(µg/mL)** | **(µg/mL)** | | | | |
| **4NNQO** | 2 | 1 | 2-0.004 | 4.80±0.45 | Positive² | 8.40±0.50 | Positive |
| **2Aan** | 5 | 0.31* | 5-0.01 | 1.08±0.01 | NEG | 6.09±0.29 | Positive |

| | | | | | | | |
|---|---|---|---|---|---|---|---|
| 1. MCE: Maximum Co w/o effects on bacteria growth or beta-galactosidase production. * In the presence of rat S9, the maximum concentration without effects on bacteria growth or β-galatosidase production (MCE) is 0.31µg/mL for 2Aan. 2. Positive response: > 2-fold dose-related increase in β-galactosidase activity over the mean control values. | | | | | | | |

### Stability Studies

### Chemical Stability

The chemical stability of **29h** and **39b** was investigated to confirm stability in aqueous solution and in the presence of acid. As can be seen from the table greater than 95% recovery of the starting tetraoxane was observed in all cases (Table 14). These reactions are based on recovery of material following chromatography on a 50 mg scale reaction. Apart from entry 2, where a minor product appeared on TLC, no other products of decomposition could be detected. In a control to assess column recovery 50 mg yielded **48** mg indicating, that as a percentage control, the endoperoxides examined are completely stable under the conditions tested.

**Table 14: Chemical stability studies on 39b and 29h.**

| Tetraoxane Drug | Conditions | % Recovery of Tetraoxane |
|---|---|---|
| **39b** | DCM, 7 days, RT | 96.2 (99) |
| **39b** | DCM, 1% formic acid, 7days, RT | 92.3 (95) |
| **39b** | Water, 7 days, 37 °C | 97.8 (100) |
| **39b** | Phosphate buffer, pH 7.4, 16 hrs, 37°C | 93.2 (95) |
| **29h** | Phosphate buffer, pH 7.4, 16 hrs, 37°C | 95.6 (99) |
| **29h** | Water, 7 days, 37 °C | 98.2 (100) |
| **29h** | DCM, 7 days, RT | 97.2 (99) |

| | | |
|---|---|---|
| ^{a} Figures in brackets refer to recovery as a % of control. (DCM = dichloromethane) | | |

### Stability in Presence of Fe(II) ions

Tetraoxane **39f,** ozonide **42** (also referred to below as OZ) and trioxane **43** (for structures see below, compounds selected due to UV chromophore to aid TLC analysis) were subjected to 1.0 equivalents of FeBr₂ in THF for the set time periods layed out in the table (This combination leads to complete degradation of artemisinin after 24h). The resulting residue was purified by flash column chromatography and the % recovery of starting endoperoxide calculated (Table 15).

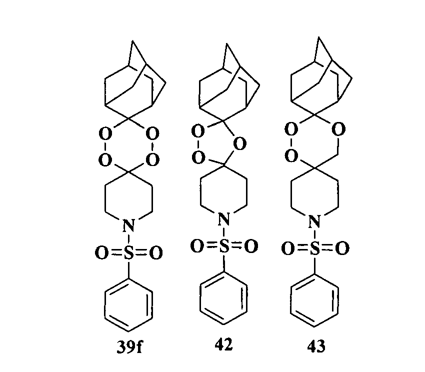

**Table 15: Timed iron degradation stability studies.**

| DRUG | % Recovery Of Endoperoxide | | | |
|---|---|---|---|---|
| | 4 hrs | 8 hrs | 24 hrs | 48 hrs |
| TETRAOXANE **39f** | 88.7 | 80.5 | 72.0 | 69.0 |
| OZONIDE **42** | 11.0 | 9.0 | 2.6 | 0.0 |
| TRIOXANE **43** | 96.8 | 84.9 | 56.7 | 43.2 |

**Table 16**

| IRON SOURCE | % Recovery of Tetraoxane 39f | | | |
|---|---|---|---|---|
| | 2 hrs | 4 hrs | 8 hrs | 24 hrs |
| FeSO₄.7H₂O | 99 | 99 | 98 | 98 |
| FeCl₂.4H₂O | - | - | - | 97 |

Tables 15 and 16 demonstrate the remarkable stability of the 1,2,4,5 tetraoxane ring system. The ferrous bromide/THF system has been widely used in the literature for iron degradation reactions and in studies with artemisinin complete degradation can be achieved in less than 24h. With the OZ heterocycle we observe almost 90% degradation after 4h; the corresponding tetraoxane **39f** is only degraded by 10%.

Complete loss of OZ material **42** (100 % turnover) is observed after 48 h whereas 69% (31% turnover) can be recovered for **39f.** The tetraoxane is also more stable than the corresponding 1,2,4-trioxane **43** which was degraded by 57% after 48h.

Further studies with iron salts known to readily degrade artemisinin and synthetic endoperoxides such as arteflene proved to be ineffective at degrading **39f**.

The results confirm that the 1, 2, 4, 5- tetraoxane heterocyle is remarkably stable to decomposition with ferrous iron salts. This contrasts with the synthetic OZ derivatives where instability has contributed to major difficulties in their development.

### Experimental Preparation of Inventive Compounds

### General procedure for the preparation of bishydroperoxides

### Preparation of Cyclohexane-1,1-diyl bis-hydro peroxide 6a

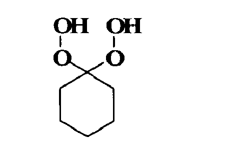

A stirred solution of cyclohexanone 6 (5.889g, 60mmol) in formic acid (40ml) was added 30% aqueous hydrogen peroxide (20ml) and the mixture was stirred at room temperature for 4 minutes. The mixture was then poured into ice-cold water and the organic products were extracted by diethyl ether (300ml). After conventional workup, the residue was separated by column chromatography on silica gel to give the bishydroperoxide in 76%.

### Preparation of Cyclododecane-1,1-diyl bis hydro peroxide 7a

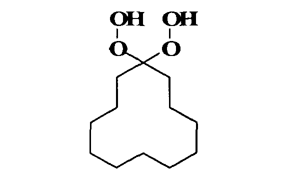

This product was prepared in 72% according to the general procedure for preparing bishydroperoxides.

### Preparation of Adamantane-2,2-diyl bishydroperoxide 8a

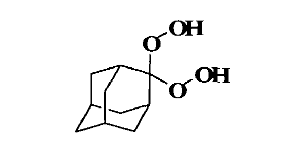

This product was prepared in 76% according to the general procedure for preparing bishydroperoxides.

### General procedure for the preparation of the tetraoxane ketones

### Preparation of 7,8,15,16-Tetraoxa-dispiro[5.2.5.2]hexadecan-3-one 10

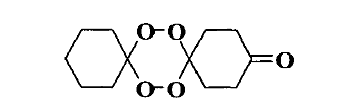

A solution of (0.12g, 2mmol) of cyclohexanone 6, (0.05g, 4mmol) of 30% H₂O₂ and (0.0005g, 0.002mmol) of methyltrioxorhenium (MTO) in 4ml of 2,2,2-trifluoroethanol (TFE) was stirred for 2 hours at room temperature. Into the solution, (0.4485g, 4mmol) of 1,4-cyclohexanedione 9 was added, followed by the addition of (0.095g, 2mmol) of 54% ethereal solution of tetrafluoroboric acid. The reaction mixture was left under stirring for an additional hour. Dichloromethane was added and the organic phases washed wish diluted NaHSO₄, dried over MgSO₄ and solvent evaporated under reduced pressure. Products were determined by NMR spectroscopy, isolated by column chromatography (SiO₂ CH₂Cl₂: Hexane = 9:1) to give the tetraoxane in 38%.

### Preparation of 7,8,21,22-Tetraoxa-dispiro[5.2.11.2]docosan-3-one 11

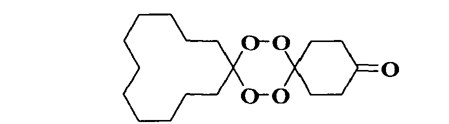

This product was prepared in 38% according to the general procedure for preparing tetraoxane ketones

### Preparation of adamantane tetraoxane ketone 12

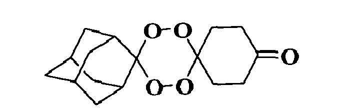

This product was prepared in 40% according to the general procedure for preparing tetraoxane ketones.

### General procedure for reductive amination of tetraoxane ketones

### Preparation of 4-(7,8,15,16-Tetraoxa-dispiro[5.2.5.2]hexadec-3-yl)-morpholine 19

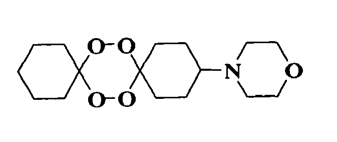

The 7,8,15,16-Tetraoxa-dispiro[5.2.5.2]hexadecan-3-one 10 (0.1g, 0.4mmol) and morpholine (0.26g, 0.26ml, 3.03mmol) were mixed in dichloromethane(15ml) before addition of sodiumtriacetoxyborohydride(0.64g, 3.03mmol). The reaction was stirred at room temperature for 18hrs and then washed with distilled water. The organic layer was dried and evaporated under vacuum to dryness. Purification by chromatography afforded the product in 56%.

### Preparation of Cyclopropyl-(7,8,15,16-tetraoxa-dispiro[5.2.5.2]hexadec-3-yl)-amine 14

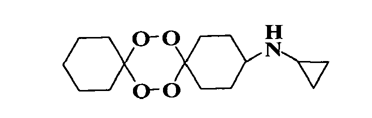

This product was prepared in 55% according to the general procedure for reductive amination of tetraoxane ketones.

### General procedure for the Wittig reaction

### Preparation of (1,4-Dioxa-spiro[4.5]dec-8-ylidene)-acetic acid ethyl ester 22

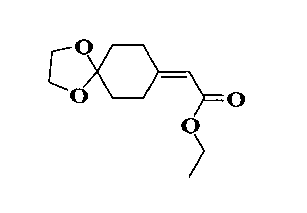

A solution of 1,4-cyclohexanedionemonoethylketal 20 (6g, 40mmol) and ethyl-(triphenylphosphoranylidene)acetate 22 (15g, 44mmol) in dry benzene (80ml) were refluxed under argon for 24hours. The solvent was removed under vacuum and product purified by flash chromatography to give the product in 90%.

### Preparation of (1,4-Dioxa-spiro[4.5]dec-8-ylidene)-acetic acid methyl ester 21

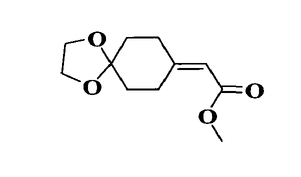

This product was prepared in 93% according to the general procedure for Wittig reactions.

### General procedure for hydrogenation reaction

### Preparation of (1,4-Dioxa-spiro[4.5]dec-8-yl)-acetic acid ethyl ester 24

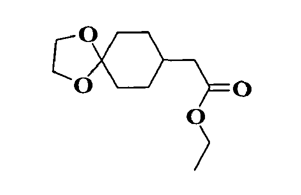

A suspension of the compound (3.14g, 13.7mmol) in ethyl acetate (80ml) and Pd-C (10%w/w, 1.97g) was stirred in a hydrogen atmosphere for 3hours. The solvent was removed under vacuum and product purified by flash chromatography to give the product in 90%.

### Preparation of (1,4-Dioxa-spiro[4.5]dec-8-yl)-acetic acid methyl ester 23

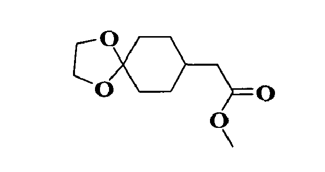

This product was prepared in 95% according to the general procedure for hydrogenation reaction.

### General procedure for the preparation of bishydroperoxide via tungstic acid catalyzed approach

### Preparation of (4,4-Bis-hydroperoxy-cyclohexyl)-acetic acid ethyl ester 26

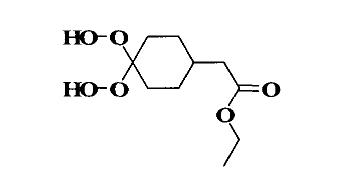

To a solution of the ketal **24** (1g, 4.4mmol) in dry THF (20ml) was treated with H₂O₂ (30% aq, 20ml) and tungstic acid (2.2g, 8.8mmol) and stirred for 48hrs at 0°C. The reaction mixture was extracted with dichloromethane, washed with brine and dried with MgSO₄. Purification by column chromatography gave the product in 73%.

### Preparation of (4,4-Bis-hydroperoxy-cyclohexyl)-acetic acid methyl ester 25

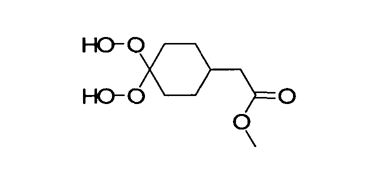

This product was prepared in 76% according to the general procedure for preparing bishydroperoxides via tungstic acid catalyzed approach.

### General procedure for the preparation of the 1,2,4,5-tetraoxane esters

### Preparation of (7,8,15,16-Tetraoxa-dispiro[5.2.5.2]hexadec-3-yl)acetic acid ethyl ester 27a

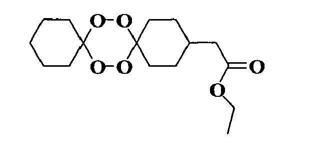

A stirred solution of cyclohexanone 6 (1.7g, 7.26mmol) in ethyl acetate was added 54% ethereal solution of HBF₄ (1.25g, 14.2mmol) to ethyl 2-(4,4-dihydroperoxycyclohexyl)acetate 26 and stirred for 3hrs at room temperature. Purification by column chromatography gave the product in 50%.

### Preparation of (7,8,21,22-Tetraoxa-dispiro[5.2.11.2]docos-3-yl)-acetic acid ethyl ester 28a

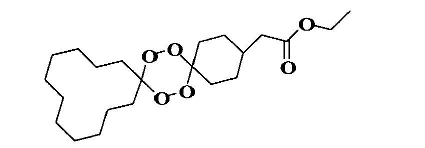

This product was prepared in 33% according to the general procedure for preparing 1,2,4,5-tetraoxane esters.

### Preparation of adamantyl tetraoxane ethylester 29a

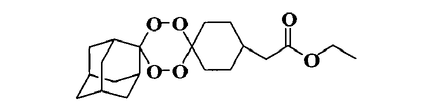

This product was prepared in 50% according to the general procedure for preparing 1,2,4,5-tetraoxane esters.

### Preparation of adamantyl tetraoxane methyl ester 30a

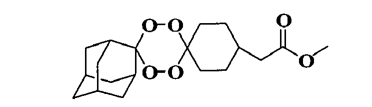

This product was prepared in 66% according to the general procedure for preparing 1,2,4,5-tetraoxane esters.

### General procedure for the preparation of the carboxylic acids

### Preparation of 7,8,15,16-Tetraoxa-dispiro[5.2.5.2]hexadec-3-yl)acetic acid 27b

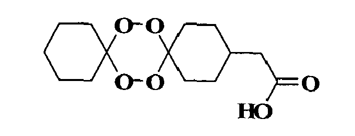

The ethyl ester 27a (1.82g, 5.8mmol) was hydrolyzed in 60ml methanol at 70°C with KOH (1.8g, 31.65mmol) and 6ml water. After one hour heating, the reaction mixture was cooled and diluted with 90ml dichloromethane and 30ml water. The aqueous layer was acidified with concentrated HCl (6ml). The aqueous layer was further extracted with DCM. The combined organic layers were washed with water, brine, dried over Na₂SO₄ and evaporated to dryness. Purification by column chromatography gave the pure acid 27b in 75%.

### Preparation of (7,8,21,22-Tetraoxa-dispiro[5.2.11.2]docos-3-yl)-acetic acid 28b

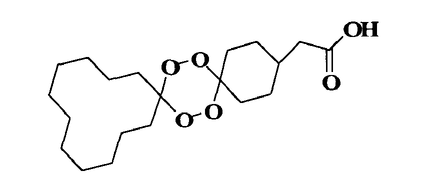

This product was prepared in 66% according to the general procedure for preparing or carbocylic acids.

### Preparation of adamantyl tetraoxane carboxylic acid 29b

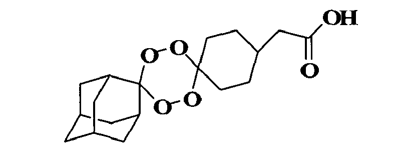

This product was prepared in 66% according to the general procedure for preparing carboxylic acids.

### General procedure for the preparation for the amide coupling reactions

### Preparation of 1-Morpholin-4-yl-2-(7,8,15,16-tetraoxa-dispiro[5.2.5.2]hexadec-3-yl)-Ethanone 27h

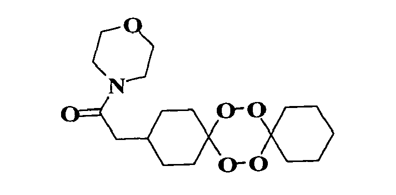

A solution of 7,8,15,16-Tetraoxa-dispiro[5.2.5.2]hexadec-3-yl)acetic acid 27b (0.1g, 0.35mmol) in dry dichloromethane (18ml), with added triethylamine (0.04g, 0.005ml, 0.35mmol) and ethylchloroformate (0,005g, 0.04ml, 0.46mmol) was stirred for 60minutes at 0°C. (0.06g, 0.06ml, 0.70mmol) of morpholine was added, and after 30minutes of stirring the reaction mixture was warmed to room temperature. After 90minutes, it was diluted with water and extracted with dichloromethane. The organic extract was washed with brine, dried over anhydrous Na₂SO₄. The crude product was purified by flash chromatography to give the pure amide in 84%.

### Preparation of N-Cyclopropyl-2-(7,8,15,16-tetraoxa-dispiro[5.2.5.2]hexadec-3-yl)-acetamide 27c

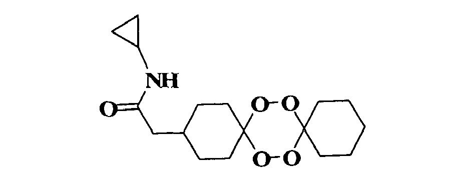

This product was prepared in 84% according to the general procedure for the amide coupling reactions.

### Preparation of N-(2-Pyrrolidin-1-yl-ethyl)-2-(7,8,15,16-tetraoxa-dispiro[5.2.5.2]-hexadec-3-yl)-acetamide 27d

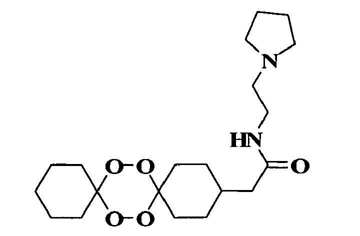

This product was prepared in 78% according to the general procedure for the amide coupling reactions.

### Preparation of N-(2-Piperidin-1-yl-ethyl)-2-(7,8,15,16-tetraoxa-dispiro[5.2.5.2]-hexadec-3-yl)-acetamide 27e

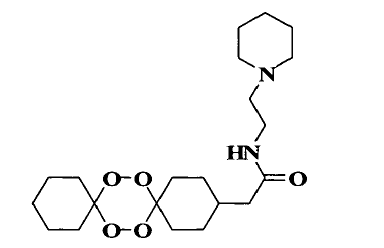

This product was prepared in 81 % according to the general procedure for the amide coupling reactions.

### Preparation of N-(2-Morpholin-4-yl-ethyl)-2-(7,8,15,16-tetraoxa-dispiro[5.2.5.2]-hexadec-3-yl)-acetamide 27f

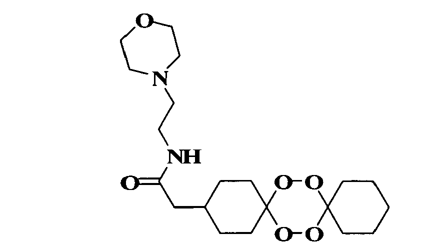

This product was prepared in 76% according to the general procedure for the amide coupling reactions.

### Preparation of N-(2-Diethylamino-ethyl)-2-(7,8,15,16-tetraoxa-dispiro[5.2.5.2]-hexadec-3-yl)-acetamide 27g

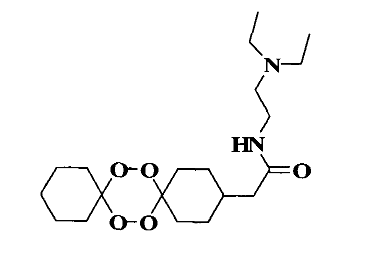

This product was prepared in 58% according to the general procedure for the amide coupling reactions.

### Preparation of (2-7,8,15,16-Tetraoxa-dispiro[5.2.5.2]hexadec-3-yl-acetylamino)-acetic acid methyl ester 27i

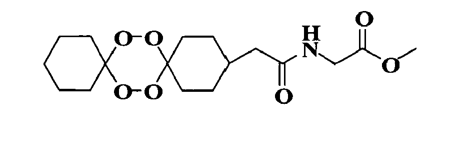

This product was prepared in 45% according to the general procedure for the amide coupling reactions.

### Preparation of N-Cyclopropyl-2-(7,8,21,22-tetraoxa-dispiro[5.2.11.2]docos-3-yl)-acet-amide 28c

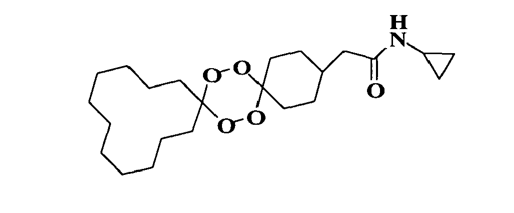

This product was prepared in 88% according to the general procedure for the amide coupling reactions.

### Preparation of N-(2-Pyrrolidin-1-yl-ethyl)-2-(7,8,21,22-tetraoxa-dispiro[5.2.11.2]docos-3-yl)-acetamide 29d

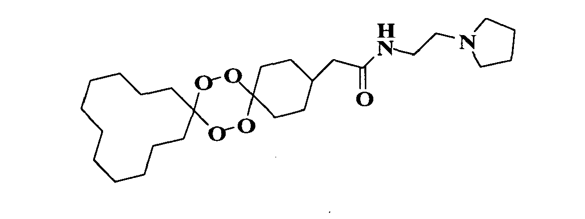

This product was prepared in 81 % according to the general procedure for the amide coupling reactions.

### Preparation of N-(2-Piperidin-1-yl-ethyl)-2-(7,8,21,22-tetraoxadispiro[5.2.11.2]-docos-3-yl)-acetamide 28e

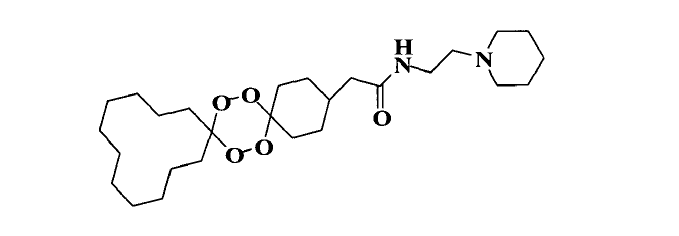

This product was prepared in 82% according to the general procedure for the amide coupling reactions.

### Preparation of N-(2-Morpholin-4-yl-ethyl)-2-(7,8,21,22-tetraoxa-dispiro[5.2.11.2]docos-3-yl)-acetamide 28f

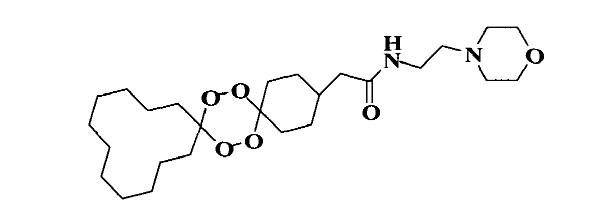

This product was prepared in 78% according to the general procedure for the amide coupling reactions.

### Preparation of N-(2-Diethylamino-ethyl)-2-(7,8,21,22-tetraoxa-dispiro[5.2.11.2]-docos-3-yl)-acetamide 28g

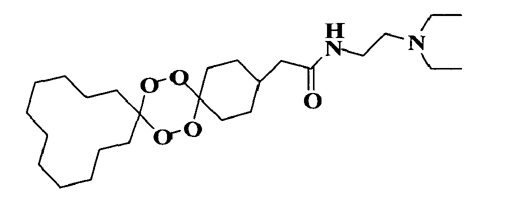

This product was prepared in 74% according to the general procedure for the amide coupling reactions.

### Preparation of 1-Morpholin-4-yl-2-(7,8,21,22-tetraoxa-dispiro[5.2.11.2]docos-3-yl)-ethanone 28h

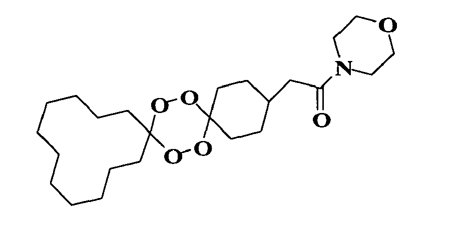

This product was prepared in 90% according to the general procedure for the amide coupling reactions.

### Preparation of 2-(7,8,21,22-Tetraoxa-dispiro[5.2.11.2]docos-3-yl)-1-thiomorpholin-4-yl-ethanone 28i

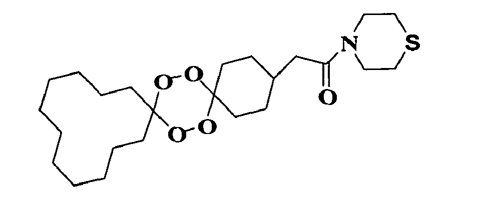

This product was prepared in 78% according to the general procedure for the amide coupling reactions.

### Preparation of adamantyl- N-Cyclopropyl tetraoxane acetamide 29c

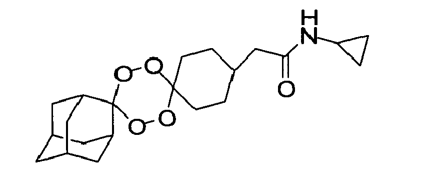

This product was prepared in 83% according to the general procedure for the amide coupling reactions.

### Preparation of N-(2-Pyrrolidin-1-yl-ethyl)-[adamantyl] acetamide 29d

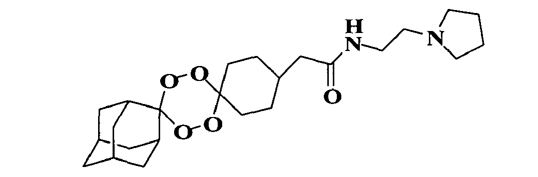

This product was prepared in 80% according to the general procedure for the amide coupling reactions.

### Preparation of N-(2-Piperidin-1-yl-ethyl)-[adamantyl ]acetamide 29e

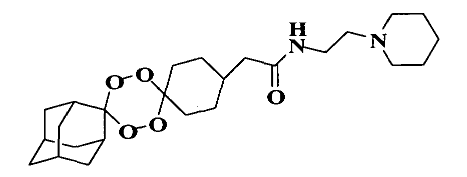

This product was prepared in 78% according to the general procedure for the amide coupling reactions.

### Preparation of N-(2-Morpholin-4-yl-ethyl)-adamantyl acetamide 29f

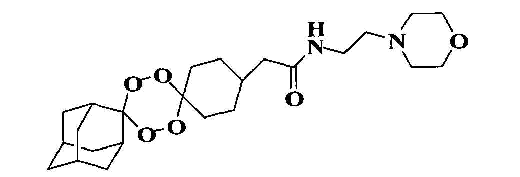

This product was prepared in 77% according to the general procedure for the amide coupling reactions.

### Preparation of N-(2-Diethylamino-ethyl)-[adamantly]acetamide 29g

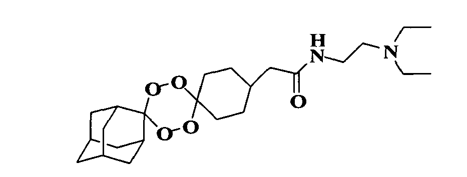

This product was prepared in 66% according to the general procedure for the amide coupling reactions.

### Preparation of adamantly-1-Morpholin-4-yl tetraoxane acetamide 29h

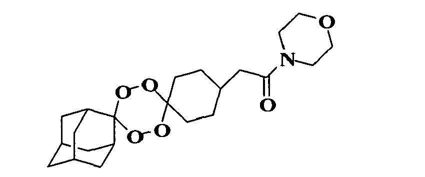

This product was prepared in 81 % according to the general procedure for the amide coupling reactions.

### Preparation of Tetraoxa-dispiro-(adamantly)-thiomorpholin-4-yl-ethanone 29i

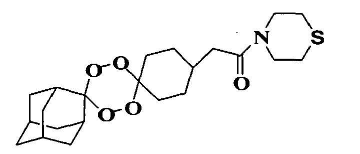

This product was prepared in 77% according to the general procedure for the amide coupling reactions.

### Preparation of adamantyl acetamide 29j

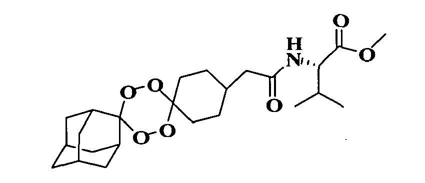

This product was prepared in 69% according to the general procedure for the amide coupling reactions.

### Preparation of tetraoxane 29k

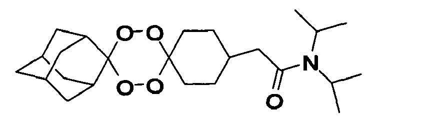

This product was isolated in 83% according to the general procedure for amide coupling reactions. This product was purified by flash column chromatography using Hexane/ethyl acetate (1:1, v/v, Rf = 0.6) as eluent.

### Preparation of tetraoxane 29l

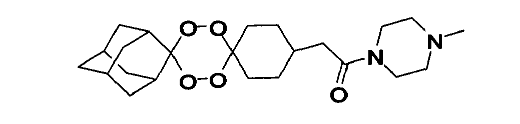

This product was isolated in 87% according to the general procedure for amide coupling reactions. This product was purified by flash column chromatography using DCM/methanol (9:1, v/v, Rf = 0.6) as eluent.

### Preparation of tetraoxane 29m

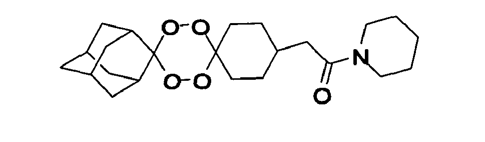

This product was isolated in 89% according to the general procedure for amide coupling reactions. This product was purified by flash column chromatography using DCM/ethyl acetate (1:1, v/v, Rf = 0.7) as eluent.

### Preparation of tetraoxane 29n

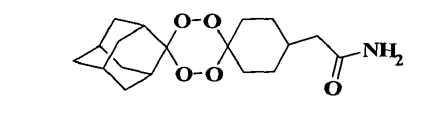

This product was isolated in 43% according to the general procedure for amide coupling reactions. This product was purified by flash column chromatography using DCM/ethyl acetate (1:1, v/v, Rf = 0.2) as eluent.

Preparation of tetraoxane **29o**

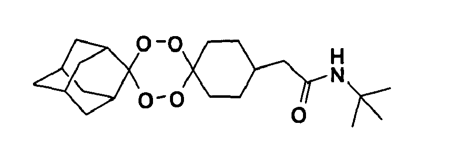

This product was isolated in 83% according to the general procedure for amide coupling reactions. This product was purified by flash column chromatography using DCM/ethyl acetate (1:1, v/v, Rf = 0.7) as eluent.

### Preparation of tetraoxane 29p

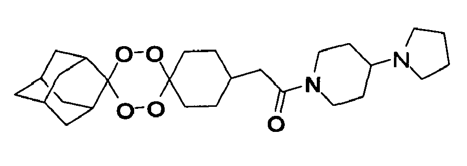

This product was isolated in 76% according to the general procedure for amide coupling reactions. This product was purified by flash column chromatography using DCM/ethyl acetate (1:1, v/v, Rf= 0.3) as eluent.

### Preparation of tetraoxane 29q

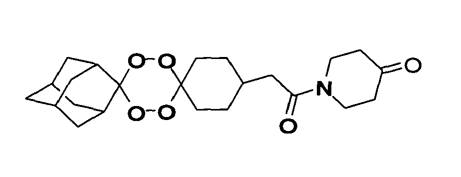

This product was isolated in 80% according to the general procedure for amide coupling reactions. This product was purified by flash column chromatography using DCM/ethyl acetate (1:1, v/v, Rf= 0.5) as eluent.

### Preparation of tetraoxane 29r

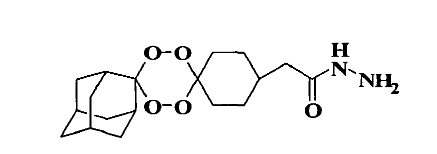

This product was isolated in 72% according to the general procedure for amide coupling reactions. This product was purified by flash column chromatography using DCM/ethyl acetate (1:1, v/v, Rf = 0.2) as eluent.

### Preparation of tetraoxane 29s

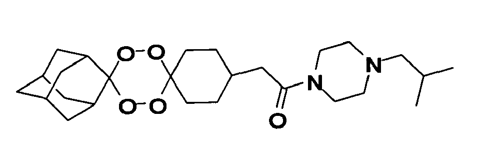

This product was isolated in 87% according to the general procedure for amide coupling reactions. This product was purified by flash column chromatography using DCM/ethyl acetate (1:1, v/v, Rf = 0.4) as eluent.

### Preparation of tetraoxane 29t

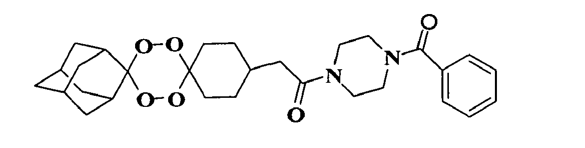

This product was isolated in 68% according to the general procedure for amide coupling reactions. This product was purified by flash column chromatography using DCM/ethyl acetate (1:1, v/v, Rf = 0.3) as eluent.

### Preparation of tetraoxane 29u

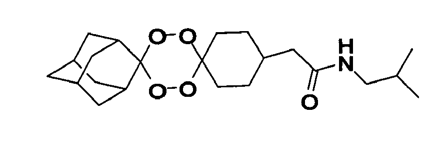

This product was isolated in 73% according to the general procedure for amide coupling reactions. This product was purified by flash column chromatography using DCM/ethyl acetate (1:1, v/v, Rf = 0.7) as eluent.

### Preparation of tetraoxane 29v

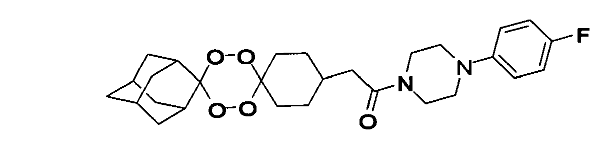

This product was isolated in 77% according to the general procedure for amide coupling reactions. This product was purified by flash column chromatography using DCM/ethyl acetate (1:1, v/v, Rf = 0.6) as eluent.

### Preparation of tetraoxane 29w

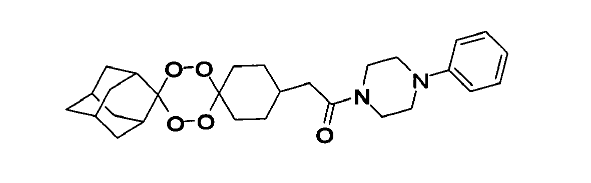

This product was isolated in 64% according to the general procedure for amide coupling reactions. This product was purified by flash column chromatography using DCM/ethyl acetate (1:1, v/v, Rf= 0.7) as eluent.

### Preparation of tetraoxane 29x

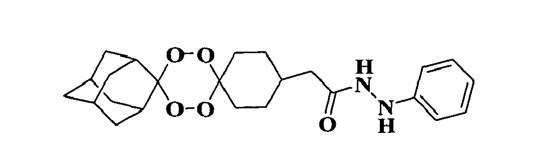

This product was isolated in 70% according to the general procedure for amide coupling reactions. This product was purified by flash column chromatography using DCM/ethyl acetate (1:1, v/v, Rf = 0.6) as eluent.

### General procedure for the preparation of tetraoxane sulfones

### Preparation of 1-(1,1-Dioxo-1λ⁶-thiomorpholin-4-yl)-2-(7,8,21,22-tetraoxa-dispiro[5.2.11.2]docos-3-yl)-ethanone 31

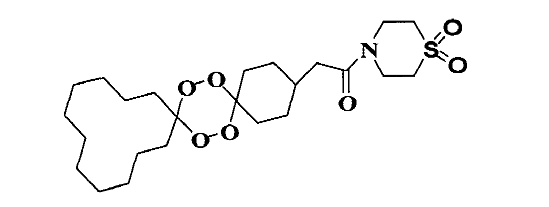

A solution of 28i (0.1g, 0.22mmol) and *m*CPBA (0.11g, 0.66mmol) in 10ml CH₂Cl₂ was stirred at room temperature for 4-6hours. After consumption of the more polar intermediate sulfoxide (monitored by tlc) the mixture was poured into a saturated solution of cold 5% K₂CO₃ solution. The mixture was then extracted with CH₂Cl₂, the organic layer separated, dried over MgSO₄ and evaporated. Purification was achieved by column chromatography to give the desired sulfone in 92%.

### Preparation of 1-(1,1-Dioxo-1λ⁶-thiomorpholin-4-yl)-2-tetraoxa-dispiro-adamantyl ethanone 32

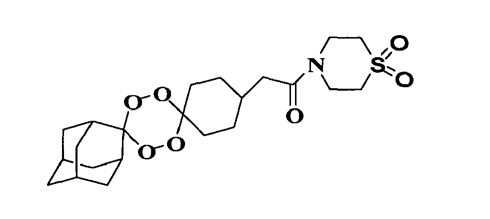

This product was prepared in 88% according to the general procedure for preparation of tetraoxane sulfones.

### Preparation of diethyl methylsulfonylmethylphosphonate 33b

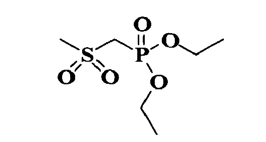

A solution of diethylmethy thiomethyl phosphorane (1g, 3.8 mmol) and *m*CPBA (1.4g, 7.98 mmol) in DCM (30 mL) was stirred at room temperature for 4-6 hours. The mixture was poored into a saturated solution of cold K₂CO₃ and then extracted with DCM. The organic layer was separated, dried over MgSO₄ and concentrated to give the product.

### Preparation of 8-(methylsulfonylmethylene)-1,4-dioxaspiro[4.5]decane 33c

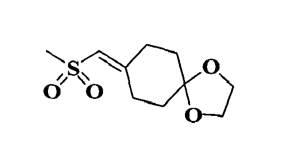

To a stirred solution of diethylmethyl sulfonomethylphosphonate (2.4g, 10 mmol) in THF (50 mL) under nitrogen and at -78_{°}C was added (6.4g, 9.2mL, 10mmol) of 1.32M ⁿBuLi in pentane. The resulting solution was stirred at -78°C for 15minutes to 3 hours at which time 1,4-cyclohexane dione monoethylene ketal (1.5g, 10mmol) was added in THF (10mL). The clear solution was stirred at -78°C for 1 hour, then allowed to warm to room temperature and stirring was continued at that temperature overnight. The resulting solution was poured into 50mL saturated solution of NH₄Cl and extracted with ether, washed with water, NaHCO₃ and brine. The combined extracts were dried over MgSO₄ and concentrated to give the product in 67%.

### Preparation of 8-(methylsulfonylmethyl)-1,4-dioxaspiro[4.5]decane 33d

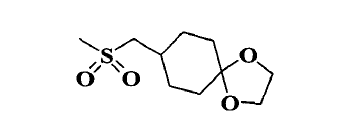

A suspension of 8-(methylsulfonylmethylene)-1,4-dioxaspiro[4.5]decane (1.6g, 6.7mmol) and 10% Pd/C (1g) in ethyl acetate (40mL) was stirred under hydrogen atmosphere for 1 hour. The reaction mixture was filtered off through celite and the filtrated concentrated to give the product in 88%.

### Preparation of tetraoxane 33f

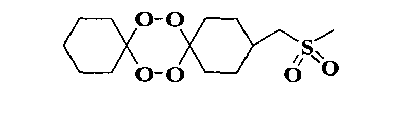

To a solution of 8-(methylsulfonylmethyl)-1,4-dioxaspiro[4.5]decane (1.6g, 6.6mmol) in THF (20 mL), 30% H₂O₂ (20 mL) and tungstic acid (3.4g, 13.7mmol) were successively added at 0°C. After 48 hours of stirring with exclusion of light, at 0°C, the mixture was extracted with DCM and the combined organic layers were washed with a saturated solution of NaCl, dried and evaporated in vacuo. The resulting gem-dihydroperoxide was dissolved in ethyl acetate (30mL) and cyclohexanone (0.7g, 6.6mmol) followed by 54% ethereal solution of tetrafluoroboric acid (1.15g, 13.08mmol) were added and the reaction mixture stirred for an hour. The mixture was washed with NaHCO₃, dried in MgSO₄ and the solvent evaporated under reduced pressure. Purification of the crude product by flash column chromatography using Hexane/ethylacetate (1:1, v/v, Rf = 0.6) as eluent gave the required product as white powder in 15%.

### Preparation of tetraoxane 33g

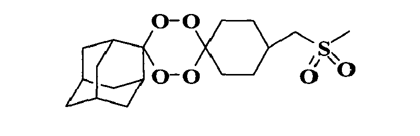

This product was isolated in 8% according to the general procedure above. This product was purified by flash column chromatography using DCM/ethyl acetate (1:1, v/v, Rf = 0.7) as eluent.

### General procedure for making Piperidinones

### Preparation of 1-benzoylpiperidin-4-one 34b

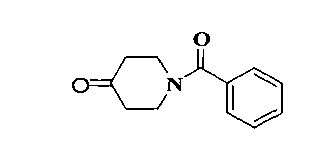

To a solution of benzoyl chloride (5g, 4.1mL, 35.6 mmol) and triethylamine (7.2g, 9.9mL, 71.2 mmol) in 50 mL toluene was added 4-piperidinone monohydrate hydrochloride (5g, 29.1 mmol) and heated to reflux for 2-3 hours. The solid was filtered off and the liquid concentrated. Purification by column chromatography using DCM/ethyl acetate (1:1, v/v, Rf = 0.4) gave the pure product as a liquid in 66%.

### Preparation of 1-(pyrrolidin-1-carbonyl)piperidin-4-one 34c

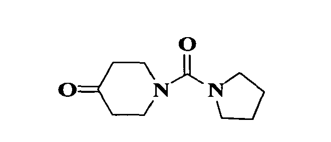

This product was isolated in 63% according to the general procedure for making the piperidinones. This product was purified by flash column chromatography using DCM/ethyl acetate (1:1, v/v, Rf = 0.4) as eluent.

### Preparation of N,N-diethyl-4-oxopiperidine-1-carboxamide 34d

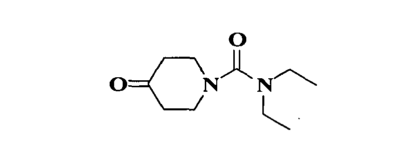

This product was isolated in 56% according to the general procedure for making the piperidinones. This product was purified by flash column chromatography using DCM/ethyl acetate (1:1, v/v, Rf = 0.3) as eluent.

### Preparation of 1-(piperidine-1-carbonyl)piperidin-4-one 34e

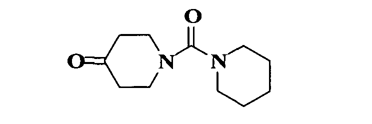

This product was isolated in 73% according to the general procedure for making the piperidinones. This product was purified by flash column chromatography using DCM/ethyl acetate (1:1, v/v, Rf = 0.3) as eluent.

### Preparation of 1-(morpholine-1-carbonyl)piperidin-4-one 34f

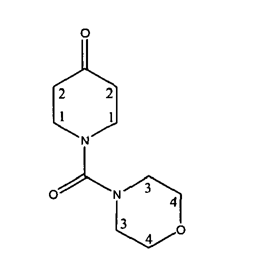

This product was isolated in 64% according to the general procedure for making the piperidinones. This product was purified by flash column chromatography using DCM/ethyl acetate (1:1, v/v, Rf = 0.3) as eluent.

### Preparation of 4-oxo-N,N-diphenylpiperidine-1-carboxamide 34g

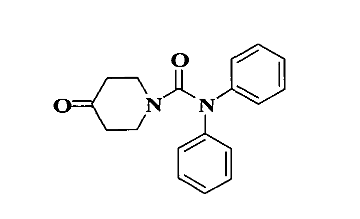

This product was isolated in 89% according to the general procedure for making the piperidinones. This product was purified by flash column chromatography using DCM/ethyl acetate (1:1, v/v, Rf = 0.7) as eluent.

### Preparation of 1-(4-trifluoromethyl)benzoyl)piperidin-4-one 34h

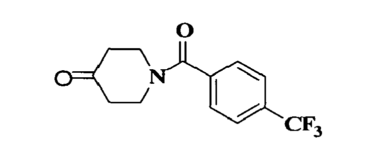

This product was isolated in 74% according to the general procedure for making the piperidinones. This product was purified by flash column chromatography using Hexane/ethyl acetate (1:1, v/v, Rf = 0.3) as eluent.

### Preparation of (4,4-dihydroperoxypiperidin-1-yl)(piperidin-1-yl)methanone 34i

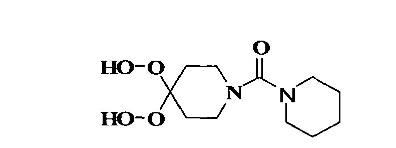

This product was isolated in quantitative yield according to the general procedure for making *gem*-dihydroperoxides. This product was purified by flash column chromatography using Hexane/ethyl acetate (1:1, v/v, Rf = 0.2) as eluent.

### Preparation of tetraoxane 34j

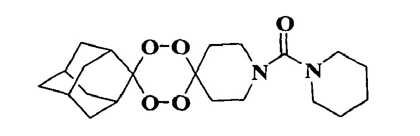

A solution of 1,1-diethoxyadamantanone (0.17g, 0.77mmol) in diethylether (5mL) was added to a stirred suspension of(4,4-dihydroperoxypiperidin-1-yl)(piperidin-1-yl)methanone (0.2g, 0.77mmol) and BF3.OEt3 (1.4 equiv.) in diethylether (5mL). The mixture was stirred until the conversion of the gem-dihydroperoxide and then K2CO3 was added. The resulting two-phase system was stirred for 30-60 minutes and the organic phase separated. The aq. Phase was extracted with diethylether and dried with MgSO4, concentrated and chromatographed with DCM/ethylacetate (1:1, v/v, Rf = 0.6) as eluent to give the product in 33%.

### Preparation of tetraoxane 34k

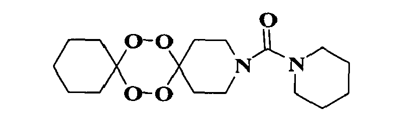

This product was isolated in 37% according to the general procedure above. This product was purified by flash column chromatography using Hexane/ethyl acetate (1:1, v/v, Rf = 0.6) as eluent.

### Preparation of tetraoxane 34o

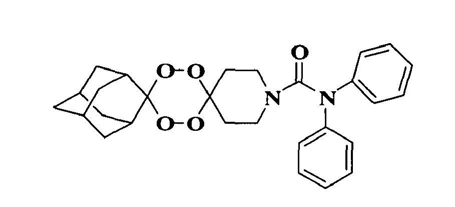

This product was isolated in 18% according to the general procedure above. This product was purified by flash column chromatography using DCM/ethyl acetate (1:1, v/v, Rf = 0.5) as eluent.

### Preparation of tetraoxane 34l

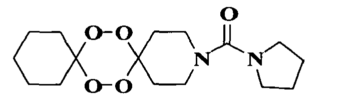

This product was isolated in 19% according to the general procedure above. This product was purified by flash column chromatography using Hexane/ethyl acetate (1:1, v/v, Rf = 0.2) as eluent.

### Preparation of tetraoxane 34m

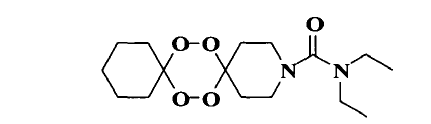

This product was isolated in 32% according to the general procedure above. This product was purified by flash column chromatography using DCM/ethyl acetate (1:1, v/v, Rf = 0.7) as eluent.

### Preparation of tetraoxane 34n

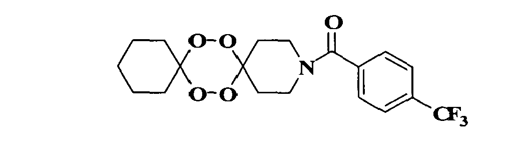

To a stirred solution of 1,2-dihydroperoxycyclohexane (0.97g, 6.65mmol) in ethyl acetate (30mL) was added 1-(4-trifluoromethyl)benzoyl)piperidin-4-one (1.8g, 6.54mmol). A 54% ethereal solution of tetrafluoroboric acid (1.15g, 13.08mmol) was added and the reaction mixture stirred for an hour. The mixture was washed with NaHCO₃, dried in MgSO₄ and the solvent evaporated under reduced pressure. Purification of the crude product by flash column chromatography using Hexane/ethylacetate (1:1, v/v, Rf = 0.6) as eluent gave the required product as white powder in 24%.

### Preparation of morpholine urea 1,2,4,5-tetraoxane 34p

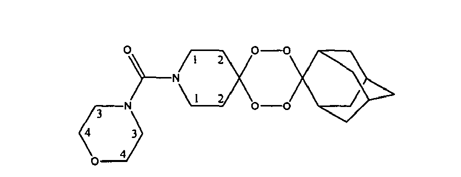

A solution of ketone (250 mg, 1.18 mmol), 30% H₂O₂ (0.27 ml, 2.36 mmol, 2.0 eq) and MTO (trace) in HFIP (2.36 ml) was stirred at room temperature for 2 hours. After this time 2-adamantanone (355 mg, 2.36 mol, 2.0 eq) was added followed by dropwise addition of a 54% ethereal solution of HBF₄ (0.33 ml, 2.36 mmol, 2.0 eq). The reaction was then stirred at room temperature for 1 hour. Dichloromethane (10 ml) was added and the organic layer washed with a sat. soln. of NaHCO₃ dried over MgSO₄ and the solvent removed *in vacuo.* The resulting residue was purified by flash column chromatography (SiO₂, hexane:EtOAc = 9:1) to give the title compound (30.8 mg, 6.6%).

### Preparation of tetraoxanes incorporating fused ring moieties

### Preparation of indanone tetraoxane 35d

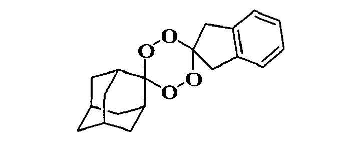

To a solution of 2-indanone (2g, 6.6 mmol) in 10 mL acetonitrile was added 5 mL formic acid and 5mL 30% H₂O₂ at 0°C. The mixture was stirred for 15 minutes and DCM added. The organic phase was washed with saturated NaHCO₃, dried and concentrated. The resulting gem-dihydroperoxide was dissolved in ethyl acetate (30mL) and 2-adamantanone (3g, 18mmol) followed by 54% ethereal solution of tetrafluoroboric acid (2.7g, 2.3mL, 30.3mmol) were added and the reaction mixture stirred for an hour. The mixture was washed with NaHCO₃, dried in MgSO₄ and the solvent evaporated under reduced pressure. Purification of the crude product by flash column chromatography using Hexane/ethylacetate (9:1, v/v, Rf = 0.5) as eluent gave the required product as white powder in 20%.

### Preparation of tetralone tetraoxane 36c

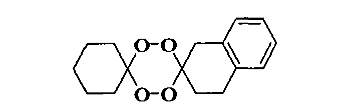

This product was isolated in 28% according to the general procedure above. This product was purified by flash column chromatography using Hexane/ethyl acetate (9:1, v/v, Rf = 0.6) as eluent.

### Preparation of tetralone tetraoxane 36d

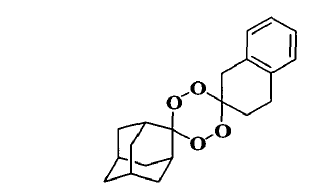

This product was isolated in 26% according to the general procedure above. This product was purified by flash column chromatography using Hexane/ethyl acetate (9:1, v/v, Rf = 0.5) as eluent.

### General procedure for preparation of sulfonyl piperidones

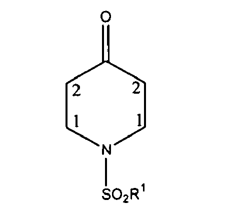

R¹-sulfonyl chloride (17.48 mmol, 1.5 eq) was added to a slurry of 4-piperidone monohydrate hydrochloride salt (2.00g, 11.65 mmol), K₂CO₃ (4.03g, 29.13 mmol, 2.5 eq), water (16 ml) and chloroform (16 ml). The bi-phasic reaction was stirred at room temperature overnight. The reaction was then quenched with saturated NaHCO₃ aq. The aqueous layer was separated and extracted with DCM (3 x 30ml). The combined organic extracts were dried over NaSO₄ and concentrated. The resulting residue was purified by flash column chromatography (SiO₂, EtOAc:hexane = 3:2) to give the desired sulfonyl piperidones.

### Preparation of 1-methanesulfonyl-piperidin-4-one 38a

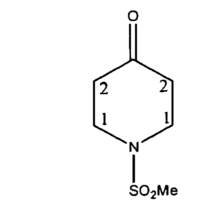

This product was prepared in 62% according to the general procedure for preparing sulfonyl piperidones.

### Preparation of 1-ethanesulfonyl-piperidin-4-one 38b

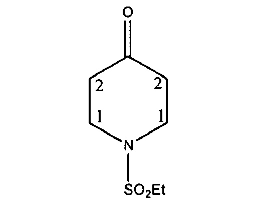

This product was prepared in 59% according to the general procedure for preparing sulfonyl piperidones.

### Preparation of 1-(propane-2-sulfonyl)-piperidin-4-one 38c

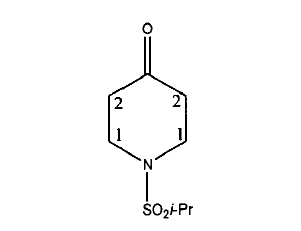

This product was prepared in 52% according to the general procedure for preparing sulfonyl piperidones.

### Preparation of 1-cyclopropylsulfonyl-piperidin-4-one 38d

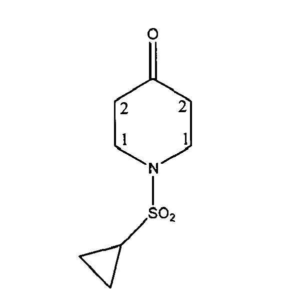

This product was prepared in 59% according to the general procedure for preparing sulfonyl piperidones.

### Preparation of 1-(2,2,2-trifluoroethanesulfonyl)-piperidin-4-one 38e

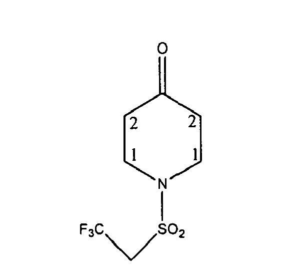

This product was prepared in 62% according to the general procedure for preparing sulfonyl piperidones.

### Preparation of 1-benzenesulfonyl-piperidin-4-one 38f

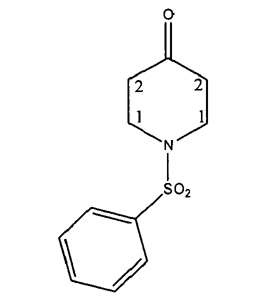

This product was prepared in 98% according to the general procedure for preparing sulfonyl piperidones.

### Preparation of 1-(4-chloro-benzenesulfonyl)-piperidin-4-one 38g

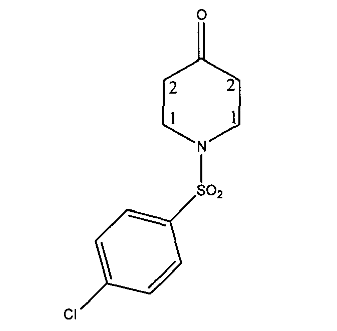

This product was prepared in 99% according to the general procedure for preparing sulfonyl piperidones.

### Preparation of 1-(4-fluoro-benzenesulfonyl)-piperidin-4-one 38h

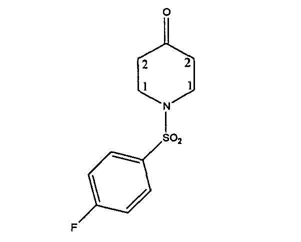

This product was prepared in 98% according to the general procedure for preparing sulfonyl piperidones.

### Preparation of 1-(4-trifluoromethyl-benzenesulfonyl)-piperidin-4-one 38i

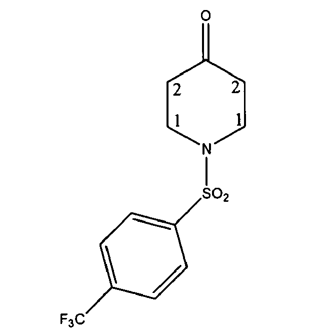

This product was prepared in 95% according to the general procedure for preparing sulfonyl piperidones.

### General procedure for preparation of adamantyl-1,2,4,5-tetraoxanes

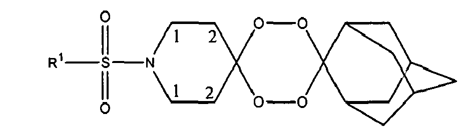

A solution of 1-R¹ sulfonyl-piperidin-4-one (1.13 mmol), 30% H₂O₂ (0.26 ml, 2.26 mmol, 2.0 eq) and MTO (trace) in HFIP (2.27 ml) was stirred at room temperature for 2 hours. After this time 2-adamantanone (339 mg, 2.26 mol, 2.0 eq) was added followed by dropwise addition of a 54% ethereal solution of HBF₄ (368 mg, 2.26 mmol, 2.0 eq). The reaction was then stirred at room temperature for 1 hour. Dichloromethane (10 ml) was added and the organic layer washed with a sat. soln. of NaHCO₃, dried over MgSO₄ and the solvent removed *in vacuo.* The resulting residue was purified by flash column chromatography (SiO₂, hexane:EtOAc = 9:1) to give the desired dispiro-1,2,4,5-tetraoxane.

### Preparation of 1,2,4,5-tetraoxane 39a

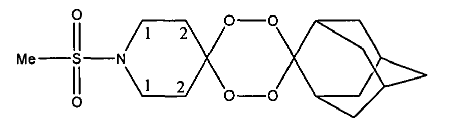

This product was prepared in 61 % according to the general procedure for preparing 1,2,4,5-tetraoxanes.

### Preparation of 1,2,4,5-tetraoxane 39b

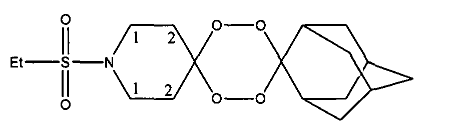

This product was prepared in 60% according to the general procedure for preparing 1,2,4,5-tetraoxanes.

### Preparation of 1,2,4,5-tetraoxane 39c

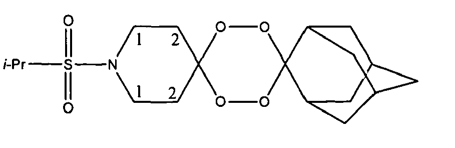

This product was prepared in 56% according to the general procedure for preparing 1,2,4,5-tetraoxanes.

### Preparation of 1,2,4,5-tetraoxane 39d

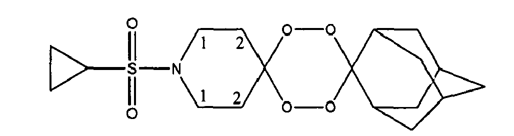

This product was prepared in 53% according to the general procedure for preparing 1,2,4,5-tetraoxanes.

### Preparation of 1,2,4,5-tetraoxane 39e

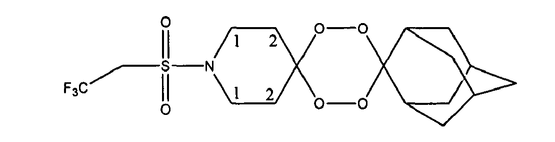

This product was prepared in 51 % according to the general procedure for preparing 1,2,4,5-tetraoxanes.

### Preparation of 1,2,4,5-tetraoxane 39f

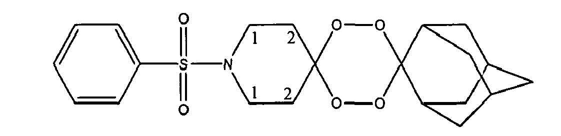

This product was prepared in 35% according to the general procedure for preparing 1,2,4,5-tetraoxanes.

### Preparation of 1,2,4,5-tetraoxane 39g

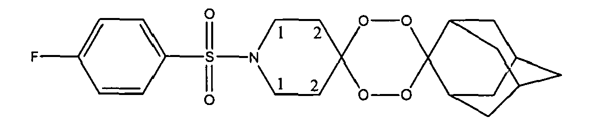

This product was prepared in 41% according to the general procedure for preparing 1,2,4,5-tetraoxanes.

### Preparation of 1,2,4,5-tetraoxane 39h

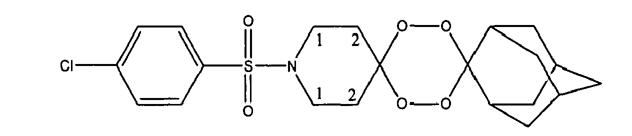

This product was prepared in 38% according to the general procedure for preparing 1,2,4,5-tetraoxanes.

### Preparation of 1,2,4,5-tetraoxane 39i

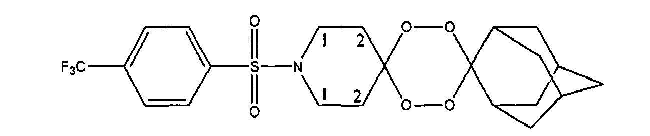

This product was prepared in 25% according to the general procedure for preparing 1,2,4,5-tetraoxanes.

### General procedure for preparation of cyclododecyl-1,2,4,5-tetraoxanes

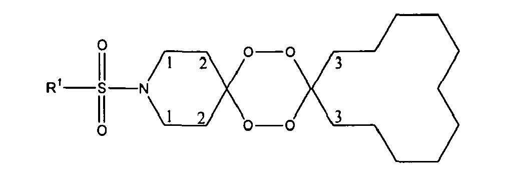

A solution of 1-R¹ sulfonyl-piperidin-4-one () (1.13 mmol), 30% H₂O₂ (0.26 ml, 2.26 mmol, 2.0 eq) and MTO (trace) in HFIP (2.27 ml) was stirred at room temperature for 2 hours. After this time cyclododecanone (412 mg, 2.26 mol, 2.0 eq) was added followed by dropwise addition of a 54% ethereal solution of HBF₄ (368 mg, 2.26 mmol, 2.0 eq). The reaction was then stirred at room temperature for 1 hour. Dichloromethane (10 ml) was added and the organic layer washed with a sat. soln. of NaHCO₃, dried over MgSO₄ and the solvent removed *in vacuo.* The resulting residue was purified by flash column chromatography (SiO₂, hexane:EtOAc = 9:1) to give the desired dispiro-1,2,4,5-tetraoxane.

### Preparation of 1,2,4,5-tetraoxane 40a

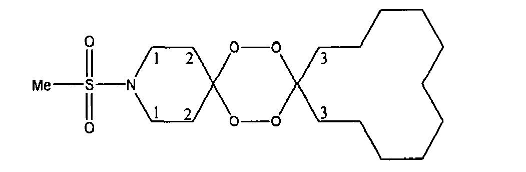

This product was prepared in 36% according to the general procedure for preparing 1,2,4,5-tetraoxanes.

### Preparation of 1,2,4,5-tetraoxane 40b

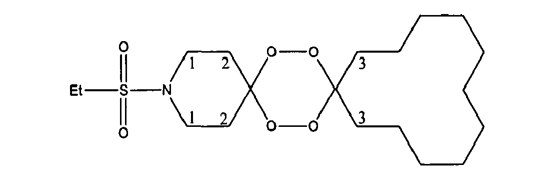

This product was prepared in 32% according to the general procedure for preparing 1,2,4,5-tetraoxanes.

### Preparation of 1,2,4,5-tetraoxane 40c

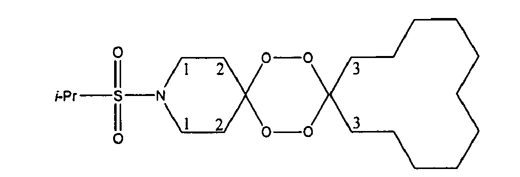

This product was prepared in 38% according to the general procedure for preparing 1,2,4,5-tetraoxanes.

### Preparation of 1,2,4,5-tetraoxane 40d

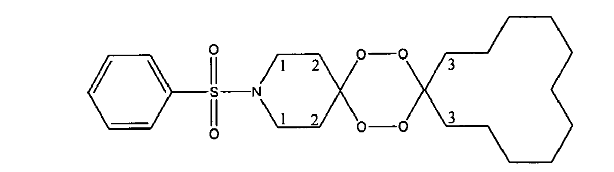

This product was prepared in 20% according to the general procedure for preparing 1,2,4,5-tetraoxanes.

### Preparation of tetraoxanes incorporating briding moieties

### Preparation of 8-Aza-bicyclo[3.2.1]octan-3-one 41b

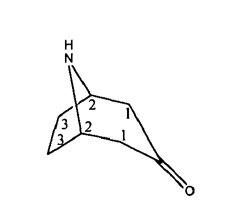

A solution of tropinone (4.38 g, 31.51 mmol) in 1,2-dicloroethane (44 ml) was cooled to 4°C, 1-chloroethyl chloroformate (3.77 ml, 34.66 mmol, 1.1 eq) was added and the solution heated at reflux for 12 hours. After cooling the solvent was evaporated and the residue dissolved in methanol (44 ml). The solution was then refluxed for an additional 5 hours. After cooling the solution was evaporated to half its volume and acetone (25 ml) was added. The flask was then placed in the fridge overnight. The product precipitated out of solution and was filtered and dried under vacuum to give the title compound as a pale yellow solid (3.27 g, 83%).

### Preparation of 8-Ethanesulfonyl-8-aza-bicyclo[3.2.1]octan-3-one 41c

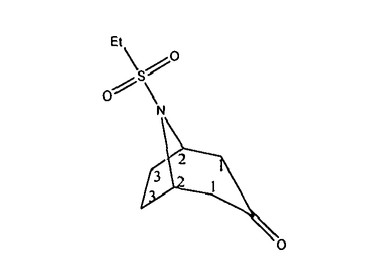

Triethylamine (0.67 ml, 4.80 mmol, 1.5 eq) was added to a solution of ketone (400 mg, 3.20 mmol) in dichloromethane (6.5 ml). The solution was cooled to 0°C and ethane sulfonyl chloride (0.32 ml, 3.84 mmol, 1.2 eq) added. The reaction was then stirred at room temperature overnight and was subsequently quenched with saturated NaHCO₃ aq. The aqueous layer was separated and extracted with DCM (3 x 10ml)). The combined organic extracts were dried over NaSO₄ and concentrated. The resulting residue was purified by flash column chromatography (SiO₂, EtOAc:hexane = 3:2) to give the title compound (437 mg, 63%).

### Preparation of tropinone derived 1,2,4,5-tetraoxane 41d

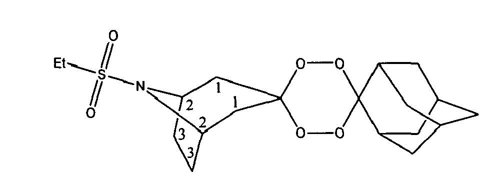

A solution of ketone (200 mg, 0.92 mmol), 30% H₂O₂ (0.21 ml, 1.84 mmol, 2.0 eq) and MTO (trace) in HFIP (1.89 ml) was stirred at room temperature for 2 hours. After this time 2-adamantanone (335 mg, 1.84 mol, 2.0 eq) was added followed by dropwise addition of a 54% ethereal solution of HBF₄ (0.25 ml, 1.84 mmol, 2.0 eq). The reaction was then stirred at room temperature for 1 hour. Dichloromethane (10 ml) was added and the organic layer washed with a sat. soln. of NaHCO₃, dried over MgSO₄ and the solvent removed *in vacuo.* The resulting residue was purified by flash column chromatography (SiO₂ hexane:EtOAc = 9:1) to give the title compound (128 mg, 35%).

### APPENDIX A

### Characterisation Data

### Cyclohexane-1,1-diyl bis-hydro peroxide 6a

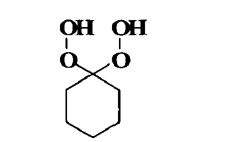

¹HNMR (400MHz, CDCl₃) δ_{H}, 1.46(m, 2H, cyclohexyl), 1.58(m, 4H, cyclohexyl), 1.84(t, 4H, J = 6.46Hz, cyclohexyl), 8.1(s, 2H, OH), ¹³CNMR (100MHz, CDCl₃), δ_{C} 22.81, 25.61, 25.69, 29.91, 111.20.

### Cyclododecane-1,1-diyl bis hydro peroxide 7a

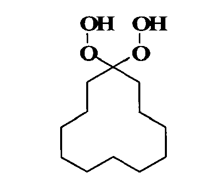

¹HNMR (400MHz, CDCl₃) δ_{H}, 1.22-1.44(m, 14H, cyclododecanyl), 1.53(m, 3H, cyclodecanyl), 1.69(d, 4H, cyclododecanyl), 2.47(d, 1H, cyclododecanyl), 8.1(s, 2H, OH) ¹³CNMR (100MHz, CDCl₃), δ_{C} 19.39, 21.94, 22.20, 22.27, 24.33, 24.83, 25.93, 25.99, 26.16, 26.51, 40.50, 115.15 MS (ES+) [M + Na] ⁺ (100), 255.2 HRMS calculated 255.1596 C₁₂H₂₄O₄Na found, 255.1607.

### Adamantane-2,2-diyl bishydroperoxide 8a

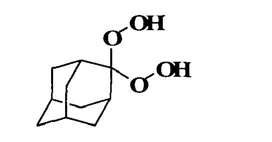

¹HNMR (400MHz, CDCl₃) δ_{H}, 1.66-1.73(m, 6H, adamantylidene), 1.88(s, 2H, adamantylidene), 1.96(s, 2H, adamantylidene), 2.0(s, 2H, adamantylidene), 2.36(s, 2H, adamantylidene), 8.02(s, 2H, OH), ¹³CNMR (100MHz, CDCl₃), δ_{C} 27.42, 31.56, 34.14, 37.44, 112.85 MS (ES+) [M + Na] ⁺ (100), 223.1, HRMS calculated for 223.0970 C₁₂H₁₆O₄Na found, 233.0962.

### 7,8,15,16-Tetraoxa-dispiro[5.2.5.2]hexadecan-3-one 10

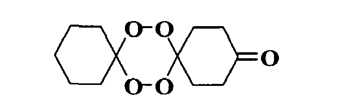

Mpt. 78-80°C *V*ₘₐₓ (CHCl₃)/cm⁻¹1719.8, 2856.2, 2942.3, 3012.7 ¹HNMR (400MHz, CDCl₃) δ_{H}, 1.5(m, 6H, cyclohexyl), 1.80(s, 4H, cyclohexyl), 2.15(t, 2H, CH₂), 2.30(t, 2H, CH₂), 2.5(m, 4H, CH₂), ¹³CNMR (100MHz, CDCl₃), δ_{C} 14.0, 23.07, 25.84, 31.98, 37.25, 106.60, 108.56, 210.77, MS (ES+) [M + Na] ⁺ (100), 265.0, [M + Na + CH₃OH]⁺ (60) 297.1

### 7,8,21,22-Tetraoxa-dispiro[5.2.11.2]docosan-3-one 11

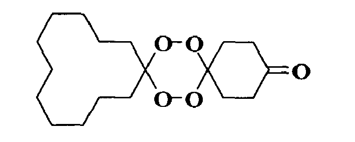

*V*ₘₐₓ (CHCl₃)/cm⁻¹ 1715.9, 2856.2, 2926.7, 3012.7 Mpt. 108-110°C 1HNMR (400MHz, CDCl₃) δ_{H} 1.2-1.7(m, 18H, cyclododecanyl), 1.96(bs, 4H, cyclodecanyl), 2.28(bs, 2H, CH₂), 2.42(d, 2H, CH₂), 2.66(bs, 2H, CH₂). ¹³CNMR (100MHz, CDCl₃), δ_{C} 14.5, 22.98, 25.06, 31.97, 106.94, 113.35, 209.48. MS (ES+) [M + Na] ⁺ (100), 349.6

### Adamantane tetraoxane ketone 12

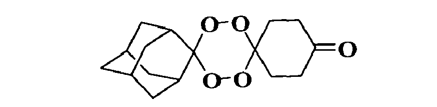

Mpt. 156-158°C *V*ₘₐₓ (CHCl₃)/cm⁻¹1722.2, 2854.9, 2912.3, 3010.7 ¹HNMR (400MHz, CDCl₃) δ_{H}, 1.59-1.83(m, 4H, adamantyl), 1.88-2.13(m, 8H, adamantyl), 2.41-2.52(m, 4H, CH₂), 2.54(bs, 4H, CH₂O), 2.68-2.78(m, 2H, CH₂) ¹³CNMR (100MHz, CDCl₃), δ_{C} 27.38, 27.84, 33.51, 36.69, 37.25, 39.64, 47.36, 106.97, 111.38, 209.63 MS (ES+) [M + Na] ⁺ (100), 317.1 HRMS calculated for 317.1365 C₁₆H₂₂O₅Na, found 317.1331.

### 4-(7,8,15,16-Tetraoxa-dispiro[5.2.5.2]hexadec-3-yl)-morpholine 19

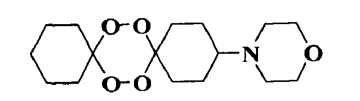

*V*ₘₐₓ(CHCl₃)/cm⁻¹ 1444.5, 2859.1, 2931.2, 3011.3 ¹HNMR (400MHz, CDCl₃) δ_{H} 1.4-1.5(m, 4H, cyclohexyl), 1.6(bs, 6H, cyclohexyl), 1.7-1.9(m, 6H, cyclohexyl), 2.15-2.3(m, 2H, cyclohexyl), 2.35(m, 1H, CH), 2.55(t, 4H, J = 4.61Hz, NCH₂), 3.7(t, 4H, J = 4.61Hz, NCH₂) ¹³CNMR (100MHz, CDCl₃), δ_{C} 22.39, 24.20, 25.31, 25.76, 30.06, 30.63, 32.76, 33.38, 34.95, 35.00, 50.14, 50.41, 62.50, 67.74, 107.99, 108.72. MS (ES+) [M + H]⁺ (100) 314.2 [M - H + Na] ⁺ (50) 336.1, HRMS (CI+) calculated for 314.19675 C₁₆H₂₈O₅N found, 314.19687

### Cyclopropyl-(7,8,15,16-tetraoxa-dispiro[5.2.5.2]hexadec-3-yl)-amine 14

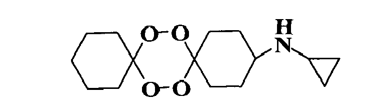

*V*ₘₐₓ (CHCl₃)/cm⁻¹ 14445.3, 2856.2, 2934.5, 3012.7, 3443.2 ¹HNMR (400MHz, CDCl₃) δ_{H} ¹³CNMR 0.36(m, 2H, cyclopropyl), 0.47(m, 2H, cyclopropyl), 1.37-1.37(m, 4H, cyclohexyl), 1.52-1.66(m, 6H, cyclohexyl), 1.84-1.99(m, 4H, cyclohexyl), 2.14(m, 1H, CH), 2.18-2.49(m, 4H, cyclohexyl), 2.75(m, 1H, CH), 5.7(bs, 1H, NH) (100MHz, CDCl₃), δ_{C} 8.54, 22.38, 24.19, 25.76, 27.76, 28.64, 28.82, 30.09, 30.52, 32.47, 32.95, 34.99, 56.00, 108.18, 109.62 MS (ES+) [M + H] ⁺ (100),283.8 HRMS (CI+) calculated for 284.18616 C₁₅H₂₆O₄N found 284.18622,

### (1,4-Dioxa-spiro[4.5]dec-8-ylidene)-acetic acid ethyl ester 22

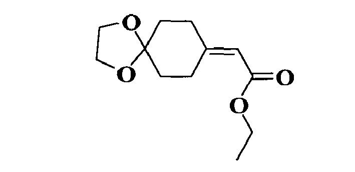

*V*ₘₐₓ (neat)/cm⁻¹ 926.3, 1104.9, 1169.1, 1237.8, 1269.8, 1301.9, 1352.3, 1430.2, 1650.1, 1709.6, 2876.1, 2949.4 ¹HNMR (400MHz, CDCl₃) δ_{H}, 1.28(t, 3H, J = 7.15Hz, CH₃), 1.77(m, 4H, cyclohexyl), 2.38(t, 2H, J = 6.68Hz, CH₂), 3.0(t, 2H, J = 7.47Hz, CH₂), 3.98(s, 4H, OCH₂), 4.15(q, 2H, J = 7.15Hz, CH₂), 5.7(s, 1H, CH), ¹³CNMR (100MHz, CDCl₃), δ_{C} 14.31, 26.09, 34.61, 35.01, 35.81, 59.63, 64.47, 108.06, 114.37, 160.14, 166.56. MS (CI) [M + H] ⁺ (100), 227 [M + NH₄] ⁺ (95), 244, HRMS calculated for 227.1283 C₁₂H₁₉O₄ found, 227.1280.

### (1,4-Dioxa-spiro[4.5]dec-8-ylidene)-acetic acid methyl ester 21

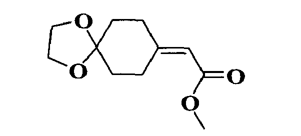

*V*ₘₐₓ (neat)/cm⁻¹ 860.1, 908.0, 1028.0, 1084.0, 1120.0, 1168.0, 1204.01272.0, 1432.0, 1652.0, 1716.0, 2879.9, 2943.9 ¹HNMR (400MHz, CDCl₃) δ_{H}, 1.73-1.80(m, 4H, cyclcohexyl), 2.38(dt, 2H, J = 6.45Hz, cyclohexyl), 3.0(dt, 2H, J = 6.46Hz, cyclohexyl), 3.68(s, 3H, OCH₃), 3.97(s, 4H, OCH₂), 5.68(s, 1H, CH) ¹³CNMR (100MHz, CDCl₃), δ_{C} 26.39, 34.90, 35.31, 36.10, 51.13, 64.75, 108.27, 114.20, 160.82, 167.18 MS (CI) [M + H] ⁺ (60), 227 [M + NH₄] ⁺ (100), 230, HRMS calculated for 213.1127 C₁₁H₁₇O₄ found, 213.1122

### (1,4-Dioxa-spiro[4.5]dec-8-yl)-acetic acid ethyl ester 24

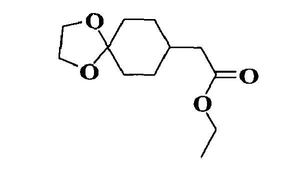

*V*ₘₐₓ (neat)/cm⁻¹ 926.3, 1031.6, 1104.9, 1169.9, 1237.8, 1288.2, 1375.2, 1443.9, 1728.0, 2876.1, 2931.0, ¹HNMR (400MHz, CDCl₃) δ_{H}, 1.25.(t, 3H, J = 7.15Hz, CH₃), 1.33(m, 2H, cyclohexyl), 1.56(m, 2H, cyclohexyl), 1.74(d, 4H, J = 6.99Hz, cycloheyxl), 2.2(d, 2H, J = 6.99Hz, CH₂CO), 3.93(s, 4H, OCH₂), 4.13(q, 2H, J = 7.15Hz, CH₂), 5.7(s, 1H, CH), ¹³CNMR (100MHz, CDCl₃), δ_{C} 14.29, 30.02, 30.16, 33.34, 33.50, 34.16, 34.48, 41.01, 60.35, 64.25, 108.62, 172.87. MS (CI) [M + H] ⁺ (100), 229 [M + NH₄] ⁺ (30), 246, HRMS calculated for 229.1440 C₁₂H₁₉O₄ found, 229.1440.

### (1,4-Dioxa-spiro[4.5]dec-8-yl)-acetic acid methyl ester 23

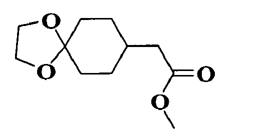

*V*ₘₐₓ (neat)/cm⁻¹ 932.1, 1032.0, 1108.0, 1164.0, 1240.0, 1288.0, 1436.0, 1732.0, 2879.9, 2935.9, ¹HNMR (400MHz, CDCl₃) δ_{H}, 1.24-1.37(m, 2H, cyclohexyl), 1.56(dt, 2H, J = 12.91H,z, 12.52Hz, cyclohexyl), 1.73(4H, J = 9.49Hz, cyclohexyl), 1.79-1.90(m, 1H, CH), 2,24(d, 2H, J = 7.02Hz, CH₂CO), 3.67(s, 3H, OCH₃), 3.94(s, 4H, OCH₂) ¹³CNMR (100MHz, CDCl₃), δ_{C} 30.31, 33.75, 34.59, 41.03, 51.66, 64.53, 108.85, 173.56 MS (CI) [M + H] ⁺ (100), 215 [M + NH₄] ⁺ (40), 232, HRMS calculated for 215.1283 C₁₁H₁₉O₄ found, 215.1283.

### (4,4-Bis-hydroperoxy-cyclohexyl)-acetic acid ethyl ester 26

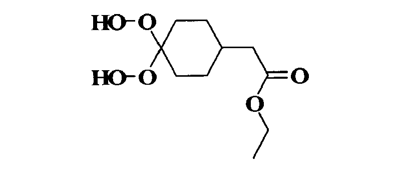

¹HNMR (400MHz, CDCl₃) δ_{H}, 1.26(t, 3H,. J = 7.15Hz, CH₃), 1.62(m, 2H, cyclohexyl),1.78(m, 4H, cyclohexyl), 1.92(m, 2H, cyclohexyl), 2.22(d, 2H, J = 13.51Hz, CH₂CO), 2.4(m, 1H, CH), 4.14(q, 2H, J = 7.15Hz, OCH₂), 8.55(bs, 2H, OH), ¹³CNMR (100MHz, CDCl₃), δ_{C} 14.20, 24.78, 28.19, 41.76, 60.67, 109.58

### (4,4-Bis-hydroperoxy-cyclohexyl)-acetic acid methyl ester 25

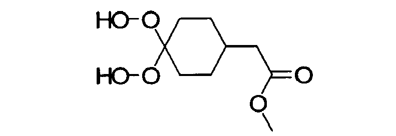

*V*ₘₐₓ (CHCl₃)/cm⁻¹ 1111.7, 1166.0, 1243.0, 1292.8, 1351.7, 1437.7, 1709.7, 2858.9, 2940.4, 3393.2 ¹HNMR (400MHz, CDCl₃) δ_{H}, 1.22-1.34(m, 2H, cyclohexyl), 1.52(dt, 2H, J = 13.09Hz, 13.66Hz, cyclohexyl), 1.70(dd, 2H, J, 3.42Hz, cyclohexyl), 1.80-1.94(m, 3H, cyclohexyl/CH), 2.27(d, 2H, J = 7.03Hz, CH₂CO), 3.68(3H, OCH3), 9.72(bs, 2H, OH) ¹³CNMR (100MHz, CDCl₃), δ_{C} 25.89, 28.79, 29.19, 34.07, 40.97, 51.98, 110.11, 174.24 MS (ES+) [M + Na] ⁺ (100), 243.1 HRMS calculated for 243.0845 C₉H₁₆O₆Na found, 243.0891

### (7,8,15,16-Tetraoxa-dispiro[5.2.5.2]hexadec-3-yl)acetic acid ethyl ester 27a

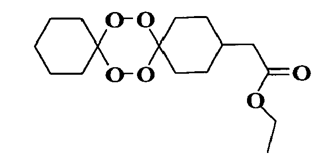

*V*ₘₐₓ (CHCl₃)/cm⁻¹ 1444.8, 1731.6, 2853.8, 2926.4, 3014.3 ¹HNMR (400MHz, CDCl₃) δ_{H}, 1.25(t, 4H, J = 7.15Hz, CH₃), 1.4-1.84(m, 14H, cyclohexyl), 1.9(m, 2H, CH₂), 2.14-2.50(m, 4H, cyclohexyl), 3.09(bs, 1H, CH), 4.13(q, 2H, J = 4.45Hz, CH₂) ¹³CNMR (100MHz, CDCl₃), δ_{C} 14.65, 22.57, 25.75, 28.75, 29.10, 31.46, 34.07, 41.12, 60.79, 108.15, 108.70, 173.07 MS (ES+) [M + Na] ⁺ (100), 337.2 [2M + Na] ⁺, 651.4 HRMS calculated for 337.1627 C₁₂H₂₀O₆Na found, 337.1615

### (7,8,21,22-Tetraoxa-dispiro[5.2.11.2]docos-3-yl)-acetic acid ethyl ester 28a

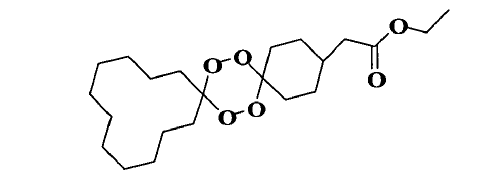

*V*ₘₐₓ (CHCl₃)/cm⁻¹ 1450.0, 1723.0, 2849.8, 2936.8, 3020.4, 3435.3 ¹HNMR (400MHz, CDCl₃) δ_{H}, 1.25(t, 3H, J = 7.21Hz, CH₃), 1.26-1.40(m, 16H, CH₂), 1.40-1.49(m, 4H, CH₂), 1.50-1.62(m, 4H, CH₂), 1.64-1.81(m, 6H, CH₂), 1.83-1.99(m, 1H, CH), 1.23(d, 2H, J = 4.56Hz, CH₂CO), 4.13(q, 2H, J = 7.21Hz, OCH₂) ¹³CNMR (100MHz, CDCl₃), δ_{C} 14.67, 22.65, 22.97, 24.62, 24.99, 25.15, 25.77, 26.29, 26.39, 27.82, 34.10, 40.79, 41.15, 60.71, 107.94, 112.81, 173.11 MS (ES+), m/z 398.53 [M + Na] ⁺ (100), 421.1HRMS calculated for421.2566 C₂₂H₃₈O₆Na found, 421.2581.

### Adamantyl tetraoxane ethylester 29a

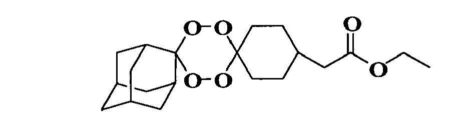

Mpt. 60-62°C *V*ₘₐₓ (CHCl₃)/cm⁻¹ 1446.8, 1718.5, 2858.9, 2922.3, 3003.8 ¹HNMR (400MHz, CDCl₃) δ_{H}, 1.25(t, 3H, J = 7.31Hz, CH₃), 1.28-1.37(m, 2H, CH₂), 1.48-1.79(m, 10H, CH₂), 1.87(bs, 2H, CH₂), 1.91-2.20(m, 9H, CH₂/CH), 2.23(d, 2H, J = 6.83Hz, CH₂CO), 4.13(q, 2H, J = 7.21Hz, CH₂)¹³CNMR (100MHz, CDCl₃), δ_{C} 14.62, 27.48, 27.87, 34.10, 36.72, 37.37, 39.65, 41.12, 47.38, 60.62, 107.99, 110.78, 173.00 MS (ES+), [M + Na] ⁺ (100), 389.1 [2M + Na] ⁺ 755.2 HRMS calculated for 389.1940 C₂₀H₃₀O₆Na found, 389.1954.

### Adamantyl tetraoxane methyl ester 30a

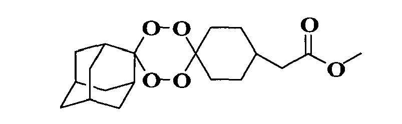

*V*ₘₐₓ (neat)/cm⁻¹ 1921.5, 994.0, 1043.81102.6, 1161.5, 1238.5, 1446.8, 1736.6, 2849.8, 29.13.2 ¹HNMR (400MHz, CDCl₃) δ_{H}, 1.18-1.37(m, 2H, adamantly), 1.50-1.77(m, 12H, CH2), 1.80-1.89(m, 4H, CH₂), 1.90-2.03(m, 5H, CH), 2.25(d, 2H, J = 6.64Hz, CH₂CO), 3.68(s, 3H, OCH₃) ¹³CNMR (100MHz, CDCl₃), δ_{C} 27.48, 32.61, 33.54, 34.08, 34.60, 35.32, 36.87, 37.37, 40.85, 51.85, 107.96, 110.79, 173.41 MS (ES+), [M + Na] ⁺ (100), 375.1 HRMS calculated for 375.1784 C₁₉H₂₈O₆Na found, 375.1774.

### 7,8,15,16-Tetraoxa-dispiro[5.2.5.2]hexadec-3-yl)acetic acid 27b

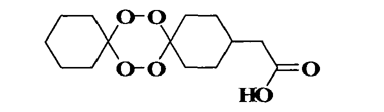

¹HNMR (400MHz, CDCl₃) δ_{H}, 1.2-1.37(m, 4H, cyclohexyl), 1.46(m, 2H, cyclohexyl), 1.57(bs, 6H, cyclohexyl), 1.75(m, 4H, cyclohexyl), 1.88(m, 1H, CH), 2.27(d, 2H, J = 6.3Hz, CH₂CO), 2.12-2.39(m, 2H, cyclohexyl) ¹³CNMR (100MHz, CDCl₃), δ_{C} 23.12, 25.76, 25.80, 25.92, 28.97, 30.02, 30.24, 30.95, 32.28, 33.86, 40.63, 107.51, 108.04, 178.42 MS (ES+), [M - H] ⁺ (100), 285.1, [2M-H]⁺, 571.1

### (7,8,21,22-Tetraoxa-dispiro[5.2.11.2]docos-3-yl)-acetic acid 28b

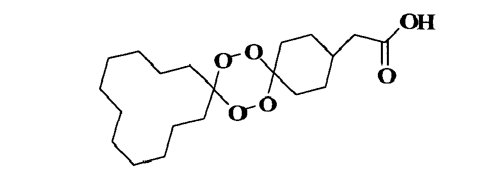

*V*ₘₐₓ (CHCl₃)/cm⁻¹ 1692.8, 2851.1, 2931.2, 3019.3, 3355.7 ¹HNMR (400MHz, CDCl₃) δ_{H}, 1.22-1.45(m, 22H, CH₂), 1.51-1.64(m, 4H, CH₂), 1.65-1.77(m, 4H, CH₂), 1.90-1.90(m, 1H, CH), 2.28(d, 2H, J = 7.03Hz, CH₂CO), ¹³CNMR (100MHz, CDCl₃), δ_{C} 19.77, 22.41, 22.98, 24.70, 25.06, 25.20, 26.37, 29.77, 40.81, 107.30, 118.89, 177.48

### Adamantyl tetraoxane carboxylic acid 29b

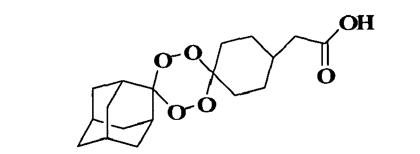

*V*ₘₐₓ (CHCl₃)/cm⁻¹ 991.8, 1057.5, 1446.7, 1694.3, 2844.0, 2924.8, 3005.7 3355.7 ¹HNMR (400MHz, CDCl₃) δ_{H}, 1.22-1.46(m, 2H, CH₂), 1.50-1.90(m, 12H, CH₂), 1.01-2.05(m, 4H, CH₂), 2.06-2.15(m, 5H, CH), 2.29(d, 2H, J = 6.83Hz, CH₂CO), ¹³CNMR (100MHz, CDCl₃), δ_{C} 27.47, 27.84, 33.52, 33.86, 36.69, 37.35, 39.65, 40.75, 47.34, 108.89, 110.79, 178.23. MS (ES+), [M - H] ⁺ (100), 337.2 HRMS calculated for 337.1651 C₁₈H₂₅O₆ found, 337.1663

### 1-Morpholin-4-yl-2-(7,8,15,16-tetraoxa-dispiro[5.2.5.2]hexadec-3-yl)-ethanone 27h

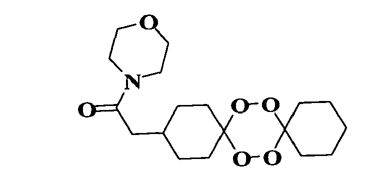

*V*ₘₐₓ (CHCl₃)/cm⁻¹ 1444.5, 1632.7, 2851.1, 2931.2, 3011.3 Mpt. 126-128°C ¹HNMR (400MHz, CDCl₃) δ_{H}, 1.19-1.35(m, 4H, cyclohexyl), 1.46(bs, 2H, cyclohexyl), 1.57(bs, 6H, cyclohexyl), 1.77(m, 4H, cyclohexyl), 1.98(m, 1H, CH) 2.16-2.35(m, 4H, CH₂/cyclohexyl), 3.45(t, 2H, J = 4.76Hz, NCH₂), 3.59-3.67(m, 6H, CH₂O). ¹³CNMR (100MHz, CDCl₃), δ_{C} 25.76, 34.20, 39.22, 67.35, 108.21, 108.69, 170.9 MS (ES+), [M + Na] ⁺ (100) 378.2, [2M + Na]⁺ 733.4 HRMS calculated for 378.1893 C₁₈H₂₉NO₆Na found, 378.1886.

### N-Cyclopropyl-2-(7,8,15,16-tetraoxa-dispiro[5.2.5.2]hexadec-3-yl)-acetamide 27c

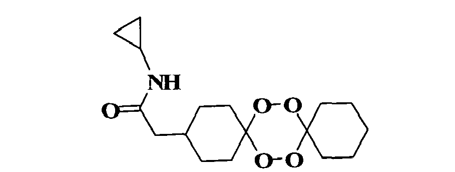

*V*ₘₐₓ (CHCl₃)/cm⁻¹ 1444.6, 1535.6, 1636.7, 2851.1, 2939.2, 3019.3, 3299.6 Mpt. 148-150°C ¹HNMR (400MHz, CDCl₃) δ_{H} 0.47(m, 2H, cyclopropyl), 0.77(m, 2H, cyclopropyl), 1.25(m, 4H, cyclohexyl), 1.46(m, 2H, cyclohexyl), 1.57(bs, 6H, cyclohexyl), 1.72(m, 4H, cyclohexyl), 1.94(m, 1H, CH), 2.02(d, 2H, J = 5.04Hz, CH₂CO), 1.96-2.08(m, 2H, cyclohexyl), 2.71(m, 1H, CH-cyclopropyl), 5.7(bs, 1H, NH) ¹³CNMR (100MHz, CDCl₃) δ_{C} 7.03, 22.99, 25.75, 34.43, 43.49, 108.20, 108.67, 173.54 MS (ES+), [M + Na] ⁺ (100) 348.2, [2M + Na]+ 673.3 HRMS calculated for 348.1787 C₁₇H₂₇O₅NNa found, 348.1791.

### N-(2-Pyrrolidin-1-yl-ethyl)-2-(7,8,15,16-tetraoxa-dispiro[5.2.5.2]-hexadec-3-yl)-acetamide 27d

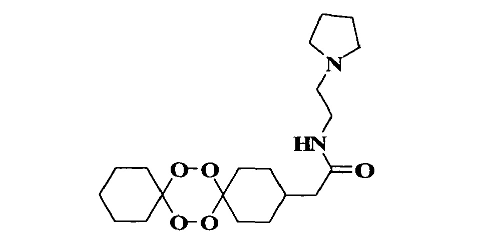

*V*ₘₐₓ (CHCl₃)/cm⁻¹ 1444.6, 1512.6, 1652.8, 2859.2, 2931.2, 3011.3, 3315.6 Mpt. 110-112°C ¹HNMR (400MHz, CDCl₃) δ_{H}, 1.2-1.34(m, 4H, cyclohexyl), 1.47(m, 2H, cyclohexyl), 1.57(bs, 6H, cyclohexyl), 1.73(m, 4H, cyclohexyl), 1.83-2.1(m, 5H, CH/CH₂), 2.16(d, 2H, J = 6.99Hz, CH₂CO), 2.23-2.32(m, 2H,cyclohexyl), 2.68-2.79(m, 2H, CH₂N), 2.86(t, 4H, J = 6.04Hz, NCH₂), 3.49(q, 2H, J = 5.88Hz, NHCH₂), 6.98(bs, 1H, NH). ¹³CNMR (100MHz, CDCl₃), δ_{C} 15.01, 23.77, 23.82, 25.75, 34.42, 37.26, 43.33, 54.42, 55.57, 108.23, 108.61, 172.79 MS (ES+), m/z 382.49 [M + H] ⁺ (74.77) 383.1, [M + Na] ⁺ (100) 405.1 HRMS calculated for 383.2546 C₂₀H₃₅O₅ found, 383.2553 and 405.2365 C₂₀H₃₄N₂O₅Na, found, 405.2364.

### N-(2-Piperidin-1-yl-ethyl)-2-(7,8,15,16-tetraoxa-dispiro[5.2.5.2]-hexadec-3-yl)-acetamide 27e

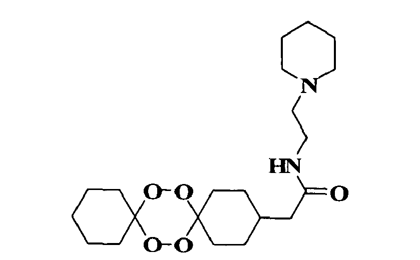

*V*ₘₐₓ (CHCl₃)/cm⁻¹ 1444.4, 1508.6, 1648.8, 2856.2, 2934.5, 3012.7, 3325.8 Mpt. 68-78°C ¹HNMR (400MHz, CDCl₃) δ_{H}, 1.25(t, 4H, J = 7.16Hz, cyclohexyl), 1.47(m, 4H, cyclohexyl/piperidyl), 1.57(bs, 6H, cyclohexyl), 1.65(m, 4H, cyclohexyl), 1.74(m, 4H, piperidyl), 1.94(m, 1H, CH), 2.15(d, 2H, J = 7.0Hz, CH₂CO), 2.30(m, 2H, cyclohexyl), 2.53(m, 2H, NCH₂), 2.66(m, 4H, CH₂N), 3.45(q, 2H, J = 5.73Hz, NHCH₂), 6.94(bs, 1H, NH). ¹³CNMR (100MHz, CDCl₃), δ_{C} 20.08, 21.53, 21.99, 22.75, 23.41, 32.11, 33.24, 35.34, 41.07, 52.26, 52.39, 55.38, 55.77, 58.78, 105.89, 106.27, 170.30 MS (ES+), [M + H] ⁺ (66.29) 397.1, [M + Na]⁺ (100) 419.1 HRMS calculated for 397.2702 C₂₁H₃₇N₂O₅ found, 397.2704, and for 419.2522 C₂₁H₃₆N₂O₅Na found 419.2518.

### N-(2-Morpholin-4-yl-ethyl)-2-(7,8,15,16-tetraoxa-dispiro[5.2.5.2]-hexadec-3-yl)-acetamide 27f

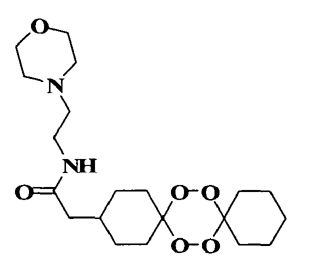

*V*ₘₐₓ (CHCl₃)/cm⁻¹ 1444.4, 1508.6, 1656.8, 2811.1, 2851.1, 2931.2, 3307.6 ¹HNMR (400MHz, CDCl₃) δ_{H}, 1.25(m, 4H, cyclohexyl), 1.47(m, 2H, cyclohexyl), 1.58(m, 6H, cyclohexyl), 1.70-1.78(m, 4H, cyclohexyl), 1.94(m, 1H, CH), 2.1(d, 2H, J = 7.15Hz, CH₂CO), 2.13-2.37(m, 2H, cyclohexyl), 2.41-2.51(m, 6H, CH₂N/NCH₂), 3.36(q, 2H, J = 5.88Hz, NHCH₂), 3.7(m, 4H, CH₂O), 5.98(bs, 1H, NH) ¹³CNMR (100MHz, CDCl₃), δ_{C} 15.03, 25.74, 34.44, 35.92, 43.66, 53.75, 57.52, 67.25, 67.29, 108.20, 108.68, 172.16 MS (ES+), [M + Na] ⁺ (100) 421.1, HRMS calculated for 421.2315 C₂₀H₃₄O₆N₂Na found, 421.2323.

### N-(2-Diethylamino-ethyl)-2-(7,8,15,16-tetraoxa-dispiro[5.2.5.2]-hexadec-3-yl)-acetamide 27g

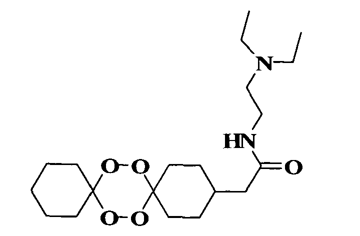

*V*ₘₐₓ (CHCl₃)/cm⁻¹ 1444.5, 1508.6, 1652.8, 2859.1, 2939.2, 3011.4, 3323.6 ¹HNMR (400MHz, CDCl₃) δ_{H}, 1.02(t, 3H, J = 7.15Hz, CH₃), 1.05(t, 3H, J = 7.15Hz, CH₃), 1.25(m, 4H, cyclohexyl), 1.46(m, 2H, cyclohexyl), 1.59(bs, 6H, cyclohexyl), 1.74(m, 4H, cyclohexyl), 1.94(m, 1H, CH), 2.1(d, 2H, J = 7.16Hz, CH₂CO), 2.14-2.35(m, 2H, cyclohexyl), 2.57(m, 6H, CH₂N/NCH₂), 3.23(q, 1H, J = 5.88Hz, NHCH₂), 3.33(q, 1H, J = 6.2Hz, NHCH₂), 6.30(bs, 1H, NH) ¹³CNMR (100MHz, CDCl₃), δ_{C} 11.76, 11.98, 15.02, 25.73, 34.43, 36.95, 43.60, 47.19, 51.99, 52.33, 108.21, 108.60, 172.25 MS (ES+), [M + H] ⁺ (100) 385.2, HRMS calculated for 385.2702 C₂₀H₃₇N₂O₅ found, 385.2695.

### (2-7,8,15,16-Tetraoxa-dispiro[5.2.5.2]hexadec-3-yl-acetylamino)-acetic acid methyl ester 27i

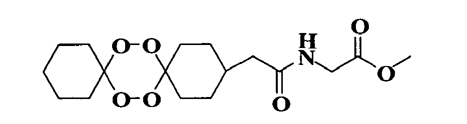

*V*ₘₐₓ (CHCl₃)/cm⁻¹1440.5, 1516.6, 1692.8, 1744.9, 2859.1, 2931.2, 3011.3, 3419.7 ¹HNMR (400MHz, CDCl₃) δ_{H} 1.24(m, 4H, cyclohexyl), 1.46(bs, 2H, cyclohexyl), 1.51, bs, 4H, cyclohexyl), 1.90(m, 1H, CH), 2.18(d, 2H, J = 7.15Hz, CH₂CO), 2.20-2.48(m, 2H, cyclohexyl), 3.70(s, 3H, OCH₃), 4.05(d, 2H, J = 5.08Hz, NCH₂), 6.04(s, 1H, NH) ¹³CNMR (100MHz, CDCl₃), δ_{C} 22.44, 25.75, 33.89, 41.56, 43.25, 51.14, 52.74, 108.17, 108.68, 170.86, 172.51 MS (ES+), [M + Na] ⁺ (100) 380.1 HRMS calculated for 380.1685 C₁₇H₂₇NO₇ Na found, 380.1778.

### N-Cyclopropyl-2-(7,8,21,22-tetraoxa-dispiro[5.2.11.2]docos-3-yl)-acet-amide 28c

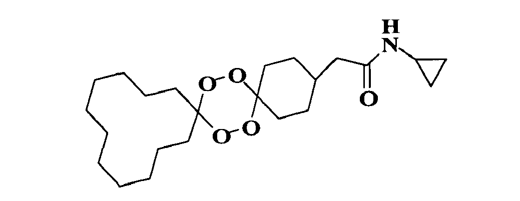

Mpt. 136-138°C *V*ₘₐₓ (CHCl₃)/cm⁻¹ 1523.8, 1637.0, 2849.8, 2931.3, 3003.8, 3311.7 ¹HNMR (400MHz, CDCl₃) δ_{H}, 0.46(m, 2H, cyclopropyl), 0.77(m, 2H, cyclopropyl), 1.14-1.47(m, 22H, CH₂), 1.50-1.84(m, 8H, CH₂),1.94(m, 1H, CH), 2.02(d, 2H, J = 7.02Hz, CH₂CO), 2.70(m, 1H, CH), 5.6(bs, 1H, NH), ¹³CNMR (100MHz, CDCl₃), δ_{C} 7.04, 8.88, 22.43, 22.72, 23.00, 26.33, 26.39, 28.74, 29.56, 34.46, 43.51, 107.99, 112.77, 173.54 MS (ES+), [M + Na] ⁺ (100), 432.2 [2M + Na] ⁺, 841.4 HRMS calculated for 432.2726 C₂₃H₃₉O₅Na found, 432.2723.

### N-(2-Pyrrolidin-1-yl-ethyl)-2-(7,8,21,22-tetraoxa-dispiro[5.2.11.2]do-cos-3-yl)-acetamide 29d

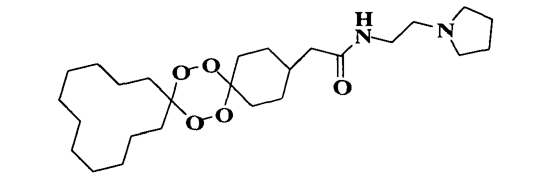

Mpt. 108-110°C *V*ₘₐₓ(CHCl₃)/cm⁻¹ 1548.6, 1628.7, 2859.1,, 2931.5, 3003.7, 3327.1 ¹HNMR (400MHz, CDCl₃) δ_{H}, 1.10-1.49(m, 22H, CH₂), 1.50-1.83(m, 8H, CH₂),1.94-2.00(m, 5H, CH), 2.16(d, 2H, J = 7.03Hz, CH₂CO), 2.81-3.18 (m, 6H, NCH₂/CH₂N), 3.51(q, 2H, J = 5.70Hz, NHCH₂), 7.1(bs, 1H, NH) ¹³CNMR (100MHz, CDCl₃), δ_{C} 18.60, 19.74, 22.34, 22.70, 23.77, 26.30, 26.37, 28.58, 29.40, 31.54, 34.41, 37.14, 43.28, 54.42, 55.48, 107.97, 112.67, 172.88 MS (ES+), [M + H] ⁺ (100), 467.3 HRMS calculated for 467.3485 C₂₆H₄₇O₅N₂ found, 467.3487.

### N-(2-Piperidin-1-yl-ethyl)-2-(7,8,21,22-tetraoxadispiro[5.2.11.2]-docos-3-yl)-acetamide 28e

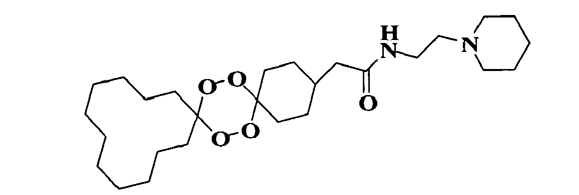

Mpt. 96-98°C *V*ₘₐₓ (CHCl₃)/cm⁻¹ 1505.7, 1650.6, 2849.0, 2931.3, 3019.3, 3320.8 ¹HNMR (400MHz, CDCl₃) δ_{H}, 1.18-1.64(m, 30H, CH₂), 1.65-1.79(m, 6H, CH₂), 1.89-1.86-1.90(m,1H, CH)1.13(d, 2H, J = 7.02Hz, CH₂CO), 2.49-2.62(m, 6H, CH₂N/NCH₂), 3.41(q, 2H, J = 5.88Hz, NHCH₂), 6.20(bs, 1H, NH) ¹³CNMR (100MHz, CDCl₃), δ_{C} 22.72, 24.17, 25.51, 26.39, 34.50, 35.81, 43.55, 54.65, 57.69, 108.03, 112.73, 172.47 MS (ES+), [M + Na] ⁺ (100), 5.5.2 [M + H] ⁺, 481.2 HRMS calculated for 503.3461C₂₇11₄₈ON₂Na found, 503.3449.

### N-(2-Morpholin-4-yl-ethyl)-2-(7,8,21,22-tetraoxa-dispiro[5.2.11.2]do-cos-3-yl)-acetamide 28f

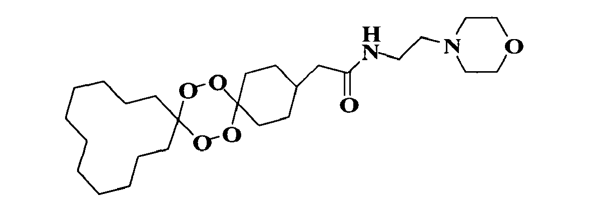

Mpt. 78-80°C *V*ₘₐₓ (CHCl₃)/cm⁻¹ 1533.1, 1643.0, 2806.2, 2850.2, 2920.5, 3315.9 ¹HNMR (400MHz, CDCl₃) δ_{H}, 1.10-1.49(m, 22H, CH₂), 1.50-1.80(m, 8H, CH₂),1.86(m, 1H, CH), 2.11(d, 2H, J = 7.03Hz, CH₂CO), 2.42-2.51(m, 6H, NCH₂/CH₂N), 3.37(q, 2H, J = 5.88Hz, NHCH₂),3.72(t, 4H, J = 4.55Hz, CH₂O), 6.0(bs, 1H, NH) ¹³CNMR (100MHz, CDCl₃), δ_{C} 19.67, 19.73, 19.81, 22.33, 2251, 22.59, 26.28, 26.35, 26.54, 26.59, 26.98, 27.06, 28.77, 29.21, 29.49, 29.80, 31.86, 34.70, 35.87, 44.01, 53.72, 57.50, 67.26, 107.47, 112.14, 172.55 MS (ES+), [M + H] ⁺ (100), 483.3 [M + Na] ⁺, 505.2 HRMS calculated for 483.3434 C₂₆H₄₇O₆N₂ found, 483.3424.

### N-(2-Diethylamino-ethyl)-2-(7,8,21,22-tetraoxa-dispiro[5.2.11.2]-docos-3-yl)-acetamide 28g

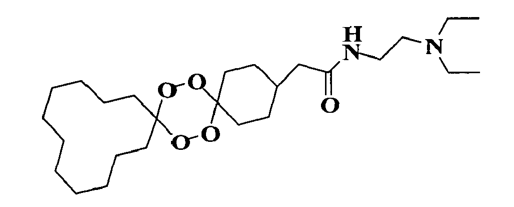

Mpt. 64-66°C *V*ₘₐₓ (CHCl₃)/cm⁻¹ 1446.6, 1660.8, 2812.3, 2931.2, 3003.8, 3251.6 ¹HNMR (400MHz, CDCl₃) δ_{H}, 1.15(t, 6H, J = 7.21Hz, CH₃), 1.23-1.49(m, 22H, CH₂), 1.50-1.79(m, 8H, CH₂), 1.85(m, 1H, CH), 2.12(d, 2H, J = 7.02Hz, CH₂CO), 2.73(q, 6H, J = 7.02Hz, NCH₂/CH₂N), 3.42(q, 2H, J = 5.89Hz, NCH₂), 6.30(bs, 1H, NH) ¹³CNMR (100MHz, CDCl₃), δ_{C} 10.97, 19.70, 19.76, 19.79, 22.40, 22.59, 22.67, 26.33, 26.38, 26.56, 26.62, 27.04, 27.11, 28.77, 29.21, 29.47, 31.86, 34.61, 36.44, 43.80, 47.51, 51.08, 52.38, 107.49, 112.11, 172.90 MS (ES+), [M + H] ⁺ (100), 469.3 [M + Na] ⁺, 491.3 HRMS calculated for 469.3641 C₂₆H₄₉O₅N₂ found, 469.3659.

### 1-Morpholin-4-yl-2-(7,8,21,22-tetraoxa-dispiro[5.2.11.2]docos-3-yl)-ethanone 28h

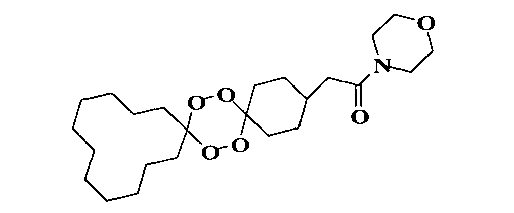

Mpt. 118-120°C *V*ₘₐₓ (CHCl₃)/cm⁻¹ 1437.7, 1632.5, 2858.9, 2931.3, 3003.7 ¹HNMR (400MHz, CDCl₃) δ_{H}, 1.15-1.49(m, 22H, CH₂), 1.50-1.84(m, 8H, CH₂),1.98(m, 1H, CH), 2.23(bs, 2H, CH₂), 3.45(m, 2H, morpholine), 3.65(m, 6H, morpholine), ¹³CNMR (100MHz, CDCl₃), δ_{C} 22.25, 22.72, 26.33, 26.39, 29.52, 31.61, 34.23, 39.23, 42.37, 46.60, 67.06, 67.37, 107.99, 112.79, 170.92 MS (ES+), [M + Na] ⁺ (100), 462.2 [2M + Na] ⁺, 901.4 HRMS calculated for 462.2832 C₂₄H₄₁O₆Na found, 462.2834.

### 2-(7,8,21,22-Tetraoxa-dispiro[5.2.11.2]docos-3-yl)-1-thiomorpholin-4-yl-ethanone 28i

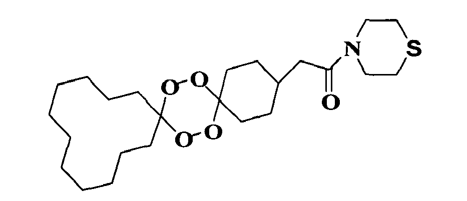

Melting pointt. 108-110°C *V*ₘₐₓ (CHCl₃)/cm⁻¹ 1169.6, 1182.1, 1290.0, 1422.8, 1443.6, 1464.3, 1638.7, 2854.8, 2921.2 ¹HNMR (400MHz, CDCl₃) δ_{H}, 1.29-1.50(m, 24H, CH₂), 1.51-1.66(m, 4H, CH₂), 1.71-1.81(m, m, 2H, CH₂), 1.91-2.03(m, 1H, CH), 2.22(bs, 2H, CH₂CO), 2.60(t, 4H, CH₂S), 3.74(2H, J = 4.36Hz, NCH₂), 3.89(bs, 2H, NCH₂) ¹³CNMR (100MHz, CDCl₃), δ_{C} 22.41, 22.73, 25.86, 26.33, 26.40, 27.86, 28.36, 29.67, 34.1839.57, 44.73, 48.84, 108.00, 112.79, 170.65. MS (ES+), [M + H] ⁺ (100), 478.2HRMS calculated for 478.2603 C₂₄H₄₁O₅Na found, 478.2605.

### Adamantyl- N-Cyclopropyl tetraoxane acetamide 29c

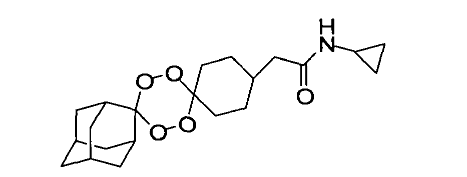

Mpt. 140-142°C *V*ₘₐₓ (CHCl₃)/cm⁻¹ 1496.6, 1664.2, 2858.9, 2922.3, 3012.8, 3320.8 ¹HNMR (400MHz, CDCl₃) δ_{H}, 0.48(m, 2H, cyclopropyl), 0.78(m, 2H, cyclopropyl), 1.14-1.38(m, 2H, CH₂), 1.40-1.80(m, 14H, CH₂),1.88(bs, 2H, CH₂CO), 1.83-2.05(m, 7H, CH/CH₂), 2.70(m, 1H, CH-cyclopropyl), 5.5(bs, 1H, NH), ¹³CNMR (100MHz, CDCl₃), δ_{C} 7.05, 8.89, 23.00, 27.47, 27.49, 33.54, 33.56, 34.46, 37.37, 39.48, 43.52, 108.09, 110.80, 173.53 MS (ES+), [M + Na] ⁺ (100), 400.2 [2M + Na]⁺, 777.4 HRMS calculated for 400.21 C₂₁H₃₁O₅NNa found, 400.2083

### N-(2-Pyrrolidin-1-yl-ethyl)-[adamantyl] acetamide 29d

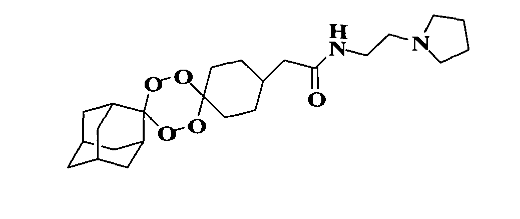

Mpt. 142-144°C *V*ₘₐₓ (CHCl₃)/cm⁻¹ 1446.7, 1559.9, 1641.1, 2859.1, 2931.2, 2937.7, 3260.8 ¹HNMR (400MHz, CDCl₃) δ_{H} 1.19-1.35(m, 2H, CH₂), 1.50-1.83(m, 14H, CH₂), 1.83-1.89(m, 4H, CH₂), 1.90-2.04(m, 5H, CH), 2.12(d, 2H, J = 7.02Hz, CH₂CO), 2.50-2.67(m, 6H, NCH₂/CH₂N), 3.31(q, 4H, J = 5.50Hz, CH₂), 6.55(bs, 1H, NH), ¹³CNMR (100MHz, CDCl₃), δ_{C} 23.81, 23.83, 27.47, 27.85, 28.61, 33.52, 33.53, 34.45, 36.69, 37.35, 37.95, 39.64, 39.80, 43.51, 47.36, 50.89, 54.29, 55.33, 55.57, 61.06, 108.12, 110.74, 172.53 MS (ES+), [M + Na] ⁺ (100), 457.2 [2M + Na] ⁺, 891.3 HRMS calculated for 457.2678 C₂₄H₃₈O₅N₂Na found, 457.268

### N-(2-Piperidin-1-yl-ethyl)-[adamantyl ]acetamide 29e

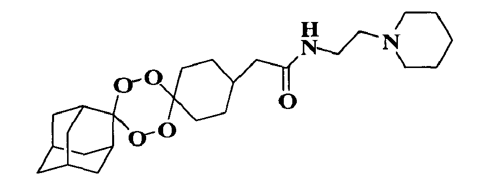

Mpt. 119-121°C *V*ₘₐₓ (CHCl₃)/cm⁻¹ 1446.7, 1541.3, 1650.3, 2794.9, 2846.8, 2919.4, 3324.1 ¹HNMR (400MHz, CDCl₃) δ_{H},) 1.22-1.41(m, 2H, CH₂), 1.45-1.79(m, 16H, CH₂), 1.86(bs, 2H, CH₂), 1.89-2.17(m, 9H, CH/CH₂), 2.24(d, 2H, J = 6.83Hz, CH₂CO), 3.10(t, 6H, J = 5.50Hz, CH₂N/NCH₂), 3.68(q, 2H, J = 5.31Hz, NHCH₂), 8.15(bs, 1H, NH), ¹³CNMR (100MHz, CDCl₃), δ_{C} 22.44, 22.94, 27.50, 33.55, 34.16, 34.48, 37.40, 43.05, 54.64, 58.15, 108.10, 110.70, 173.46 MS (ES+), [M + Na] ⁺ (100), 471.2 HRMS calculated for 471.2835 C₂₅H₄₀O₅N₂Na found, 471.2854

### N-(2-Morpholin-4-yl-ethyl)-adamantyl acetamide 29f

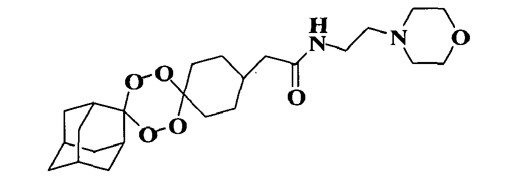

*V*ₘₐₓ (neat)/cm⁻¹ 1446.2, 1539.6, 1648.6, 2858.9, 2913.2, 2926.4, 3331.1 ¹HNMR (400MHz, CDCl₃) δ_{H}, 1.42-1.79(m, 14H, CH₂), 1.80, 1.99(m, 2H, CH₂), 1.99-2.20(m, 5H, CH), 2.30-2.07(m, 2H, CH₂), 2.09(d, 2H, J = 7.02Hz, CH₂CO), 3.28(q, 2H, J = 5.51Hz, CH₂N/NCH₂), 3.67-3.73(m, 4H, CH₂O), 6.0(bs, 1H, NH) ¹³CNMR (100MHz, CDCl₃), δ_{C} 27.45, 27.47, 33.53, 33.55, 34.46, 35.94, 37.35, 43.68, 53.74, 67.28, 108.08, 110.80, 172.23 MS (ES+), m/z 450.57 [M + Na] ⁺ (100), 473.2 [M + H/K] ⁺, 451.2/489.2 HRMS calculated for 473.2628 C₂₄H₃₈O₆N₂Na found, 473.2649

### N-(2-Diethylamino-ethyl)-[adamantly]acetamide 29g

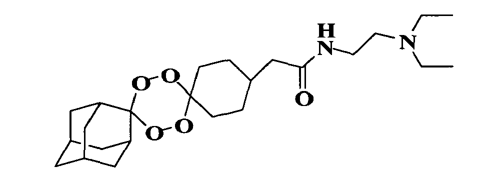

*V*ₘₐₓ (neat)/cm⁻¹ 1446.7, 1524.1, 1660.6, 2812.3, 2928.4, 2957.5, 3341.5 ¹HNMR (400MHz, CDCl₃) δ_{H}, 1.18(t, 6H, J = 7.21Hz, CH₃), 1.22-1.40(m, 2H, CH₂), 1.50-1.78(m, 14H, CH₂), 1.80-1.88(m, 2H, CH₂), 1.90-2.04(m, 5H, CH), 2.15(d, 2H, J = 7.02Hz, CH₂CO), 2.76-2.85(m, 6H, NCH₂/CH₂N), 3.45(m, 2H, NCH₂), 7.18(bs, 1H, NH) ¹³CNMR (100MHz, CDCl₃), δ_{C} 10.68, 27.47, 33.53, 34.43, 37.36, 43.39, 47.56, 50.99,52.39, 52.51, 108.10, 110.10, 172.86 MS (ES+), m/z 436.58 [M + H] ⁺ (100), 437.2 [M + Na] ⁺, 459.2 HRMS calculated for 437.3015 C₂₄H₄₁O₅N₂ found, 437.3035

### Adamantly-1-Morpholin-4-yl tetraoxane acetamide 29h

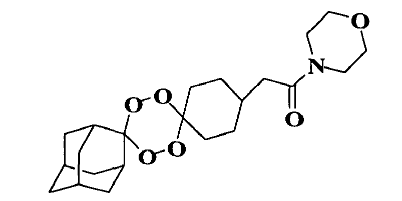

Mpt. 139-140°C *V*ₘₐₓ (CHCl₃)/cm⁻¹ 1442.3, 1632.5, 2858.9, 2913.2, 3003.8 ¹HNMR (400MHz, CDCl₃) δ_{H}, 1.11-1.38(m, 2H, CH₂), 1.50-1.82(m, 12H, CH₂), 1.85(bs, 2H, CH₂), 1.90-2.18(m, 5H, CH), 2.30(d, 2H, J = 7.02Hz, CH₂CO), 3.46(t, 2H, J = 4.56Hz, NCH₂), 3.60-3.69(m, 6H, NCH₂/CH₂O) ¹³CNMR (100MHz, CDCl₃), δ_{C} 26.52, 27.47, 27.49, 28.94, 30.69, 33.54, 33.56, 33.82, 34.25, 35.20, 37.37, 39.21, 42.37, 46.60, 67.07, 67.37, 108.10, 110.81, 170.92 MS (ES+), [M + Na] ⁺ (100), 430.2 [2M + Na] ⁺, 837.4 HRMS calculated for 430.2206 C₂₂H₃₃O₆NNa found, 430.2213

### Tetraoxa-dispiro-(adamantly)-thiomorpholin-4-yl-ethanone 29i

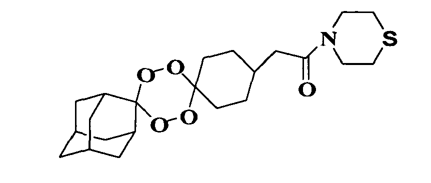

Melting point 150-152°C *V*ₘₐₓ (CHCl₃)/cm⁻¹ 955.9, 992.4, 1056.5, 1102.2, 1184.5, 1285.2, 1417.8, 1445.2, 1632.7, 2848.0, 2921.1 ¹HNMR (400MHz, CDCl₃) δ_{H}, 1.17-1.50(m, 16H, CH₂), 1.50-1.67(m, 4H, CH), 1.71-1.86(m, 2H, CH₂), 1.91-2.04(m, 1H, CH), 2.16-2.35(m, 2H, CH₂CO), 3.04(bs, 4H, CH₂S), 3.97(bs, 2H, NCH₂), 4.11(bs, 2H, NCH₂) ¹³CNMR (400MHz, CDCl₃), δ_{c} 14.60, 22.67, 26.30, 26.38, 34.05, 39.26, 40.65, 44.32, 52.55, 52.69, 107.83, 112.89, 170.83. MS (ES+), [M + Na] ⁺ (100), 446.0 HRMS calculated for 446.1977 C₂₂H₃₃O₅NSNa found, 446.1974.

### Adamantyl acetamide 29j

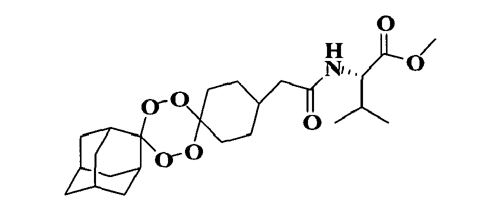

Mpt. 108-110°C *V*ₘₐₓ(CHCl₃)/cm⁻¹1536.3, 1650.3, 2859.1, 2919.4, 2931.2, 3376.0 ¹HNMR (400MHz, CDCl₃) δ_{H}, 0.87-0.99(m, 6H, CH₃), 1.25(t, 2H, J = 7.21Hz, CH₂), 1.49-1.80(m, 12H, CH₂), 1.86(bs, 2H, CH₂), 1.90-2.03(m, 5H, CH), 2.06-2.22(m, 5H, CH/CH₂), 3.76(s, 3H, OCH₃), 4.57(dd, 1H, J = 4.94Hz, CH), 5.92(d, 1H, J = 8.54Hz, NH), ¹³CNMR (400MHz, CDCl₃), δ_{C} 18.13, 18.21, 19.34, 27.48, 31.62, 33.55, 34.43, 37.38, 43.62, 52.50, 57.32, 108.07, 110.77, 172.10, 173.00 MS (ES+), [M + Na] ⁺ (100), 474.2 HRMS calculated for 474.2468 C₂₄H₃₇O₇NNa found, 474.2448.

### 1-(1,1-Dioxo-1λ⁶-thiomorpholin-4-yl)-2-(7,8,21,22-tetraoxa-dispiro[5.2.11.2]docos-3-yl)-ethanone 31

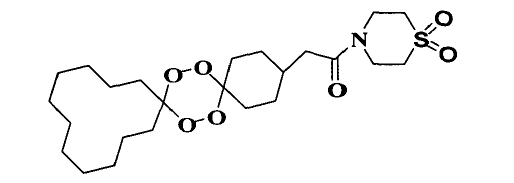

Melting point 170-172°C *V*ₘₐₓ (CHCl₃)/cm⁻¹ 859.8, 946.7, 1065.6, 1120.5, 1170.8, 1285.2, 1321.7, 1431.5, 1463.5, 1637.3, 2857.1, 2930.3, 3012.6 ¹HNMR (400MHz, CDCl₃) δ_{H}, 1.50-1.82(m, 24H, CH₂), 1.86(bs, 2H, CH₂), 1.91-2.04(m, 5H, CH₂/CH), 2.18-2.28(m, 2H, CH₂CO), 2.60(t, 4H, J = 4.93Hz, CH₂S), 3.73(t, 2H, J = 4.93Hz, NHCH₂), 3.89(bs, 2H, NCH₂) ¹³CNMR (100MHz, CDCl₃), δ_{C} 27.46, 27.46, 27.86, 28.36, 33.53, 33.55, 33.83, 36.77, 37.34, 39.55, 44.73, 48.85, 108.12, 110.83, 170.68 MS (ES+), [M + Na] ⁺ (100), 510.0 HRMS calculated for 510.2501 C₂₄H₄₁O₇NNa found, 510.2489.

### 1-(1,1-Dioxo-1λ⁶-thiomorpholin-4-yl)-2-tetraoxa-dispiro-adamantyl ethanone 32

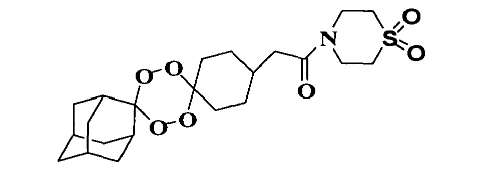

Melting point189-191°C *V*ₘₐₓ(CHCl₃)/cm⁻¹ 905.7, 1068.8, 1119.0, 1169.2, 1273.7, 1428.5, 1461.9, 1633.4, 2847.5, 2914.4 ¹HNMR (400MHz, CDCl₃) δ_{H} 1.20-1.37(m, 4H, adamantylidine), 1.50-1.82(m, 14H, CH₂), 1.90-2.05(m, 2H, CH₂CO), 3.03(q, 4H, J = 4.94Hz, CH₂SO₂), 4.97(t, 2H, J = 4.74Hz, NCH₂), 4.07-4.16(m, 2H, NCH₂) ¹³CNMR (100MHz, CDCl₃), δ_{C} 27.01, 27.03, 29.70, 33.12, 33.14, 33.66, 36.91, 38.85, 40.24, 43.91, 52.13, 52.28, 107.53, 110.54, 170.44 MS (ES+), [M + Na] ⁺ (100), 478.0 HRMS calculated for 478.1875 C₂₂H₃₃O₇NSNa found, 4578.1864.

### Tetraoxane 29k

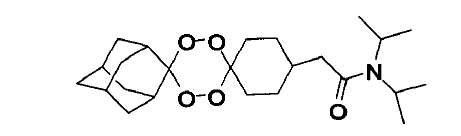

¹H NMR (400MHz, CDCl₃) δ_{H}, 1.19 (d, 6H, J = 6.5Hz, CH₃), 1.37 (d, 6H, J = 6.8Hz, CH₃), 1.22-1.30 (m, 4H, adamantylidene), 1.50-1.81 (m, 12H, adamantylidene/CH₂), 1.86 (bs, 2H, CH₂), 1.91-2.06 (m, 5H, adamantylidene/CH), 2.19 (d, 2H, J = 6.5Hz, CH₂CO), 3.89-4.01 (m, 2H, CH) ¹³C NMR (100MHz, CDCl₃), δ_{C} 21.2, 21.4, 21.5, 27.5, 33.6, 34.3, 37.4, 41.4, 46.1, 108.3, 110.8, 171.1. MS(ES+), [M+Na] ⁺ (100), 444.2 [2M + Na ] ⁺ 865.5 HRMS calculated for 444.2726 C₂₄H₃₉O₅NNa, found 444.2713.

### Tetraoxane 29l

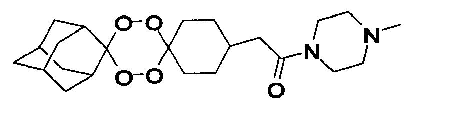

¹H NMR (400MHz, CDCl₃) δ_{H}, 1.10-1.30 (m, 4H, adamantylidene), 1.45-1.75 (m, 12H, adamantylidene/CH₂), 1.79 (bs, 2H, CH₂), 1.84-1.98 (m, 5H, adamantylidene/CH), 2.16 (bs, 2H, CH₂CO), 2.24 (s, 3H, CH₃), 2.27-2.34 (m, 4H, CH₂N), 3.38-3.45 (m, 2H, NCH₂), 3.54-3.60 (m, 2H, NCH₂) ¹³C NMR (100MHz, CDCl₃), δ_{C} 27.5, 33.6, 34.3, 37.4, 39.4, 41.9, 44.0, 46.2, 46.5, 55.1, 55.2, 55.6, 108.2, 110.8, 170.7. MS (ES+), [M + H ] ⁺ (100), 421.3 [M + Na ] ⁺ 443.1 HRMS calculated for 443.2522 C₂₃H₃₆O₅N₂Na, found 443.2526.

### Tetraoxane 29m

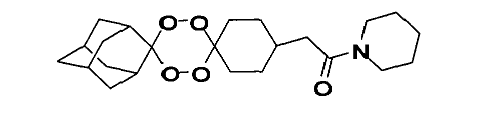

¹H NMR (400MHz, CDCl₃) δ_{H}, 1.20-1.35(m, 4H, adamantylidene), 1.49-1.80 (m, 18H, adamantylidene/CH₂), 1.86 (bs, 2H, CH₂), 1.91-2.03 (m, 5H, adamantylidene/CH/CH₂CO), 2.24 (d, 2H, J = 7.22 Hz, CH₂CO), 3.39 (t, 2H, J = 5.4 Hz, NCH₂), 3.56 (t, 2H, J = 5.4 Hz, NCH₂) ¹³C NMR (100MHz, CDCl₃), δ_{C} 25.0, 26.1, 27.0, 27.5, 33.6, 34.4, 37.4, 39.4, 43.1, 47.3, 108.2, 110.8, 170.5 MS (ES+), [M + Na ] ⁺ (100), 428.1 [2M + Na ] ⁺ 883.1 HRMS calculated for 428.2413 C₂₃H₃₅O₅NNa, found 428.2416.

### Tetraoxane 29n

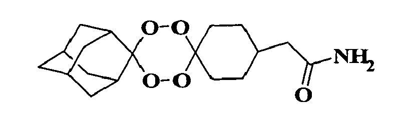

¹H NMR (400MHz, CDCl₃) δ_{H}, 1.20-1.36 (m, 4H, adamantylidene), 1.50-1.82 (m, 12H, adamantylidene/CH₂), 1.86(bs, 2H, CH2), 1.91-2.05 (m, 5H, adamantylidene), 2.29 (d, 2H, J = 6.8Hz, CH2CO), ¹³C NMR (100MHz, CDCl₃), δ_{C} 27.5, 30.1, 33.6, 34.0, 37.4, 40.5, 107.9, 110.9, 177.3 MS (ES+), [M + Na ] ⁺ (100), 360.0 HRMS calculated for 360.1787 C₁₈H₂₇O₅NNa, found 360.1776.

### Tetraoxane 29o

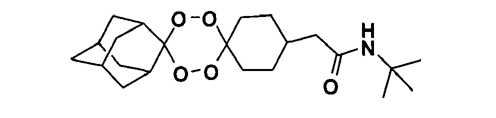

¹H NMR (400MHz, CDCl₃) δ_{H}, 1.20-1.31(m, 4H, adamantylidene), 1.34(s, 9H, CH₃) 1.50-1.78 (m, 13H, adamantylidene/CH₂), 1.86 bs, 2H, CH₂), 1.91-2.04 (m, 6H, adamantylidene/CH/CH₂CO), 5.20 (bs, 1H, NH) ¹³C NMR (100MHz, CDCl₃), δ_{C} 26.5, 27.5, 28.2, 29.3, 30.0, 33.5, 34.5, 37.4, 39.7, 44.7, 51.6, 108.2, 110.8, 171.5 MS (ES+), [M + Na ] ⁺ (100), 416.2 HRMS calculated for 416.2413 C₂₂H₃₅O₅NNa, found 416.2397.

### Tetraoxane 29p

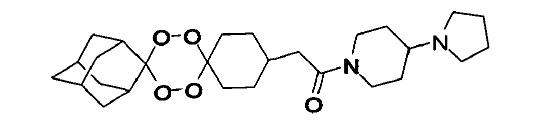

¹H NMR (400MHz, CDCl₃) δ_{H} 1.20-1.36 (m, 4H, adamantylidene), 1.42-1.80 (m, 14H, admantylidene/CH₂), 1.81-1.89 (m, 8H, CH₂), 1.90-2.04 (m, 5H, adamantylidene/CH), 2.24 (d, 2H, J = 7.22 Hz, CH₂CO), 2.62-2.73 (m, 4H, NCH₂), 3.05 (t, 1H, J = 11.6 Hz, NH), 3.85 (t, 2H, J = 13.5 Hz, NCH₂), 4.56 (t, 2H, J = 13.5 Hz, NCH₂) ¹³C NMR (100MHz, CDCl₃), δ_{C} 23.7, 27.5, 31.0, 32.0, 33.6, 34.4, 37.4, 39.4, 40.7, 44.8, 51.7, 62.1, 108.2, 110.8, 170.5 MS (ES+), [M + Na] ⁺ (100), 475.3 HRMS calculated for 475.3172 C₂₇H₄₃O₅N₂Na, found 475.3163.

### Tetraoxane 29q

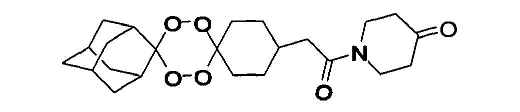

¹H NMR (400MHz, CDCl₃) δ_{H} 1.20-1.36 (m, 4H, adamantylidene), 1.52-1.83 (m, 12H, adamantylidene/CH₂), 1.86 (s, 2H, CH₂), 1.90-2.02 (m, 5H, adamantylidene/CH), 2.29-2.37 (m, 2H, CH₂CO), 2.48 (t, 4H, J = 6.0 Hz, CH₂CO), 3.76 (t, 2H, J = 6.3 Hz, NCH₂), 3.90 (t, 2H, J = 6.3 Hz, NCH₂) ¹³C NMR (100MHz, CDCl₃), δ_{C} 27.5, 33.5, 33.6, 34.2, 37.3, 39.5, 41.2, 41.7, 44.6, 108.1, 110.8, 171.1, 207.1 MS (ES+), [M + Na + CH₃OH] ⁺ (100), 474.2 [2M + Na + CH₃OH] ⁺ 925.5 HRMS calculated for 474.2468 C₂₄H₃₇O₇NNa, found 474.2480.

### Tetraoxane 29r

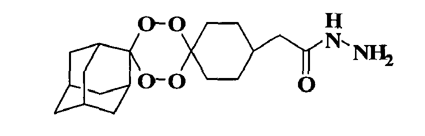

¹H NMR (400MHz, CDCl₃) δ_{H} 1.20-1.42 (m, 4H, adamantylidene), 1.50-1.82 (m, 12H, adamantylidene/CH₂), 1.86 (bs, 2H, CH₂), 1.90-2.05 (m, 5H, adamantylidene/CH), 2.64 (d, 2H, J = 6.8 Hz, CH₂CO), 6.81 (bs, 2H, NH₂), 8.46 (bs, 1H, NH) ¹³C NMR (100MHz, CDCl₃), δ_{C} 27.5, 28.2, 33.6, 34.3, 36.7, 38.0, 39.5, 41.4, 108.0, 110.9, 173.0 MS (ES+), [M + Na] ⁺ (100), 375.2 HRMS calculated for 375.1896 C₁₈H₂₈O₅N₂Na, found 375.1891.

### Tetraoxane 29s

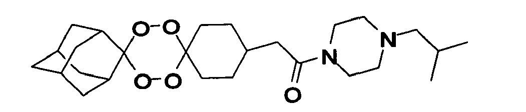

¹H NMR (400MHz, CDCl₃) δ_{H}, 0.89 (m, 6H, J = 6.46Hz, CH₃), 1.17-1.36 (m, 2H, adamantylidene), 1.50-1.84 (m, 14H, adamantylidene/CH₂), 1.86 bs, 2H, CH₂), 1.90-2.04 (m, 6H, CH), 2.08 (d, 2H, J = 7.40Hz, CH₂CO), 2.20-2.27 (m, 2H, NCH₂), 2.35 (t, 4H, CH₂N), 3.45(t, 2H, J = 4.74Hz, NCH₂), 3.62(t, 2H, J = 4.74Hz, NCH₂) ¹³C NMR (100MHz, CDCl₃), δ_{C} 21.2, 25.8, 27.4, 33.5, 34.4, 37.3, 39.4, 42.1, 46.3, 53.7, 54.2, 67.2, 108.2, 110.8, 170.7 MS (ES+), [M + Na] ⁺ (100), 463.3 HRMS calculated for 463.3172 C₂₆H₄₃O₅N₂Na, found 4632.3187.

### Tetraoxane 29t

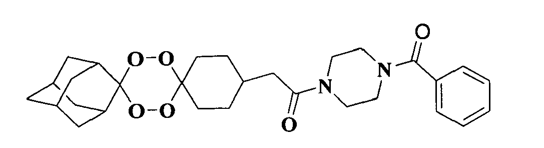

¹H NMR (400MHz, CDCl₃) δ_{H} 1.22-1.36 (m, 4H, adamantylidene), 1.50-1.82 (m, 13H, adamantylidene/CH₂), 1.86 (bs, 2H, CH₂), 1.91-2.04 (m, 5H, adamantylidene/CH), 2.26 (bs, 2h, CH2CO), 3.40-3.76 ( m, 8H, NCH₂/CH₂N), 7.38-7.46 (m, 5H, Ar) ¹³C NMR (100MHz, CDCl₃), δ_{C} 27.5, 33.5, 33.6, 34.2, 37.4, 39.5, 53.2, 108.1, 110.8, 127.5, 129.0, 130.4, 135.6, 171.0 MS (ES+), [M + Na] ⁺ (100), 533.1 HRMS calculated for 533.2628 C₂₉H₃₈O₆N₂Na, found 533.2653.

### Tetraoxane 29u

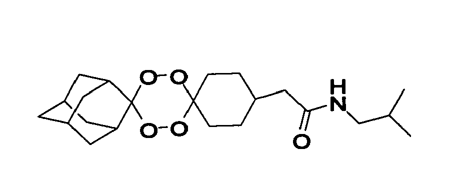

¹H NMR (400MHz, CDCl₃) δ_{H} 0.92 (d, 6H, J = 6.6 Hz, CH₃), 1.20-1.35 (m, 4H, adamantylidene), 1.50-1.82 (m, 13H, adamantylidene/CH₂), 1.86 (bs, 2H, CH₂), 1.90-2.04 (m, 5H, adamantylidene/CH), 2.09 (d, 2H, J = 7.22 Hz, CH₂CO), 3.09 (t, 2H, J = 6.2 Hz, NCH₂), 5.41 (s, 1H, NH) ¹³C NMR (100MHz, CDCl₃), δ_{C} 20.5, 27.5, 28.9, 33.5, 34.5, 37.4, 44.0, 47.2, 108.1, 110.8, 172.1 MS (ES+), [M + Na] ⁺ (100), 416.1, [2M + Na] ⁺ 809.2 HRMS calculated for 416.2413 C₂₂H₃₅O₅NNa, found 416.2392.

### Tetraoxane 29v

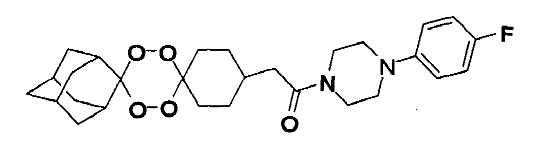

¹H NMR (400MHz, CDCl₃) δ_{H}, 1.22-1.36 (m, 4H, adamantylidene), 1.50-1.80 (m, 12H, adamantylidene/CH₂), 1.86 (bs, 2H, CH₂), 1.91-2.06 (m, 5H, adamantylidene/CH), 2.28 (d, 2H, J = 7.2 Hz, CH₂CO), 3.15 (t, 4H,J = 4.9 Hz, NCH₂), 3.63 (t, 4H, J = 4.9 Hz, CH₂N), 3.79 (t, 2H, J = 4.8 Hz, NCH₂), 6.88-6.95 (m, 2H, Ar), 7.25-7.31 (m, 2H, Ar) ¹³C NMR (100MHz, CDCl₃), δ_{C} 27.5, 33.6, 34.4, 37.4, 39.4, 42.0, 46.1, 49.9, 50.2, 108.1, 110.8, 117.0, 121.0, 130.0, 151.3, 171.0 MS (ES+), [M + Na] ⁺ (100), 523.3 [2M + Na] ⁺ 1024.6 HRMS calculated for 523.2584 C₂₈H₃₇O₅N₂Na, found 523.2568.

### Tetraoxane 29w

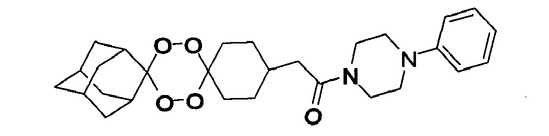

¹H NMR (400MHz, CDCl₃) δ_{H}, 1.22-1.35 (m, 4H, adamantylidene), 1.51-1.82 (m, 12H, adamantylidene/CH₂), 1.86 (bs, 2H, CH₂), 1.91-2.06 (m, 5H, adamantylidene/CH), 2.28 (d, 2H, J = 7.2 Hz, CH₂CO), 3.15 (t, 4H,J = 4.9Hz, NCH₂), 3.63 (t, 4H, J = 4.9 Hz, CH₂N), 3.77 (t, 2H, J = 4.8 Hz, NCH₂), 6.88-6.95 (m, 3H, Ar), 7.25-7.31(m, 2H, Ar) ¹³C NMR (100MHz, CDCl₃), δ_{C} 27.5, 33.6, 34.4, 37.4, 39.4, 42.0, 46.2, 49.9, 50.2, 108.1, 110.8, 117.0, 121.0, 129.6, 151.3, 170.8 MS (ES+), [M + Na ] ⁺ (100), 483.2 HRMS calculated for 483.2859 C₂₈H₃₉O₅N₂Na, found 483.2881.

### Tetraoxane 29x

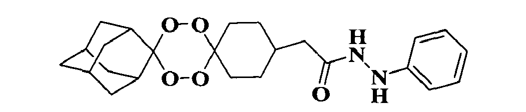

¹H NMR (400MHz, CDCl₃) δ_{H} 1.21-1.39 (m, 4H, adamantylidene), 1.51-1.81 (m, 12H, adamantylidene/CH₂), 1.86 (bs, 2H, CH₂), 1.91-2.06 (m, 5H, adamantylidene/CH), 2.16 (d, 2H, J = 7.21 Hz, CH₂CO), 6.60 (s, 1H, NH), 6.75-6.84 (m, 1H, Ar), 6.90 (t, 2H, J = 6.5 Hz, Ar), 7.19-7.19 (m, 2H, Ar), 8.60 (bs, 1H, NH) ¹³C NMR (100MHz, CDCl₃), δ_{C} 27.0, 33.1, 33.2, 33.9, 36.9, 40.9, 107.6, 110.5, 113.6, 121.5, 129.4, 147.9, 171.9 MS (ES+), [M + Na] ⁺ (100), 451.3 [2M + Na] ⁺ 879.6 HRMS calculated for 451.2209 C₂₄H₃₂O₅N₂Na, found 451.2213.

### Diethyl methylsulfonylmethylphosphonate 33b

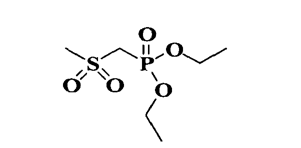

¹H NMR (400MHz, CDCl₃) δ_{H} 1.38 (t, 6H, J = 7.0 Hz, CH₃), 3.21 (s, 3H, SO₂CH₃), 3.60 (s, 1H, SO₂CH₂), 3.64 (s, 1H, SO₂CH₂), 4.21-4.29 (m, 4H, OCH₂). ¹³C NMR (100MHz, CDCl₃), δ_{C} 16.3, 42.7, 51.6, 53.0, 63.8 MS (CI+), [M + NH₄] ⁺ (100), 248 HRMS calculated for 231.0456 C₆H₁₆O₅SP, found 231.0453

### 8-(methylsulfonylmethylene)-1,4-dioxaspiro[4.5]decane 33c

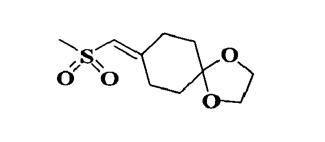

¹H NMR (400MHz, CDCl₃) δ_{H} 1.81 (t, 4H, J = 6.5 Hz, CH₂), 2.42 (t, 4H, J = 6.5 Hz, CH₂), 2.96 (s, 3H, SO₂CH₃), 3.98 (s, 4H, OCH₂), 6.17 (s, 1H, CH) ¹³C NMR (100MHz, CDCl₃), δ_{C} 26.1, 35.9, 44.6, 65.0, 107.6, 124.3, 159.6 MS (ES+), [M + Na] ⁺ (100), 255.1 [2M + Na] ⁺ 487.2 HRMS calculated for 255.0667 C₁₀H₁₆O₄SNa, found 255.0648.

### 8-(methylsulfonylmethyl)-1,4-dioxaspiro[4.5]decane 33d

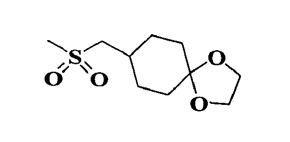

¹H NMR (400MHz, CDCl₃) δ_{H} 1.41-1.79 (m, 5H, cyclohexyl), 1.94-2.18 (m, 4H, cyclohexyl), 2.92 (s, 3H, SO₂CH₃), 2.96 (d, 2H, J = 6.5 Hz, CH₂SO₂), 3.95 (s, 4H, OCH₂) ¹³C NMR (100MHz, CDCl₃), δ_{C} 28.5, 29.5, 40.4, 58.6, 62.6, 106.2 MS (ES+), [M + Na] ⁺ (100), 257.1 HRMS calculated for 257.0824 C₁₀H₁₈O₄SNa, found 257.0836.

### Tetraoxane 33f

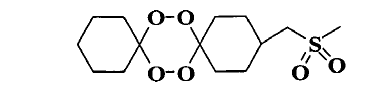

¹H NMR (400MHz, CDCl₃) δ_{H} 1.36-1.50 (m 4H, CH₂), 1.51-1.68 (m, 10H, CH₂), 1.71-2.34 (m, 5H, CH₂/CH), 2.92 (s, 3H, SO₂CH₃), 2.95 (d, 2H, CH₂SO₂) ¹³C NMR (100MHz, CDCl₃), δ_{C} 23.8, 26.2, 30.0, 30.2, 32.3, 35.5, 43.0, 60.9, 107.9, 109.4 MS (ES+), [M + Na] ⁺ (100), 343.1 HRMS calculated for 343.1191 C₁₄H₂₄O₆SNa, found 343.1198.

### Tetraoxane 33g

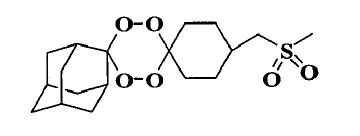

¹H NMR (400MHz, CDCl₃) δ_{H} 1.06-1.38 (m, 4H, adamantylidene), 1.41-1.78 (m, 12H, adamantylidene/CH₂), 1.87 (bs, 2H, CH₂), 1.91-2.06 (m, 5H, adamantylidene/CH), 2.92 (s, 3H, SO₂CH₃), 2.95 (d, 2H, J = 5.9 Hz, CH₂SO₂) ¹³C NMR (100MHz, CDCl₃), δ_{C} 26.1, 27.5, 29.0, 31.8, 33.1, 33.5, 37.3, 42.5, 60.5, 107.3, 111.0 MS (ES+), [M + Na] ⁺ (100), 395.2 HRMS calculated for 395.1504 C₁₈H₂₈O₆SNa, found 395.1482.

### 1-benzoylpiperidin-4-one 34b

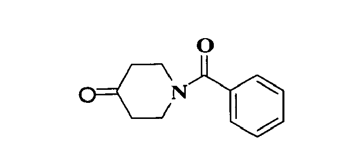

νₘₐₓ (neat)/cm⁻¹ 3089.1, 2958.5, 2877.0, 1714.0, 1632.5, 1433.2, 1365.3, 1315.5, 1274.7, 1243.0, 1143.4, 708.7 ¹H NMR (400MHz, CDCl₃) δ_{H} 2.50 (bs, 4H, CH₂CO), 3.89 (bs, 4H, NCH₂), 7.41-7.49 (m, 5H, Ar) ¹³C NMR (100MHz, CDCl₃), δ_{C} 41.6, 42.4, 127.4, 129.0, 130.6, 135.6, 171.3, 207.0

### 1-(pyrrolidin-1-carbonyl)piperidin-4-one 34c

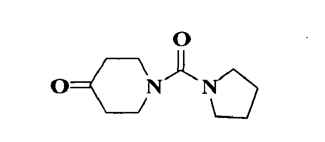

νₘₐₓ (neat)/cm⁻¹ 2963.9, 2871.3, 1714.6, 1629.8, 1467.8, 1421.6, 1340.6, 1228.8, 1190.2, 1132.4, 750.6 ¹H NMR (400MHz, CDCl₃) δ_{H} 1.85-1.90 (m, 4H, CH₂), 2.49 (t, 4H, J = 6.3 Hz, CH₂CO), 3.43 (t, 4H, J = 6.7Hz, NCH₂), 3.58 (t, 4H, J = 6.3 Hz, CH₂N) ¹³C NMR (100MHz, CDCl₃), δ_{C} 25.9, 41.8, 46.0, 48.3, 48.9, 162.6, 208.6

### N,N-diethyl-4-oxopiperidine-1-carboxamide 34d

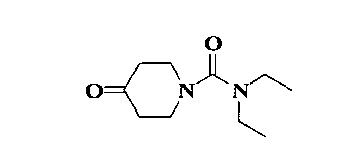

νₘₐₓ (neat)/cm⁻¹ 2965.8, 2928.3, 2872.1, 1714.8, 1639.8, 1419.7, 1358.8, 1260.5, 1166.8, 1101.2, 979.4, 773.4 ¹H NMR (400MHz, CDCl₃) δ_{H} 1.16 (t, 6H, J = 7.0 Hz, CH₃), 2.49 (t, 4H, J = 6.3 Hz, CH₂CO), 3.29 (q, 4H, J = 7.0 Hz, NCH₂), 3.50 (t, 4H, J = 6.1 Hz, CH₂N) ¹³C NMR (100MHz, CDCl₃), δ_{C} 13.3, 41.4, 42.0, 46.7, 164.0, 208.1 MS (CI+), [M + H] ⁺ (100), 199 HRMS calculated for 199.1447 C₁₀H₁₉O₂N₂, found 199.1452

### 1-(piperidine-1-carbonyl)piperidin-4-one 34e

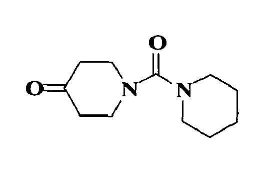

¹H NMR (400MHz, CDCl₃) δ_{H} 1.50-1.68 (m, 6H, CH₂), 2.48 (t, 4H, J = 6.3 Hz, CH₂CO), 3.27 (t, 4H, J = 5.7 Hz, NCH₂), 3.52 (t, 4H, J = 6.1Hz, NCH₂) ¹³C NMR (100MHz, CDCl₃), δ_{C} 25.1, 26.1, 41.8, 46.9, 48.3, 164.2, 208.4

### 1-(Morpholine-4-carbonyl)-piperidin-4-one 34f

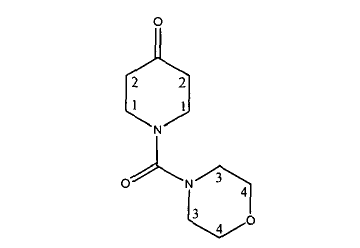

δ_{H} (400 MHz, CDCl₃), 3.75 (4H, m, 4H1), 3.60 (4H, t, *J* 6.3, 4H3), 3.31 (4H, m, 4H2), 2.50 (4H, t, *J* 6.2, 4H4); δ_{C} (100MHz, CDCl₃), 207.8, 163.8, 67.0, 47.8, 46.7, 41.6; m/z (CI, +ve, NH₃), 213 ([M+H]⁺, 100%); Found [M+H]⁺, 213.12448, C₁₀H₁₇N₂O₃ requires 213.12392.

### 4-oxo-N,N-diphenylpiperidine-1-carboxamide 34g

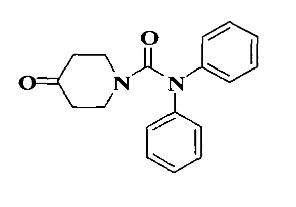

νₘₐₓ (neat)/cm⁻¹ 3013.4, 2965.8, 2861.1, 1711.9, 1650.0, 1588.2, 1493.0, 1412.1, 1264.5, 1212.2, 755.3 ¹H NMR (400MHz, CDCl₃) δ_{H} 2.3 (t, 4H, J = 6.3 Hz, CH₂CO), 3.63 (t, 4H, J = 6.3 Hz, CH₂N), 7.09 (d, 2H, J = 7.4 Hz, Ar), 7.15 (t, 4H, 7.4 Hz, Ar), 7.32 (t, 4H, 7.4 Hz, Ar) ¹³C NMR (100MHz, CDCl₃), δ_{C} 41,1, 45.1, 125.7, 129.8, 160.3, 207.5, MS (ES+), [M + Na + CH₃OH] ⁺ (100), 349.1 HRMS calculated for 349.15289 C₁₉H₂₂O₃N₂Na, found 349.1513.

### 1-(4-trifluoromethyl)benzoyl)piperidin-4-one 34h

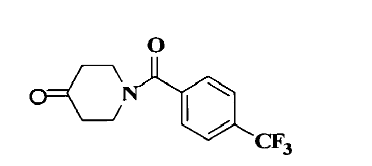

νₘₐₓ (neat)/cm⁻¹ 2964.1, 2908.5, 2871.4, 1712.2, 1638.0, 1512.8, 1438.6, 1322.7, 1123.3, 1016.6, 974.9, 849.7 ¹H NMR (400MHz, CDCl₃) δ_{H} 2.53 (bs, 4H, CH₂CO), 3.60-4.14 (m, 4H, CH₂N), 7.59 (d, 2H, J = 8.0 Hz, Ar), 7.72 (d, 2H, J = 8.0 Hz, Ar) ¹³C NMR (100MHz, CDCl₃), δ_{C} 41.4, 46.5, 125.4, 126.2, 126.2, 127.7, 139.1, 169.8, 206.3 MS (ES+), [M + Na + CH₃OH] ⁺ (100), 326.1 HRMS calculated for 326.0980 C₁₄H₁₆O₃NF₃, found 326.0982.

### (4,4-dihydroperoxypiperidin-1-yl)(piperidin-1-yl)methanone 34i

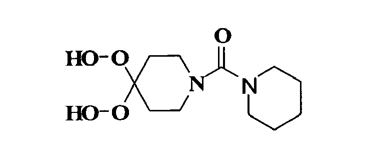

¹H NMR (400MHz, CDCl₃) δ_{H} 1.42-1.59 (m, 6H, CH₂), 1.74 (t, 4H, J = 5.7Hz, CH₂), 3.01-3.15 (m, 4H, NCH₂), 3.28-3.35 (m, 4H, NCH₂), 11.13 (s, 2H, OH), ¹³C NMR (100MHz, CDCl₃), δ_{C} 24.6, 25.7, 29.9, 43.7, 47.6, 107.1, 163.4 MS (ES+), [M + Na ] ⁺ (100), 283.1 [2M + Na ] ⁺ 543.1 HRMS calculated for 283.1270 C₁₁H₂₀O₅N₂Na, found 283.1282.

### Tetraoxane 34j

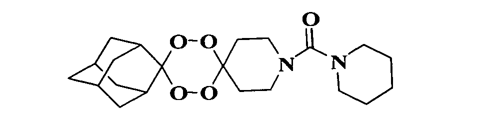

¹H NMR (400MHz, CDCl₃) δ_{H} 1.50-1.90 (m, 14H, adamantylidene/CH₂), 1.91-2.05 (m, 8H, CH₂), 3.15-3.22 (m, 4H, CH₂N), 3.26-3.37 (m, 4H, NCH₂) ¹³C NMR (100MHz, CDCl₃), δ_{C} 25.1, 26.1, 27.7, 33.5, 34.2, 36.2, 36.7, 37.3, 39.7, 107.1, 111.1, 164.4 MS (ES+), [M + Na ] ⁺ (100), 415.2 [2M + Na ] ⁺ 807.5 HRMS calculated for 415.2209 C₂₁H₃₂O₅N₂Na, found 415.2209

### Tetraoxane 34k

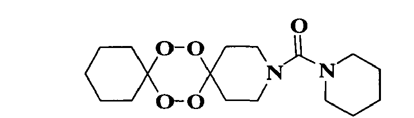

¹H NMR (400MHz, CDCl₃) δ_{H} 1.44-1.51 (m, 4H, CH2), 1.52-1.65 (m, 12H, CH₂), 2.15-2.51 (m, 4H, CH₂), 3.17-3.21 (m, 4H, CH₂N), 3.26-3.33 (m, 4H, NCH₂) ¹³C NMR (100MHz, CDCl₃), δ_{C} 22.4, 25.1, 25.7, 26.1, 30.1, 32.1, 48.3, 107.2, 109.0, 164.4 MS (ES+), [M + Na ] ⁺ (100), 363.2 [2M + Na ] 703.4 HRMS calculated for 363.1896 C₁₇H₂₈O₅N₂Na, found 363.1879.

### Tetraoxane 34l

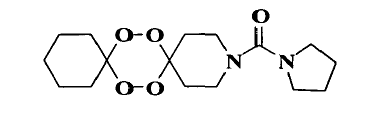

¹H NMR (400MHz, CDCl₃) δ_{H} 1.36-1.51 (m, 2H, CH₂), 1.54-1.75 (m, 12H, CH₂), 1.79-1.88 (m, 4H, CH₂), 2.20-2.50 (m, 4H, NHCH₂), 3.36 (t, 4H, J = 6.7 Hz, CH₂N) ¹³C NMR (100MHz, CDCl₃), δ_{C} 21.3, 24.7, 25.7, 28.7, 34.0, 48.8, 107.2, 109.0, 162.9 MS (ES+), [M + Na ] ⁺ (100), 349.1 [2M + Na ] ⁺ 675.2 HRMS calculated for 349.1739 C₁₆H₂₆O₅N₂Na, found 349.1737.

### Tetraoxane 34m

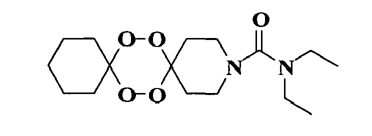

¹H NMR (400MHz, CDCl₃) δ_{H}, 1.12 (t, 6H, J = 7.2 Hz, CH₃), 1.43-1.81 (m, 6H, cyclohexyl), 2.20-2.51 (m, 4H, cyclohexyl), 3.20 (q, 4H, J = 7.0Hz, NCH₂), 3.28 (bs, 4H, NCH₂), ¹³C NMR (100MHz, CDCl₃), δ_{C} 13.6, 22.4, 25.7, 30.1, 31.8, 42.3, 44.4, 107.2, 109.0, 164.6 MS (ES+), [M + Na ] ⁺ (100), 351.1 [2M + Na ] ⁺ 679.3 HRMS calculated for 351.1896 C₁₆H₂₈O₅N₂Na, found 351.1899.

### Tetraoxane 34n

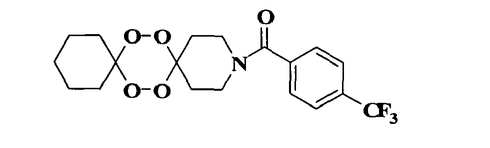

¹H NMR (400MHz, CDCl₃) δ_{H} 1.13-1.19 (m, 10H, CH₂), 2.14-2.66 (m, 4H, CH₂), 3.44 (bs, 2H, CH₂N), 3.82 (bs, 2H, CH₂N), 7.52 (d, 2H, J = 8.1 Hz, Ar), 7.69 (d, 2H, J = 8.1 Hz, Ar) ₁₃C NMR (100MHz, CDCl₃), δ_{C} 22.4, 25.7, 29.9, 30.6, 44.8, 106.6, 109.4, 132.4, 125.4, 127.7, 126.1, 139.6, 169.3 MS (ES+), [M + Na ] ⁺ (100), 424.1 [2M + Na ] ⁺ 825.2 HRMS calculated for 424.1348 C₁₉H₂₂O₅NNa, found 424.1364.

### Tetraoxane 34o

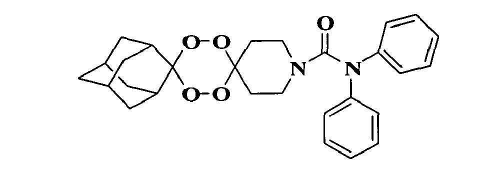

¹H NMR (400MHz, CDCl₃) δ_{H} 1.56-1.76 (m, 4H, adamantylidene) 1.83-2.24 (m, 19H, adamantylidene/CH₂), 3.45(t, 4H, J = 5.9Hz, CH₂N), 7.04 (d, 2H, J = 7.4Hz, Ar), 7.13 (t, 1H, J = 7.4Hz, Ar), 7.30 (t, 2H, J = 7.4Hz, Ar) ¹³C NMR (100MHz, CDCl₃), δ_{C} 27.4, 27.9, 31.38, 33.5, 36.7, 37.3, 39.7, 106.7, 111.2, 125.3, 125.5, 129.7, 145.3, 160.1 MS (ES+), [M + Na ] ⁺ (100), 499.2 HRMS calculated for 499.2209 C₂₈H₃₂O₅N₂Na, found 499.2206.

### Tetraoxane 34p

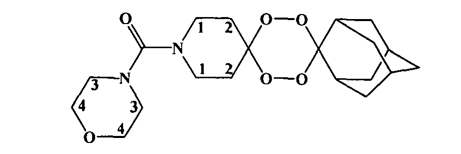

δ_{H} (400 MHz, CDCl₃), 3.70 (4H, t, J4.3, 4H3), 3.30 (4H, m, 4H1), 3.20 (4H, t, J 4.5, 4H4), 2.50 (2H, bs, 2H2a), 1.50-2.05 (16H, m, 2H2b and adamantane); δ_{C} (100MHz, CDCl₃), 163.9, 111.2, 106.8, 67.0, 47.8, 42.5, 37.3, 33.5, 31.8, 30.6, 27.4; m/z (ES, +ve, CH₃OH), 417 ([M+Na]⁺, 100%); Found [M+Na]⁺, 417.1982, C₂₀H₃₀N₂O₆Na requires 417.2002.

### Tetraoxane 35d

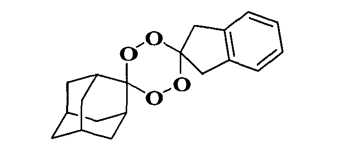

¹H NMR (400MHz, CDCl₃) δ_{H} 1.55-2.21 (m, 14H, adamantylidene), 3.07 (bs, 2H, CH₂), 3.81(bs, 2H, CH₂), 7.17 (bs, 4H, Ar) ¹³C NMR (100MHz, CDCl₃), δ_{C} 27.5, 32.7, 33.6, 37.5, 41.1, 110.2, 111.1, 125.2, 127.2, 139.5 MS (ES+), [M + Na ] ⁺ (100), 337.1[2M + Na ] ⁺ 651.2 HRMS calculated for 337.1416 C₁₉H₂₂O₄Na, found 337.1416.

### Tetraoxane 36c

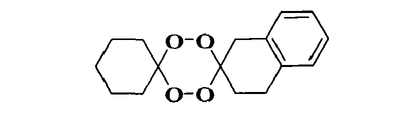

¹H NMR (400MHz, CDCl₃) δ_{H} 1.34-1.66 (m, 6H, cyclohexyl), 1.70-1.98 (m, 4H, cyclohexyl), 2.19-2.46 (m, 2H, CH₂), 2.76-3.04 (m, 4H, CH2), 7.11 (bs, 4H, Ar) ¹³C NMR (100MHz, CDCl₃), δ_{C} 22.5, 23.1, 25.8, 27.9, 31.9, 35.8, 108.2, 109.0, 126.2, 129.3, 133.9, 136.6 MS (ES+), [M + Na ] ⁺ (100), 299.1 [2M + Na ] ⁺ 575.2 HRMS calculated for 299.1259 C₁₆H₂₀O₄Na, found 199.1271.

### Tetraoxane 36d

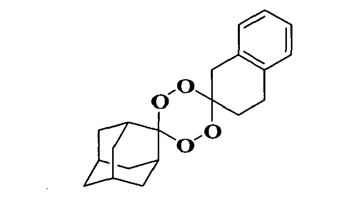

¹H NMR (400MHz, CDCl₃) δ_{H} 1.20-1.34 (m, 4H, adamantylidene), 1.55-1.80 (m, 6H, adamantylidene), 1.86 (bs, 2H, CH₂), 1.91-2.07 (m, 4H, CH), 2.55-2.35(m, 4H, CH₂), 7.14 (bs, 4H, Ar) ¹³C NMR (100MHz, CDCl₃), δ_{C} 23.1, 27.5, 32.0, 33.6, 37.4, 108.8, 110.9, 128.7, 129.3, 133.7, 136.4 MS (ES+), [M+Na] ⁺ (100), 351.1 [2M + Na ] ⁺ 679.3 HRMS calculated for 351.1572 C₂₀H₂₄O₄Na, found 351.1567.

### 1-methanesulfonyl-piperidin-4-one 38a

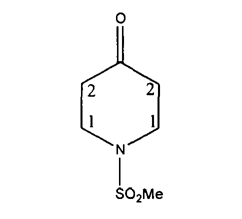

δ_{H} (400 MHz, CDCl₃), 3.60 (4H, m, 4H1), 2.91 (3H, s, C*H*₃), 2.55 (4H, m, 4H2); δ_{C} (100MHz, CDCl₃), 205.8, 46.0, 41.5, 37.2; m/z (CI, +ve, NH₃), 195 ([M+NH₃]⁺, 100%); Found [M+NH₃]⁺, 195.08052, C₆H₁₅N₂O₃S requires 195.08035.

### 1-ethanesulfonyl-piperidin-4-one 38b

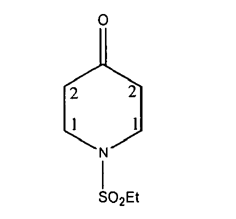

δ_{H} (400 MHz, CDCl₃), 3.71 (4H, m, 4H1), 3.10 (2H, q, *J* 7.4, C*H*₂), 2.62 (4H, m, 4H2), 1.45 (3H, t, *J* 7.4, C*H*₃); δ_{C} (100MHz, CDCl₃), 206.3, 46.2, 46.1, 42.2, 8.4; m/z (CI, +ve, NH₃), 209 ([M+NH₃]⁺, 100%); Found [M+NH₃]⁺, 209.09640, C₇H₁₇N₂O₃S requires 209.09599.

### 1-(propane-2-sulfonyl)-piperidin-4-one 38c

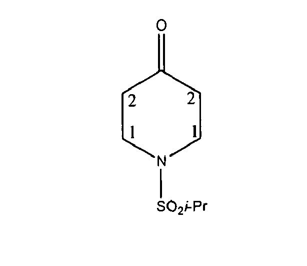

δ_{H} (400 MHz, CDCl₃), 3.68 (4H, m, 4H1), 3.25 (1H, m, C*H*(CH₃)₂), 2.55 (4H, m, 4H2), 1.35 (6H, d, *J* 6.9, (*CH*₃)₂); δ_{C} (100MHz, CDCl₃), 206.7, 54.6, 46.6, 42.7, 17.7.

### 1-cyclopropylsulfonyl-piperidin-4-one 38d

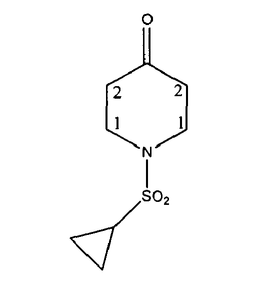

δ_{H} (400 MHz, CDCl₃), 3.65 (4H, m, 4H1), 2.55 (4H, m, 4H2), 2.31 (1H, m, CH₂C*H*CH₂), 1.20 (2H, m, C*H*₂CHCH₂), 1.05 (2H, m, CH₂CHC*H*₂); δ_{C} (100MHz, CDCl₃), 206.1, 46.3, 41.7, 27.7, 5.1; m/z (CI, +ve, NH₃), 221 ([M+NH₃]⁺, 100%); Found [M+NH₃]⁺, 221.09643, C₈H₁₇N₂O₃S requires 221.09599.

### 1-(2,2,2-trifluoroethanesulfonyl)-piperidin-4-one 38e

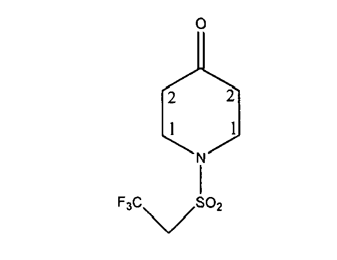

δ_{H} (400 MHz, CDCl₃), 3.94 (2H, m, C*H*₂CF₃), 3.71 (4H, m, 4H1), 2.59 (4H, m, 4H2); δ_{C} (100MHz, CDCl₃), 205.1, 122.2, 54.4, 54.1, 45.7, 41.9; m/z (ES, -ve, CH₃OH), 244 ([M-H]⁻, 100%); Found [M-H]⁻, 244.0255, C₈H₉NO₃F₃S requires 244.0246.

### 1-benzenesulfonyl-piperidin-4-one 38f

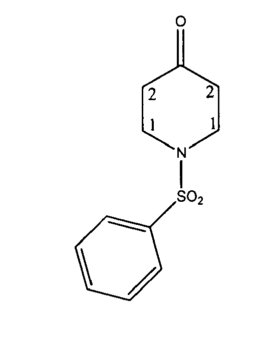

δ_{H} (400 MHz, CDCl₃), 7.84-7.51 (5H, m, aromatic), 3.45 (4H, m, 4H1), 2.50 (4H, m, 4H2); δ_{C} (100MHz, CDCl₃), 205.8, 133.6, 129.7, 127.9, 127.4, 46.3, 41.1; m/z (CI, +ve, NH₃), 257 ([M+NH₃]⁺, 100%); Found [M+NH₃]⁺, 257.09645, C₁₁H₁₇N₂O₃S requires 257.09598.

### 1-(4-fluoro-benzenesulfonyl)-piperidin-4-one 38g

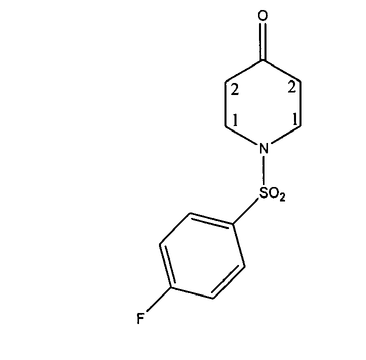

δ_{H} (400 MHz, CDCl₃), 7.90-7.15 (4H, m, aromatic), 3.40 (4H, m, 4H1), 2.55 (4H, m, 4H2); δ_{C} (100MHz, CDCl₃), 205.5, 130.6, 130.5, 117.1, 116.9, 46.2, 41.1; m/z (CI, +ve, NH₃), 275 ([M+NH₃]⁺, 100%); Found [M+NH₃]⁺, 275.08711, C₁₁H₁₆FN₂O₃S requires 275.08655.

### 1-(4-chloro-benzenesulfonyl)-piperidin-4-one 38h

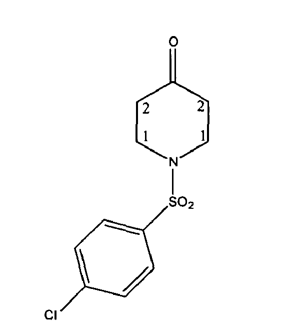

δ_{H} (400 MHz, CDCl₃), 7.80-7.45 (4H, m, aromatic), 3.40 (4H, m, 4H1), 2.55 (4H, m, 4H2); δ_{C} (100MHz, CDCl₃), 205.4, 140.3, 135.6, 130.1, 129.3, 46.2, 41.1; m/z (CI, +ve, NH₃), 291 ([M+NH_{3]}⁺, 100%); Found [M+NH₃]⁺, 291.05742, C₁₁H₁₆ClN₂O₃S requires 291.05704.

### 1-(4-trifluoromethyl-benzenesulfonyl)-piperidin-4-one 38i

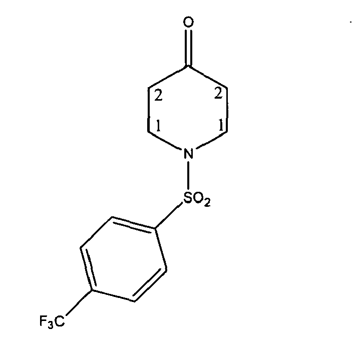

δ_{H} (400 MHz, CDCl₃), 8.25-7.70 (4H, m, aromatic), 3.45 (4H, m, 4H1), 2.55 (4H, m, 4H2); δ_{C} (100MHz, CDCl₃), 205.1, 128.4, 128.0, 127.4, 127.3, 126.9, 46.2, 41.1; m/z (CI, +ve, NH₃), 325 ([M+NH₃]⁺, 100%); Found [M+NH₃]⁺, 325.08377, C₁₂H₁₆F₃N₂O₃S requires 325.08337.

### 1,2,4,5-tetraoxane 39a

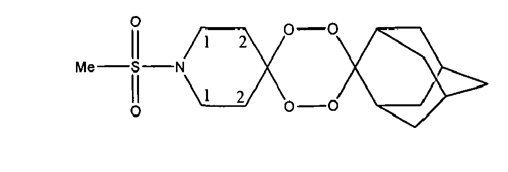

δ_{H} (400 MHz, CDCl₃), 3.22-3.45 (4H, m, 4H1), 2.80 (3H, s, C*H*₃), 2.52 (2H, s, 2H2a), 1.51-2.23 (16H, m, 2H2b and adamantane); δ_{C} (100MHz, CDCl₃), 111.6, 105.8, 41.6, 37.2, 36.2, 34.2, 33.5, 31.4, 27.4, 26.2. m/z (ES, +ve, CH₃OH), 382 ([M+Na]⁺, 100%); Found [M+Na]⁺, 382.1313, C₁₆H₂₅NO₆NaS requires 382.1300.

### 1,2,4,5-tetraoxane 39b

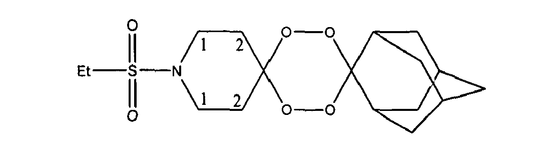

δ_{H} (400 MHz, CDCl₃), 3.22-3.45 (4H, m, 4H1), 2.95 (2H, q, *J* 7.4, C*H*₂CH₃), 2.50 (2H, s, 2H2a), 1.51-2.23 (16H, m, 2H2b and adamantane), 1.35 (3H, t, *J* 7.4, CH₂C*H*₃); δ_{C} (100MHz, CDCl₃), 111.5, 106.0, 44.9, 37.2, 36.2, 33.5, 31.35, 30.6, 27.4, 8.3. m/z (ES, +ve, CH₃OH), 396 ([M+Na]⁺, 100%); Found [M+Na]⁺, 396.1447, C₁₇H₂₇NO₆NaS requires 396.1457.

### 1,2,4,5-tetraoxane 39c

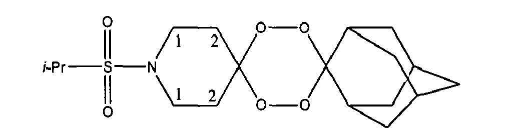

δ_{H} (400 MHz, CDCl₃), 3.22-3.35 (4H, m, 4H1), 3.15 (1H, m, CH₃C*H*CH₃), 2.50 (2H, s, 2H2a), 1.51-2.10 (16H, m, 2H2b and adamantane), 1.31 (6H, d, *J* 6.9, C*H*₃CHC*H*₃); δ_{C} (100MHz, CDCl₃), 111.5, 106.0, 54.1, 44.9, 37.3, 33.5, 32.0, 27.4, 23.0, 17.1, 14.5. m/z (ES, +ve, CH₃OH), 410 ([M+Na]⁺, 100%); Found [M+Na]⁺, 410.1600, C₁₈H₂₉NO₆NaS requires 410.1613.

### 1,2,4,5-tetraoxane 39d

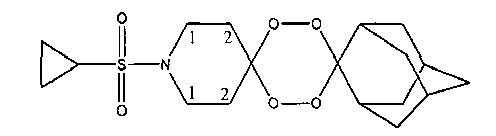

δ_{H} (400 MHz, CDCl₃), 3.30-3.50 (4H, m, 4H1), 2.50 (2H, s, 2H2a), 2.20 (1H, m, CH₂C*H*CH₂), 1.51-2.10 (16H, m, 2H2b and adamantane), 1.15 (2H, m, C*H*₂CHCH₂), 0.95 (2H, m, CH₂CHC*H*₂); δ_{C} (100MHz, CDCl₃), 111.5, 106.0, 54.1, 44.9, 37.3, 33.5, 32.0, 27.4, 26.5, 4.8. m/z (ES, +ve, CH₃OH), 408 ([M+Na]⁺, 100%); Found [M+Na]⁺, 408.1438, C₁₈H₂₇NO₆NaS requires 408.1457.

### 1,2,4,5-tetraoxane 39e

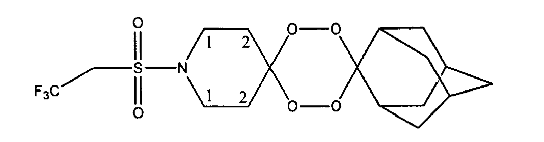

δ_{H} (400 MHz, CDCl₃), 3.70 (2H, q, *J* 9.3, C*H*₂CF₃), 3.30-3.60 (4H, m, 4H1), 2.50 (2H, s, 2H2a), 1.51-2.23 (16H, m, 2H2b and adamantane); δ_{C} (100MHz, CDCl₃), 110.2, 104.3, 52.2, 51.9, 35.9, 32.1, 31.35, 30.6, 27.4, -1.0. m/z (ES, +ve, CH₃OH), 450 ([M+Na]⁺, 100%); Found [M+Na]⁺, 450.1156, C₁₇H₂₄NO₆F₃NaS requires 450.1174.

### 1,2,4,5-tetraoxane 39f

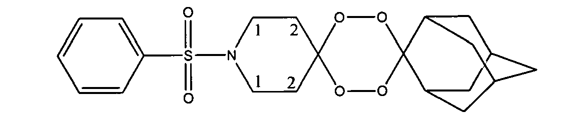

δ_{H} (400 MHz, CDCl₃), 7.50-7.85, (5H, aromatics), 3.01-3.25, (4H, m, 4H1), 2.51, (2H, s, 2H2a), 1.51-2.20 (16H, m, 2H2b and adamantane); δ_{C} (100MHz, CDCl₃), 136.6, 133.4, 129.6, 128.0, 111.5, 105.8, 47.4, 39.7, 37.2, 36.7, 33.4, 27.8, 27.3; m/z (ES, +ve, CH₃OH), 444 ([M+Na]⁺, 100%); Found [M+Na]⁺, 444.1445, C₂₁H₂₇NO₆NaS requires 444.1457.

### 1,2,4,5-tetraoxane 39g

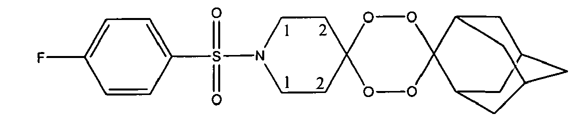

δ_{H} (400 MHz, CDCl₃), 7.10-7.80, (4H, aromatics), 3.05-3.25, (4H, m, 4H1), 2.55, (2H, s, 2H2a), 1.55-2.10 (16H, m, 2H2b and adamantane); δ_{C} (100MHz, CDCl₃), 130.7, 130.6, 117.0, 116.7, 111.5, 105.6, 47.4, 39.7, 37.2, 36.7, 33.4, 32.0, 27.3; m/z (ES, +ve, CH₃OH), 462 ([M+Na]⁺, 100%); Found [M+Na]⁺, 462.1341, C₂₁H₂₆NO₆FNaS requires 462.1363.

### 1,2,4,5-tetraoxane 39h

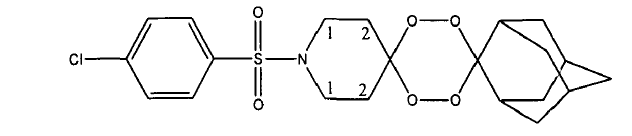

δ_{H} (400 MHz, CDCl₃), 7.50-7.85, (4H, aromatics), 3.05-3.25, (4H, m, 4H1), 2.55, (2H, s, 2H2a), 1.55-2.10 (16H, m, 2H2b and adamantane); δ_{C} (100MHz, CDCl₃), 140.0, 129.9, 129.4, 124.0, 111.5, 105.6, 47.4, 39.7, 37.2, 36.7, 33.4, 32.0, 27.3; m/z (ES, +ve, CH₃OH), 478 ([M+Na]⁺, 100%); Found [M+Na]⁺, 478.1081, C₂₁H₂₆NO₆NaSCl requires 478.1067.

### 1,2,4,5-tetraoxane 39i

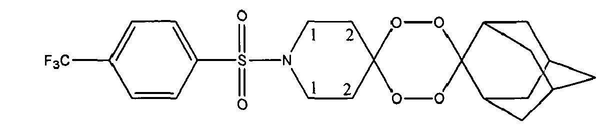

δ_{H} (400 MHz, CDCl₃), 7.75-7.95, (4H, aromatics), 3.05-3.25, (4H, m, 4H1), 2.55, (2H, s, 2H2a), 1.55-2.10 (16H, m, 2H2b and adamantane); δ_{C} (100MHz, CDCl₃), 128.4, 128.0, 127.3, 127.3, 126.9, 116.8, 111.4, 47.4, 39.7, 37.2, 36.7, 33.4, 32.0, 27.3; m/z (ES, +ve, CH₃OH), 512 ([M+Na]⁺, 100%); Found [M+Na]⁺, 512.1346, C₂₂H₂₆NO₆F₃NaS requires 512.1331.

### 1,2,4,5-tetraoxane 40a

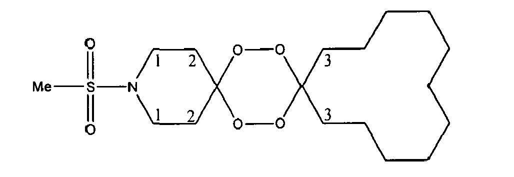

δ_{H} (400 MHz, CDCl₃), 3.25-3.42, (4H, m, 4H1), 2.80, (3H, s, C*H*₃), 2.50 (2H, bs, 2H2a), 2.25 (2H, m, 2H3a), 1.85 (2H, bs, 2H2b), 1.60 (2H, m, 2H3b), 1.21-1.49 (18H, m, dodecane ring); δ_{C} (100MHz, CDCl₃), 113.5, 105.7, 43.3, 42.1, 35.5, 29.9, 29.5, 26.3, 22.6, 19.8, 18.4; m/z (ES, +ve, CH₃OH), 414 ([M+Na]⁺, 100%); Found [M+Na]⁺, 414.1926, C₁₈H₃₃NO₆NaS requires 414.1926.

### 1,2,4,5-tetraoxane 40b

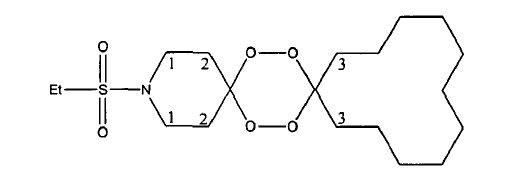

δ_{H} (400 MHz, CDCl₃), 3.31-3.50, (4H, m, 4H1), 2.95, (2H, q, *J* 7.4, C*H*₂CH₃), 2.50 (2H, bs, 2H2a), 2.25 (2H, bs, 2H3a), 1.85 (2H, bs, 2H2b), 1.60 (2H, m, 2H3b), 1.28 (3H, t, *J* 7.4, CH₂CH₃), 1.31-1.49 (18H, m, dodecane ring); δ_{C} (100MHz, CDCl₃), 113.5, 106.0, 44.9, 42.1, 35.5, 29.9, 26.4, 26.2, 22.6, 19.8, 8.26; m/z (ES, +ve, CH₃OH), 428 ([M+Na]⁺, 100%); Found [M+Na]⁺, 428.2100, C₁₉H₃₅NO₆NaS requires 428.2083.

### 1,2,4,5-tetraoxane 40c

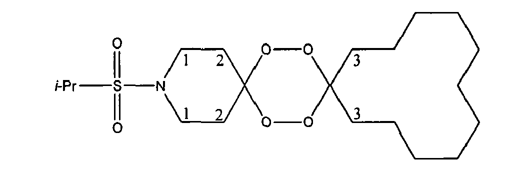

δ_{H} (400 MHz, CDCl₃), 3.31-3.50, (4H, m, 4H1), 3.15, (2H, m, CH₃C*H*CH₃), 2.50 (2H, bs, 2H2a), 2.25 (2H, bs, 2H3a), 1.85 (2H, bs, 2H2b), 1.60-1.20 (26H, m, 2H3b, C*H*₃CHC*H*₃, dodecane ring); δ_{C} (100MHz, CDCl₃), 113.5, 105.9, 54.2, 44.8, 42.0, 35.3, 29.9, 26.3, 26.1, 22.6, 19.7, 17.7; m/z (ES, +ve, CH₃OH), 442 ([M+Na]⁺, 100%); Found [M+Na]⁺, 442.2256, C₂₀H₃₇NO₆NaS requires 442.2239.

### 1,2,4,5-tetraoxane 40d

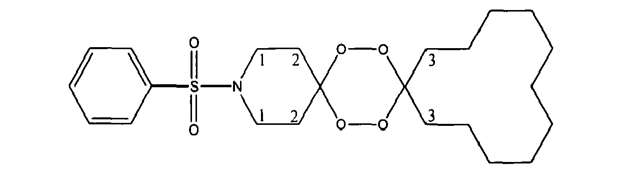

δ_{H} (400 MHz, CDCl₃), 7.95-7.45 (5H, m, aromatics), 3.05-3.35, (4H, m, 4H1), 2.50 (2H, bs, 2H2a), 2.25 (2H, bs, 2H3a), 1.85 (2H, bs, 2H2b), 1.62 (2H, m, 2H3b), 1.21-1.49 (18H, m, dodecane ring); δ_{C} (100MHz, CDCl₃), 136.5, 133.4, 129.6, 128.0, 113.4, 105.7, 43.7, 42.4, 31.6, 29.7, 26.5, 26.2, 23.1, 19.7; m/z (ES, +ve, CH₃OH), 476 ([M+Na]⁺, 100%); Found [M+Na]⁺, 476.2097, C₂₃H₃₅NO₆NaS requires 476.2083.

### 8-Aza-bicyclo[3.2.1]octan-3-one 41b

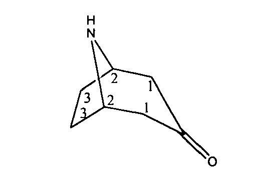

δ_{H} (400 MHz, CDCl₃), 10.45 (1H, bs, NH), 4.35, (2H, m, 2H2), 3.30 (2H, m, 2H1a), 2.51 (2H, m, 2H1b), 2.40 (2H, m, 2H3a), 1.90 (2H, m, 2H3b); δ_{c} (100MHz, CDCl₃), 202.4, 55.2, 46.7, 27.6; m/z (CI, +ve, NH₃), 126 ([M+H]⁺, 100%); Found [M+H]⁺, 126.09203, C₇H₁₂NO requires 126.09189.

### 8-Ethanesulfonyl-8-aza-bicyclo[3.2.1]octan-3-one 41c

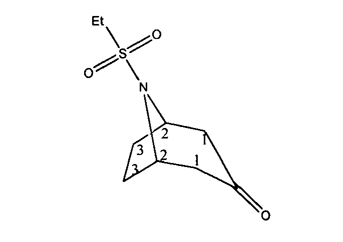

δ_{H} (400 MHz, CDCl₃), 4.40, (2H, m, 2H2), 3.10 (2H, q, *J*7.4, C*H*₂CH₃), 2.80 (2H, m, 2H1a), 2.41 (2H, m, 2H1b), 2.15 (2H, m, 2H3a), 1.80 (2H, m, 2H3b), 1.39 (3H, t, *J* 7.4, CH₂C*H*₃) ; δ_{c} (100MHz, CDCl₃), 207.2, 56.7, 50.3, 48.8, 30.5, 8.8; m/z (CI, +ve, NH₃), 235 ([M+NH₃]⁺, 100%); Found [M+NH₃]⁺, 235.11197, C₉H₁₉N₂O₃S requires 235.11163.

### Tropinone derived 1,2,4,5-tetraoxane 41d

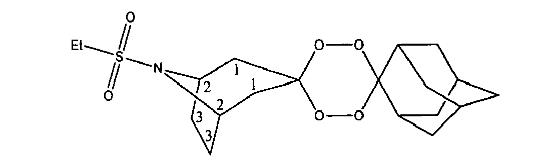

δ_{H} (400 MHz, CDCl₃), 4.30, (2H, bs, 2H2), 3.20 (2H, m, 2H1a), 3.10 (2H, m, 2H1b), 3.00 (2H, q, *J* 7.3, C*H*₂CH₃), 2.15 (2H, m, 2H3a), 1.50-2.01 (16H, m, 2H3b and adamantane), 1.39 (3H, t, *J* 7.3, CH₂C*H*₃); δ_{C} (100MHz, CDCl₃), 111.1, 106.9, 56.2, 48.8, 38.0, 37.3, 36.5, 33.5, 30.1, 27.4, 8.8; m/z (ES, +ve, CH₃OH), 422 ([M+Na]⁺, 100%); Found [M+Na]⁺, 422.1592, C₁₉H₂₉NO₆NaS requires 422.1613.

### REFERENCES

1. Ledaal, T; Acta Chem. Scand., 1967,21,1656-1659.
2. Dechy-Cabaret, O.; Benoit-Vical, F.; Robert A.; and Meunier, B.; Chembiochem, 2000,1, 281-283.
3. Spartan'04, Wavefunction, Inc., Irvine, CA. http://www.wavefun.com/
4. Trager, W; Jenson, J.B. Human Malaria Parasites in Continuous Culture. Science, 1976, 193, 673-675.
5. Desjardins, R. E.; Canfield, C. J.; Haynes, J. D.; Chulay, J.D. Quantitative Assessment of Antimalarial activity in vitro by Semi-automated Microdilution Technique. Antimicrob. Agents Chemother., 1979, 16, 710-718.

## Claims

1. A compound having the formula (I) 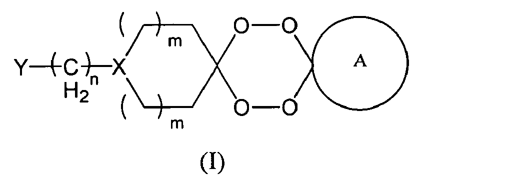 wherein
ring A represents a substituted or unsubstituted monocyclic or multicyclic ring; m=any positive integer; n=1-5;
X=CH and Y=-C(O)NR¹R², -NR¹R² or -S(O)₂R⁴ where R¹, R² and R⁴ are each individually selected from the group consisting of H, substituted or unsubstituted alkyl, substituted or unsubstituted aryl, substituted or unsubstituted amine, substituted or unsubstituted carbocyclic ring, substituted or unsubstituted heterocyclic ring, or any combination thereof, or R¹ and R² are linked so as to form part of a substituted or unsubstituted heterocyclic ring;
or
X=N and Y=-S(O)₂R³ or -C(O)R³, where R³ is selected from the group consisting of H, substituted or unsubstituted alkyl, substituted or unsubstituted aryl, substituted or unsubstituted amine, substituted or unsubstituted carbocyclic ring, substituted or unsubstituted heterocyclic ring or any combination thereof.

2. A compound according to claim 1, wherein n=1-4.

3. A compound according to claim 1, wherein n=1.

4. A compound according to claim 1, 2 or 3, wherein ring A contains 3 to 30 carbon atoms, or 5 to 15 carbon atoms.

5. A compound according to claim 1, 2, 3 or 4, wherein ring A is a substituted or unsubstituted mono- or polycyclic alkyl ring, or ring A is selected from the group consisting of a substituted or unsubstituted cyclopentyl ring, a substituted or unsubstituted cyclohexyl ring, a substituted or unsubstituted cyclododecanyl ring, and a substituted or unsubstituted adamantyl group.

6. A compound according to any preceding claim, wherein X=CH, Y=-C(O)NR¹R² or NR¹R², R¹=H and R²=alkyl group substituted with an ester group, amine group or amido group, wherein said alkyl group is optionally an ethyl group, wherein said amino group is optionally a diethylaminoethyl group, and/or wherein said ester group is optionally a methylester group.

7. A compound according to any one of claims 1 to 5, wherein X=CH, Y=-C(O)NR¹R² or -NR¹R², R¹=H and R² contains a substituted or unsubstituted carbocyclic ring or a substituted or unsubstituted heterocyclic ring, zero, one or more methylene radicals being provided in between said carbocyclic or heterocyclic ring and the nitrogen atom of group Y, optionally R² contains a substituted or unsubstituted cycloalkyl group containing 3 to 6 carbon atoms, and/or optionally said cycloalkyl group is bonded directly to the nitrogen atom of group Y.

8. A compound according to claim 7, wherein R² contains a substituted or unsubstituted heterocyclic group containing 3 to 6 carbon atoms and at least one heteroatom, the or each heteroatom being separately selected from the group consisting of nitrogen, oxygen and sulphur, optionally said heterocyclic group is linked to the nitrogen atom of group Y via two methylene radicals, and/or optionally said heterocyclic group is selected from the group consisting of a pyrrolidyl group, a piperidyl group, a morpholinyl group, a thiomorpholinyl group and a thiomorpholinyl sulfone group.

9. A compound according to any one of claims 1 to 5, wherein X=CH, Y=-C(O)NR¹R² or -NR¹R², and R¹ and R² are linked so as to form part of a substituted or unsubstituted heterocyclic ring selected from the group consisting of a pyrrolidyl group, a piperidyl group, a morpholinyl group, a thiomorpholinyl group and a thiomorpholinyl sulfone group; or wherein X=N, Y=-S(O)₂R³ or -C(O)R³, and R³ is a substituted or unsubstituted phenyl group or a substituted or unsubstituted heterocyclic group selected from the group consisting of a pyrrolidyl group, a piperidyl group, a morpholinyl group, a thiomorpholinyl group and a thiomorpholinyl sulfone group; or wherein m=1, n=1, X=CH and Y=-C(O)NR¹R², where R¹ and R² are each individually selected from the group consisting of H, substituted or unsubstituted alkyl, substituted or unsubstituted aryl, substituted or unsubstituted amine, substituted or unsubstituted carbocyclic ring, substituted or unsubstituted heterocyclic ring, or any combination thereof, or R¹ and R² are linked so as to form part of a substituted or unsubstituted heterocyclic ring, and optionally wherein ring A is an adamantyl group.

10. A compound according to any one of claims 1 to 5, wherein m=1, n=1, X=CH, Y=S(O)₂R⁴, wherein R⁴ is selected from the group consisting of H, substituted or unsubstituted alkyl, substituted or unsubstituted aryl, substituted or unsubstituted amine, substituted or unsubstituted carbocyclic ring, substituted or unsubstituted heterocyclic ring, or any combination thereof.

11. A compound according to claim 1 having the formula (XVIII) 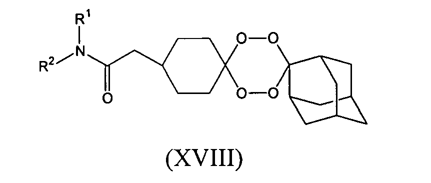 wherein
R¹ = -H and R² = -H, -NH₂, -CH(CH₂)₂, -CH₂CH(CH₃)₂, -C(CH₃)₃, - CH₂CH₂N(CH₂)₄, -CH₂CH₂N(CH₂)₅, -CH₂CH₂N(CH₂)₄O, -CH₂CH₂N(C₂H₅)₂, -CHCH(CH₃)₂CO₂CH₃, or -NHPh;
or
R¹ = -CH(CH₃)₂ and R²⁼ -CH(CH₃)₂;
or
R¹ and R² are linked so that the group -NR¹R² is selected from the group consisting of

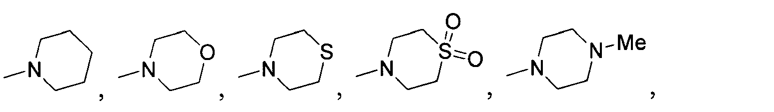

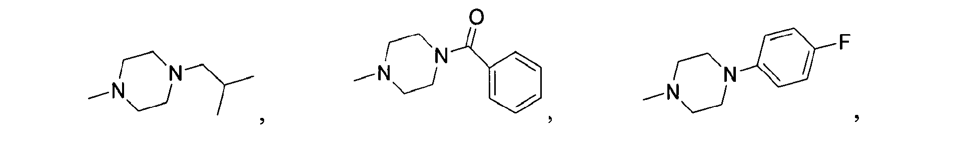

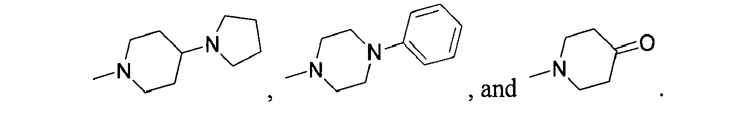

12. A compound according to claim 1 having the formula (XIX) 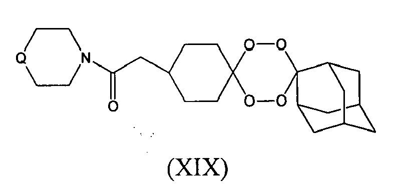 wherein
Q = CH₂, S, O, S(O)₂, or N-T, where T = H or C₁₋₄ alkyl, preferably T = Me or ^{t}Bu.

13. A compound having a formula selected from the group consisting of 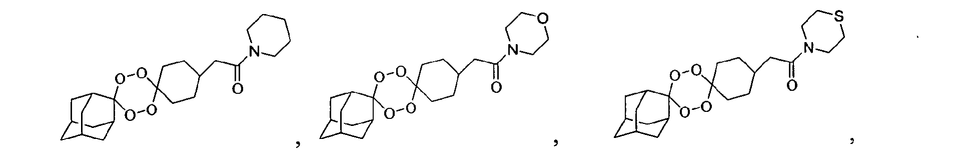 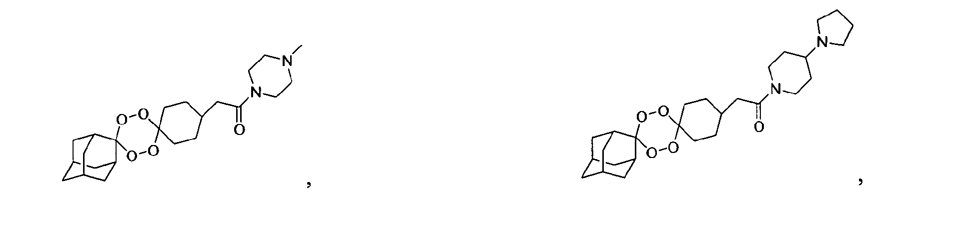 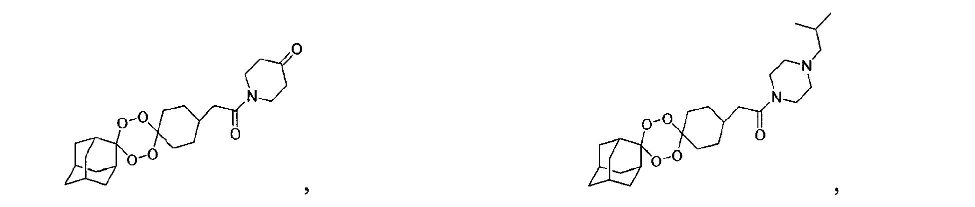 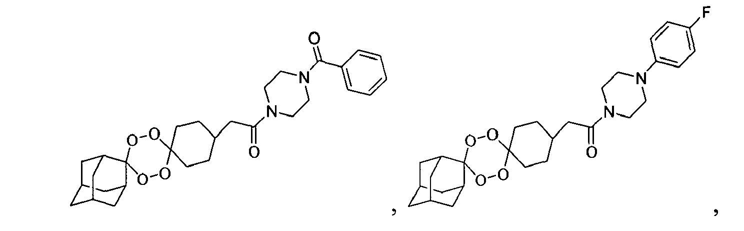 and

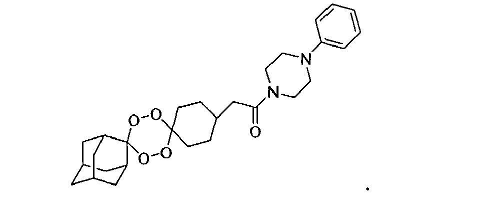

14. A pharmaceutical composition comprising a compound according to any preceding claim and a pharmaceutically acceptable excipient.

15. A pharmaceutical composition for the treatment of malaria or cancer comprising a compound according to any one of claims 1 to 13 and a pharmaceutically acceptable excipient.

16. Use of a compound according to any one of claims 1 to 13 in the preparation of a medicament for the treatment of malaria or cancer.

17. A method for the production of a compound according to claim 1, wherein the method comprises reacting a bishydoperoxide compound having formula (Ia) with a ketone having formula (Ib)

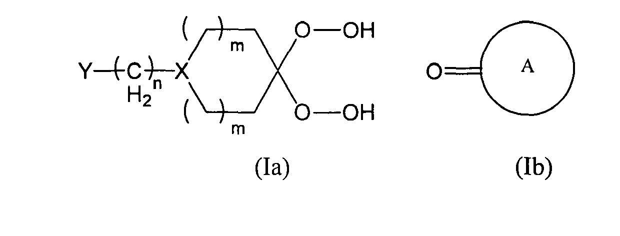

18. A method for the production of a compound according to claim 1 in which X=CH and Y=-C(O)NR¹R², wherein the method comprises an amide coupling reaction between NHR¹R² and a compound having formula (IV) 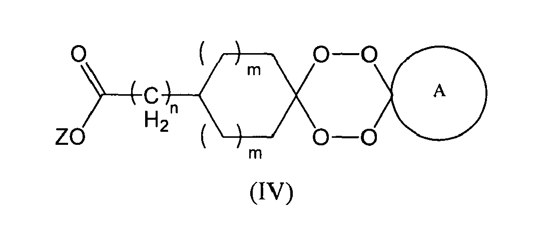 wherein
Z=H or alkyl,
and wherein
compound (IV) is optionally prepared by reacting compound (V) with compound (Ib) 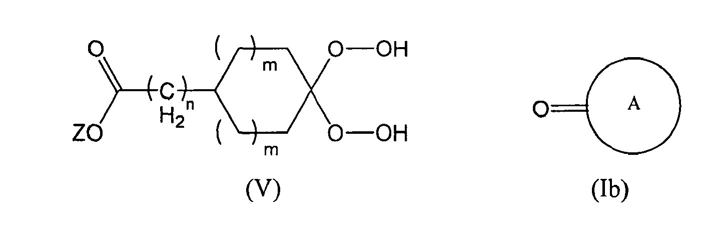 and wherein
compound (V) is optionally prepared by oxidising compound (VI) 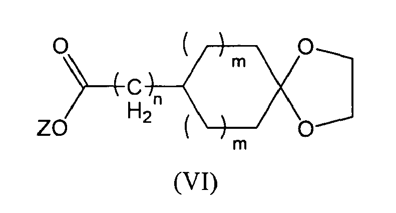 and, optionally for compounds of formula (I) in which n=1 to 4, compound (VI) may be prepared by reacting compound (VII) with compound (VIII) under conditions to facilitate a Wittig reaction between said compounds and subsequently hydrogenating the resulting C=C bond formed as a result of said Wittig reaction.

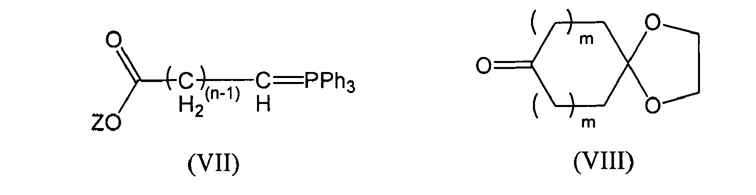

19. A method for the production of a compound according to claim 1, wherein the method comprises reacting a ketone compound (Ic) with an oxidising agent in a reaction mixture so as to oxidise said ketone (Ic) to provide a bishydoperoxide compound (Ia) and adding a ketone compound (Ib) to said reaction mixture so as to react compound (Ia) with said ketone (Ib), said oxidising reaction and said reaction of compound (Ia) with compound (Ib) being effected in the presence of a fluorinated alcoholic solvent.
